(19) Europäisches Patentamt
European Patent Office
Office européen des brevets

(11) Publication number: 0 051 404
B1

(12) EUROPEAN PATENT SPECIFICATION

(45) Date of publication of patent specification: 13.06.84

(21) Application number: 81304984.8

(22) Date of filing: 22.10.81

(51) Int. Cl.³: C 07 C 43/18, C 07 C 43/188, C 07 C 43/196, C 07 C 41/01, A 61 K 7/46, C 11 B 9/00, C 11 D 3/50

# (54) Norbornyl ethers, their preparation and their use in augmenting the aroma of a consumable material, cloth or fabric.

(30) Priority: 23.10.80 US 200012
29.12.80 US 220351

(43) Date of publication of application:
12.05.82 Bulletin 82/19

(45) Publication of the grant of the patent:
13.06.84 Bulletin 84/24

(84) Designated Contracting States:
CH DE FR GB LI NL

(56) References cited:
GB - A - 1 291 739

CANADIAN JOURNAL OF CHEMISTRY, Vol. 49 1971, Ottawa T.C. SHIELDS "Electrophilic Additions to Ethylidenenorbornene and Vinylnorbornene" pages 1142 to 1146

(73) Proprietor: INTERNATIONAL FLAVORS & FRAGRANCES INC.
521 West 57th Street
New York New York 10019 (US)

(72) Inventor: Sprecker, Mark A.
6 Sandpiper Lane
Sea Bright New Jersey 07760 (US)

(74) Representative: Brown, John David et al,
FORRESTER & BOEHMERT Widenmayerstrasse 4/I
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The instant invention relates to the use of compounds having the generic structures:

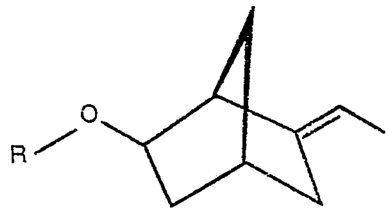
and
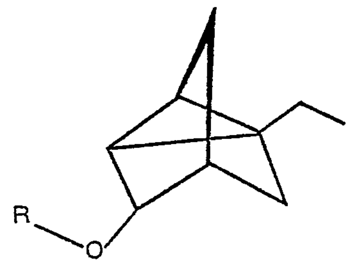

either taken alone or in admixture wherein R represents $C_3$—$C_6$ alkyl; $C_3$—$C_6$ alkenyl; $C_6$—$C_{10}$ aralkyl; $C_6$—$C_{10}$ alkaryl; hydroxy-$C_2$—$C_6$-alkyl; $C_1$—$C_4$-alkoxy-$C_2$—$C_6$ alkyl; and $C_5$—$C_8$ cycloalkyl; and wherein the R moieties in each structure are the same with the proviso that in the compound comprising the double bond R cannot be an $C_6$-alkyl, for augmenting or enhancing the aroma of perfumes, perfumed articles and colognes. These compounds as a group have long lasting, fresh, green bean, rosey, citrus, petitgrain-like, fruity, anisic, green, raw potato-like, twiggy, herbaceous, sweet, sweaty, green pea-like, chocolate-like, carrot-like and creamy aroma nuances with galbanum top-notes and anther-like and anise-like undertones.

The compounds of our invention may be prepared by reacting ethylidene norbornene having the structure:

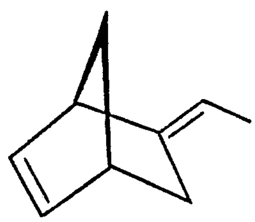

with an alcohol ROH wherein R represents $C_3$—$C_6$ alkyl; $C_3$—$C_6$ alkenyl; $C_6$—$C_{10}$ aralkyl; $C_6$—$C_{10}$ alkaryl; hydroxy-$C_2$—$C_6$-alkyl; $C_1$—$C_4$-alkoxy-$C_2$—$C_6$ alkyl and $C_5$—$C_8$ cycloalkyl in the presence of a catalyst which is either a mineral acid or a Lewis acid. Examples of mineral acid catalysts are sulfuric acid, phosphoric acid, para-toluene sulfonic acid, methane sulfonic acid and acid ion-exchange resin. Examples of Lewis acids which can be used as catalysts are boron trifluoride etherate, aluminum chloride, zinc chloride, stannic chloride, stannous chloride, zinc bromide, diethyl aluminum chloride, ethyl aluminum dichloride, ethyl aluminum dibromide and diethyl aluminum bromide. The reaction preferably takes place in the presence of an inert solvent such as tetrahydrofuran, toluene or benzene. The reaction may take place in the absence of the inert solvent an in the presence of an excess of the alcohol reactant, the excess of the alcohol reactant being used as the "solvent".

The reaction temperature may vary from about 25°C up to 120°C with reflux temperature being preferred. The reflux temperature depends upon the pressure in the reactor and the particular solvent being used as well as its concentration. The mole ratio of acid catalyst to ethylidene norbornene may vary from about 1:99 up to about 1:10. The mole ratio of ethylidene norbornene reactant to ROH alcohol reactant may vary from about 1:1 up to about 1:2 with a mole ratio of 1:1.5 of norbornene: alcohol reactant being preferred. Thus, the reaction to produce the compounds of my invention may be shown thusly:

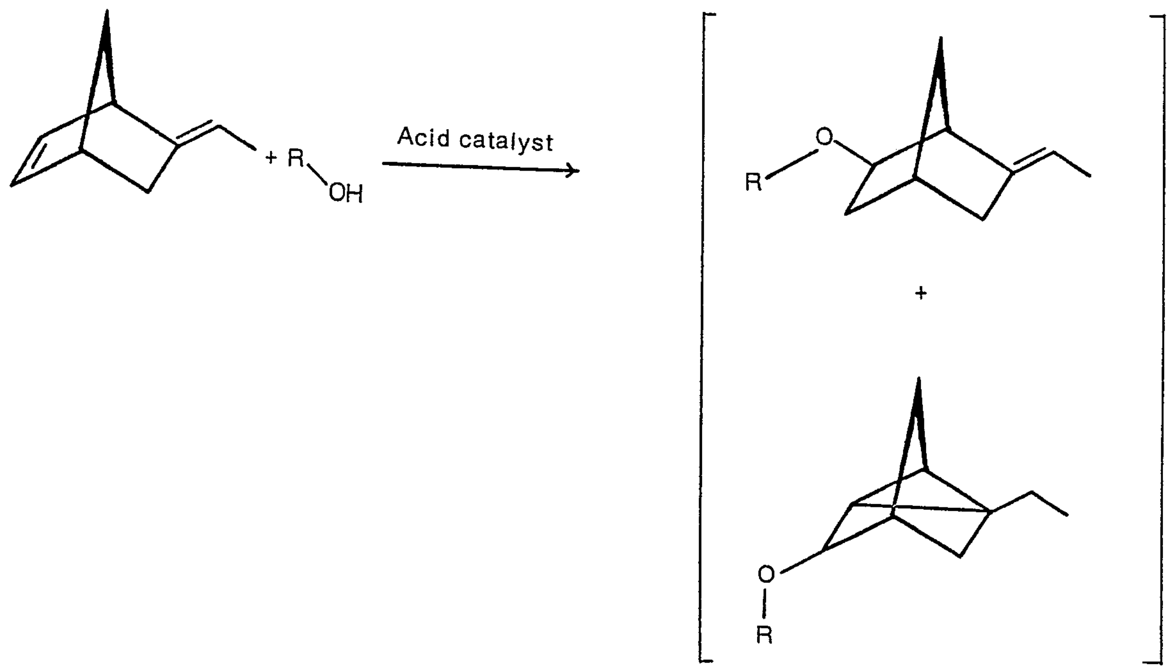


The compounds of my invention are usually prepared in admixture with compounds having the generic structure:

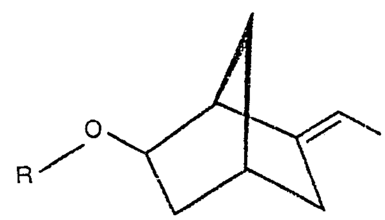

being prepared along with compounds having the structure:

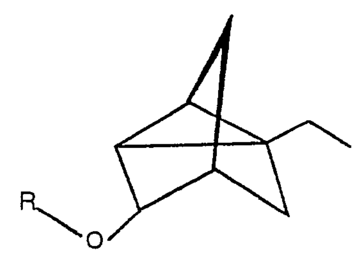

These compounds, however, may be separated by distillation extraction and preparative GLC techniques in order to yield separately compounds having the structure:

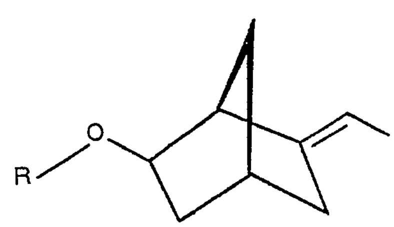

and separate therefrom compounds having the structure:

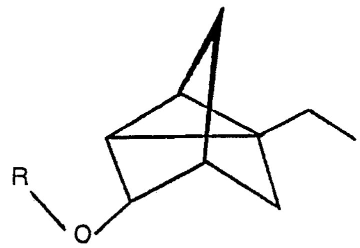

In addition, the compounds having the structures:

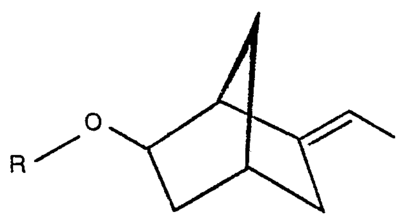

and

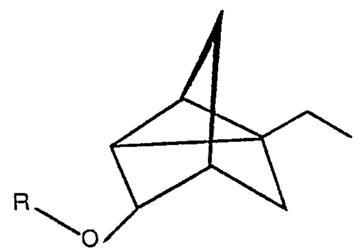

exist in isomeric forms and are produced in admixture. The mixture of these "endo" and "exo" and "cis" and "trans" isomers may be separated from one another by means of standard separation techniques including preparative GLC techniques whereby the individual isomers may be separated and then utilized individually. Structures of these isomers are as follows

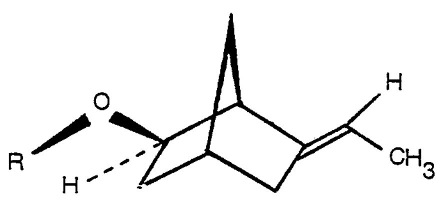

;

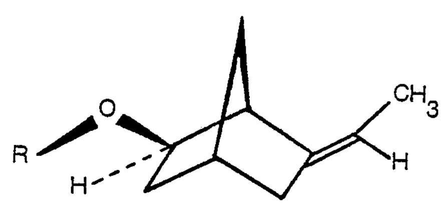

;

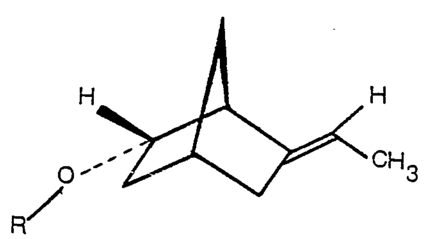

;

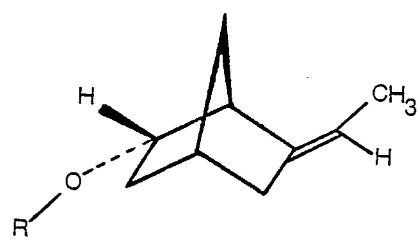

;

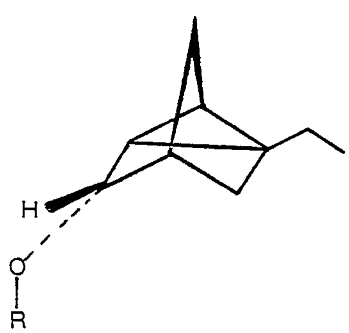

and

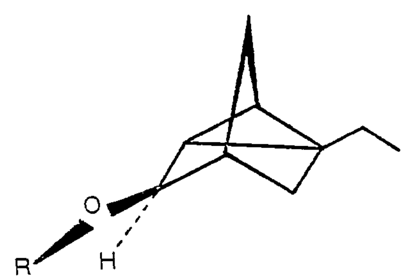

GB—A—1 291 739 (Sumitomo) describes a process for the preparation of 2-alkylidene norbornene derivatives from 1-alkenyl nortricyclenes, the reaction disclosed being:-

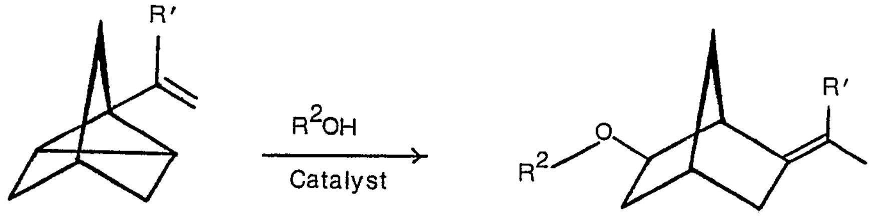


wherein $R^1$ is hydrogen or one to eight carbon alkyl and $R^2$ is hydrogen alkyl or acyl. This reference indicates that the use of the resultant compounds are as intermediates in the production of medicinal agents and agricultural chemicals.

"Electrophilic additions to ethylidenenorbornene and Vinylnorbornene", (T.C. Shields), Canadian Journal of Chemistry, Vol 49, 1971 pages 1142—1146 discloses compounds having the structures:

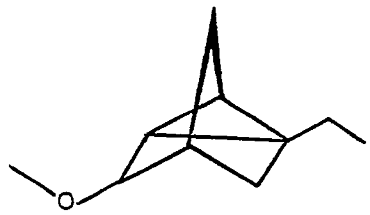

and

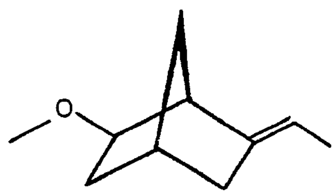

and states that electrophilic and free radical additions to 5-ethylidenebicyclo(2.2.1)hept-2-ene and 5-vinylbicyclo(2.2.1)hept-2-ene were carried out with peracetic acid, hydrogen chloride and methanol. It is further stated that, in contrast to chlorosulfonyl isocyanate, peracetic acid primarily attacks the exocyclic double bond in the 5-ethylidenebicyclo(2.21)hept-2-ene. There is also disclosed several other electrophilic additions which demonstrate the unpredictable reactivity of the norbornyl system.

Specific examples of the compounds produced according to the foregoing process and useful for the practice of my invention are set forth in Table I below:

TABLE I

| Structure of Compound | Perfumery Evaluation |
|---|---|
| 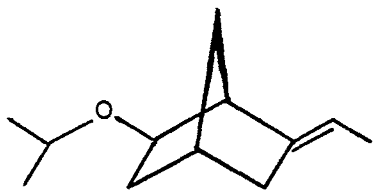<br>and<br>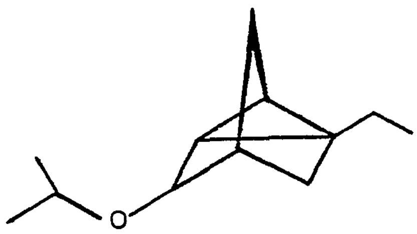<br>Produced according to either Example I(A) or I(B) | A fresh, green bean-like, rosey, citrus (petitgrain-like) aroma. |
| 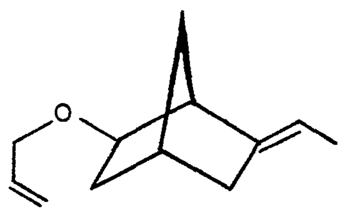<br>and<br>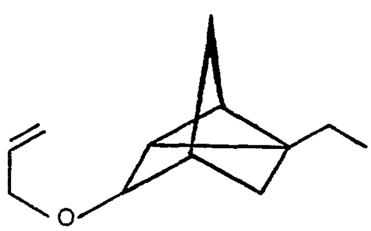<br>Produced according to Example II | A fruity, anisic, green aroma. |

TABLE I (Continued)

| Structure of Compound | Perfumery Evaluation |
| --- | --- |
| 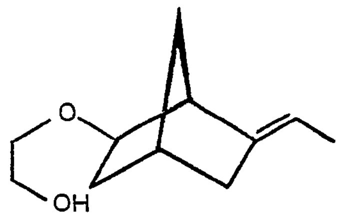 and 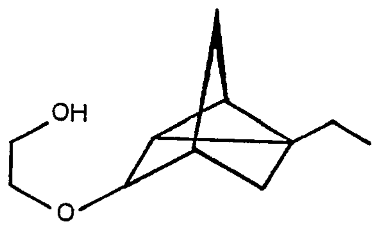 Produced according to Example III | A green, raw potato aroma with galbanum topnotes. |
| 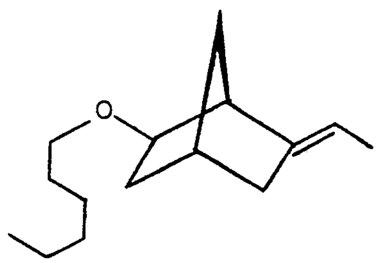 and 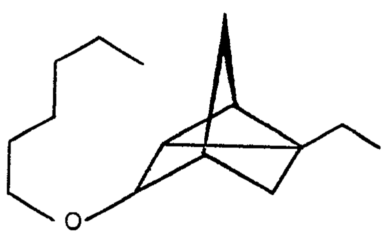 Produced according to Example IV | A long lasting, green, twiggy, fruity and herbaceous aroma. |

TABLE I (Continued)

| Structure of Compound | Perfumery Evaluation |
|---|---|
| 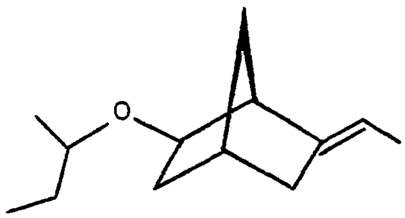<br>and<br>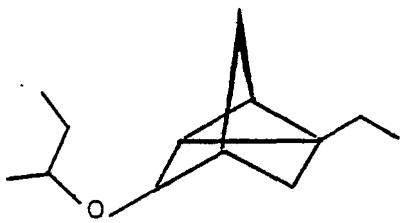<br>Produced according to Example V | A sweet, fruity aroma. |
| 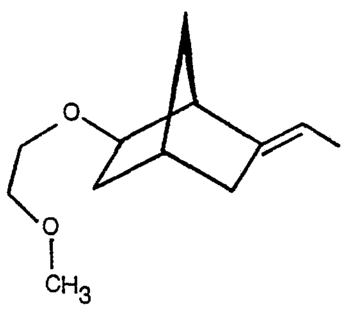<br>and<br>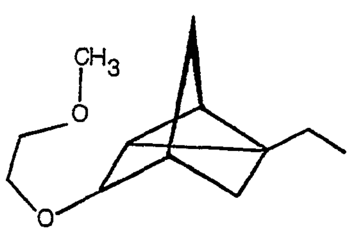<br>Produced according to Example VI | An excellent green aroma. |

TABLE I (Continued)

| Structure of Compound | Perfumery Evaluation |
|---|---|
| 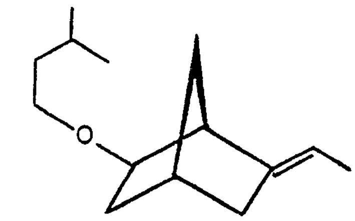<br>and<br>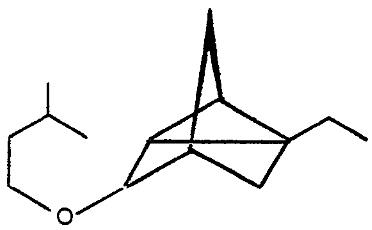<br>Produced according to Example VII | A green, sweaty aroma. |
| 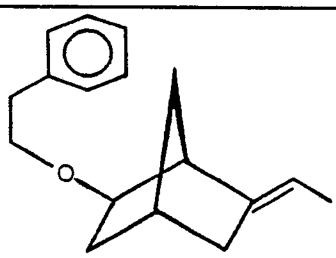<br>and<br>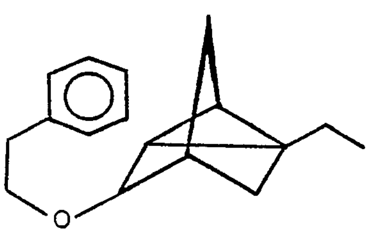<br>Produced according to Example VIII | A green pea-like and green aroma. |

TABLE I (Continued)

| Structure of Compound | Perfumery Evaluation |
| --- | --- |
| 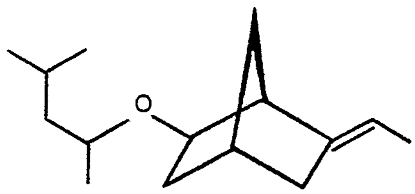<br>and<br>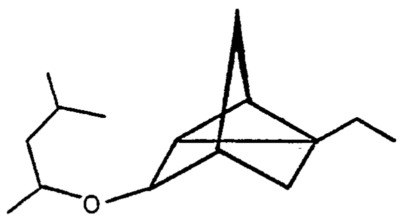<br>Produced according to Example IX | A green and chocolate-like aroma with anther-like undertones. |
| 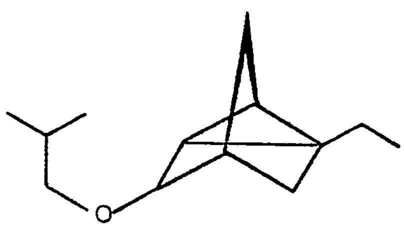<br>and<br>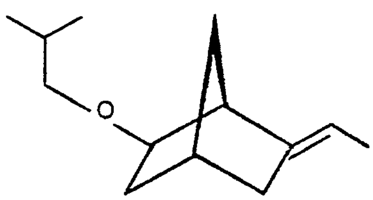<br>Produced according to Example X | A floral, green and carrot-like aroma. |

TABLE I (Continued)

| Structure of Compound | Perfumery Evaluation |
|---|---|
| 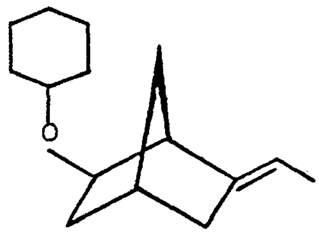<br>and<br>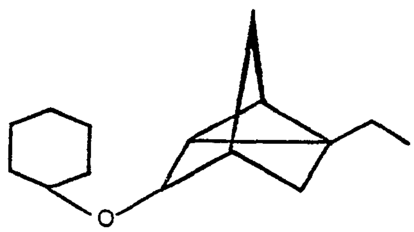<br>Produced according to Example XI | A green, creamy, string-bean-like herbaceous and floral-rosey aroma with anise-like undertones. |
| 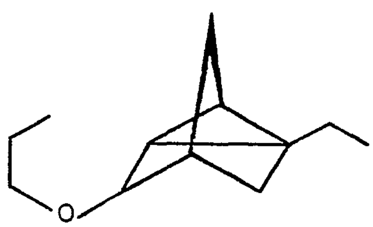<br>and<br>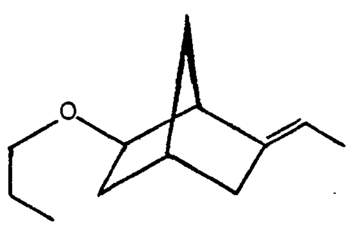<br>Produced according to Example XIV | A harsh, anise-like, sweet, metallic aroma lacking fresh, clean, herbaceous notes but having a strong, fruity nuance. |

TABLE I (Continued)

| Structure of Compound | Perfumery Evaluation |
|---|---|
| 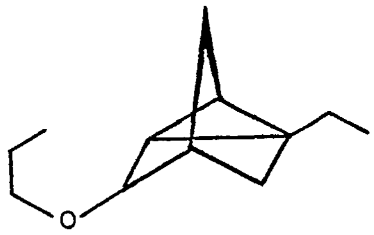<br>Prepared according to Example.XIV | An aniseed-like, woody aroma with a slightly fruity character. |
| 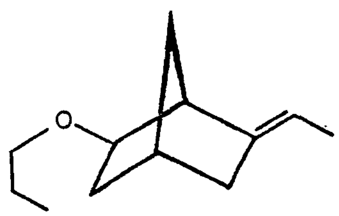<br>Prepared according to Example XIV | A light, fresh, slightly herbaceous aroma, which is relatively long-lasting. |
| 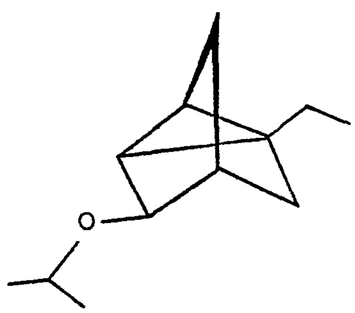<br>Prepared according to Example I | A fresh, green, green bean, herbaceous, citrus (lemony) aroma profile. |
| 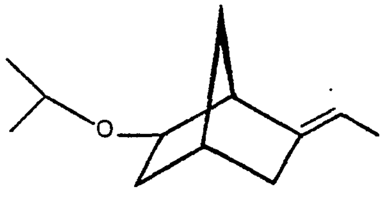<br>Prepared according to Example I | A metallic, potato-like, galbanum-like, eucalyptol-like, costos-oil-like aroma. |

The norbornyl ether derivative(s) and one or more auxiliary perfume ingredients, including, for example, hydrocarbons, alcohols, ketones, aldehydes, nitriles, esters, lactones, ethers other than said norbornyl ether derivative(s), hydrocarbons, synthetic essential oils and natural essential oils may be admixed so that the combined odors of the individual components produce a pleasant and desired fragrance, particularly and preferably in citrusy and/or green woody and/or piney fragrances. Such perfume compositions usually contain (a) the main note or the "bouquet" or foundation stone of the composition; (b) modifiers which round off and accompany the main note; (c) fixatives which include odorous substances which lend a particular note to the perfume throughout all stages of evaporation and substances which retard evaporation; and (d) topnotes which are usually low-boiling, fresh-smelling materials.

In perfume compositions, it is the individual components which contribute to their particular olfactory characteristics, however, the overall sensory effect of the perfume composition will be at least the sum total of the effects of each of the ingredients. Thus, one or more of the norbornyl ether derivative(s) of my invention can be used to alter, modify or enhance the aroma characteristic of a perfume composition, for example, by utilizing or moderating the olfactory reaction contributed by another ingredient in the composition.

The amount of norbornyl ether derivative(s) of my invention which will be effective in perfume compositions as well as in perfumed articles and colognes depends upon many factors, including the other ingredients, their amounts and the effects which are desired. It has been found that perfume compositions containing as little as 0.005% of norbornyl ether derivative(s) or even less (e.g. 0.002%) can be used to impart fresh, green bean, rosey, citrus, petitgrain-like, fruity, anisic, green, raw-potato-like, twiggy, herbaceous, sweet, sweaty, green pea-like, chocolate-like, floral, carrot-like and creamy aroma nuances with galbanum topnotes and anther-like and anise-like undertones to soaps, cosmetics, detergents (including anionic, nonionic, zwitterionic and cationic solid or liquid detergents) or other products. The amount employed can range up to 70% of the fragrance components and will depend upon considerations of cost, nature of the end product, the effect desired on the finished product and the particular fragrance sought.

The norbornyl ether derivative(s) of my invention are useful (taken alone or together with other detergents in perfume compositions) in detergents and soaps, space odorants and deodorants, perfumes, colognes, toilet water, bath preparations such as lacquers, brilliantines, pomades and shampoos; cosmetic preparations such as creams, deodorants, hand lotions and sun screens; powders such as talcs, dusting powders, face powders and the like. As little as 0.25% of the norbornyl ether derivative(s) will suffice to impart an intense, green, petitgrain-like, rosey and citrusy note to citrusy, woody, floral and piney perfume formulations. Generally, no more than 5% of the norbornyl ether derivative(s) based on the ultimate end product is required to be used as is or in the perfume composition.

Furthermore, as little as 0.25% of the norbornyl ether derivative(s) will suffice to impart such aroma to perfumed articles per se, whether in the presence of other perfume materials or whether used by itself. Thus, the range of use of the norbornyl ether derivative(s) of my invention in perfumed articles may vary from 0.25% up to 5% by weight based on the total weight of the perfumed article.

In addition, the perfume composition or fragrance composition of my invention can contain a vehicle, or carrier for the norbornyl ether derivative(s). The vehicle can be a liquid such as a non-toxic alcohol, e.g., ethanol, a non-toxic glycol, e.g., propylene glycol or the like. The carrier can also be an absorbent solid, such as gum (e.g., gum arabic), or components for encapsulating the composition by means of coacervation (such as gelatin).

It will thus be apparent that the norbornyl ether derivative(s) of my invention can be utilized to alter, modify, or enhance the aroma of perfume compositions, colognes or perfumed articles.

Examples I(B) and XII—XIV serve to illustrate processes for specifically producing the norbornyl ether derivative(s) useful in our invention.

The following examples, in general, serve to illustrate specific embodiments of my invention. It will be understood that these examples are illustrative and the invention is to be considered restricted thereto only as indicated in the appended claims. All parts and percentages given herewith are by weight unless otherwise specified.

Example I

*Preparation of a mixture of 2-ethyl-5-isopropyl-tricyclo [2.2.1.0*$^{(2,6)}$]heptane and 2-ethylidene-6-isopropoxynorbornane

Reaction:

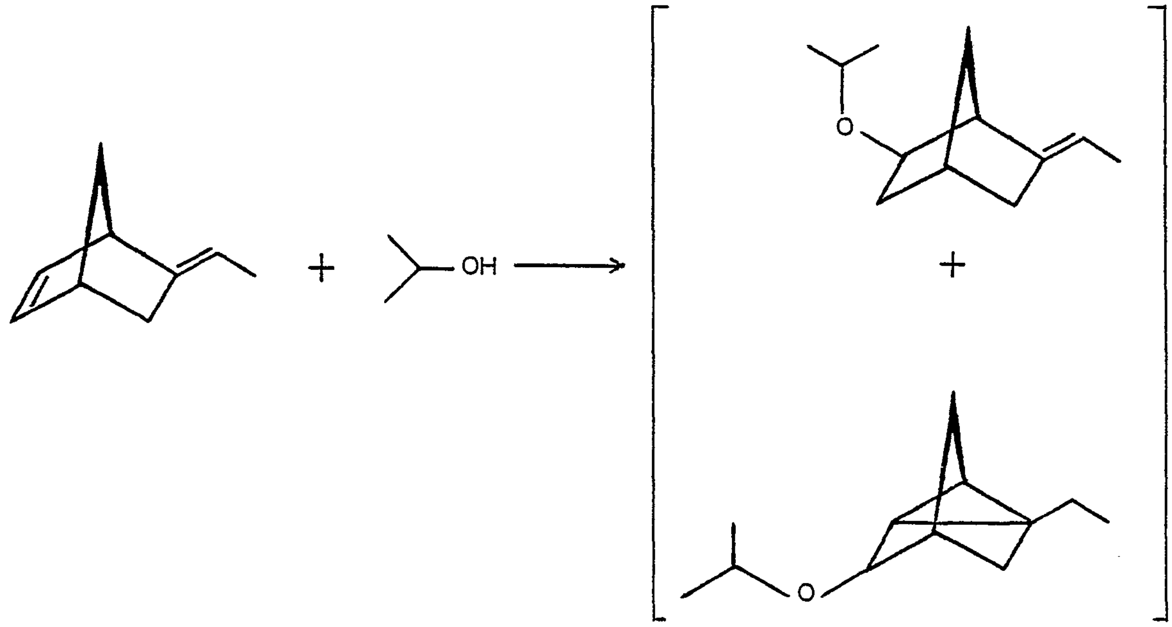

Example 1(A)

Vinylidene norbornene (480 grams) is added over a 90 minute period to a stirred solution of isopropanol (300 grams) and boron trifluoride etherate (12 grams) at reflux (temperature varies from 75°C to 97°C). The reaction mass is quenched with 1 liter of water. The organic layer is subsequently washed with 500 ml of 10% NaOH. Distillation through a 1—1/2" x 12" Goodloe packed column affords 651 grams or product (b.p. 75°C at 5 mm/Hg. pressure).

Figure 1 sets forth the GLC profile for the crude reaction product of this example containing the compounds having the structures:

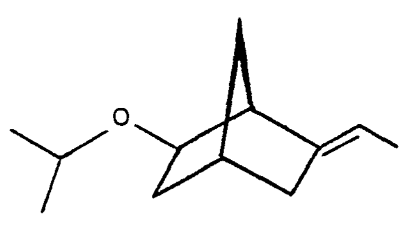

and

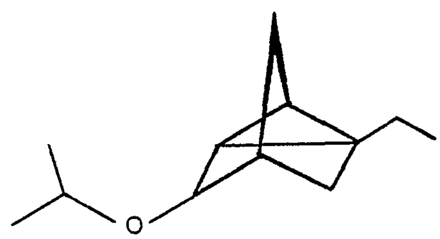

Figure 1(A) is the GLC profile of the purified reaction product of this example containing the compounds having the structures:

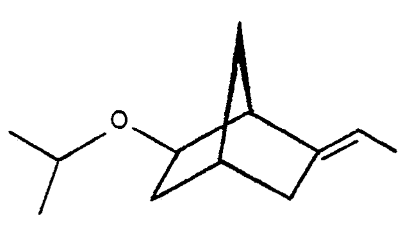

and

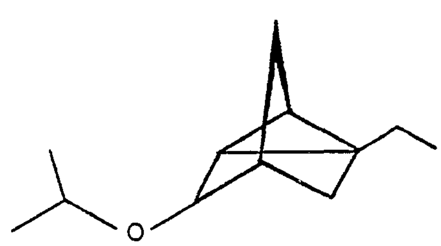

wherein peak 2 on said figure 1(A) is the compound having the structure:

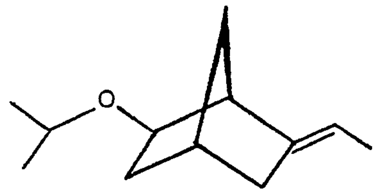

and peak 3 on said figure 1(A) consists of the compound having the structure:

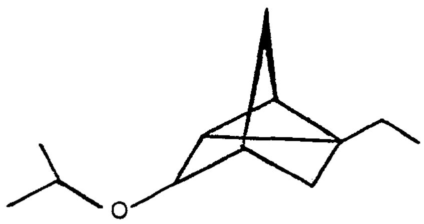

Figure 2 sets forth the NMR spectrum of the product mixture consisting of 2-ethyl-5-isopropyl-tricyclo[2.2.1.0$^{(2,6)}$]heptane and 2-ethylidene-6-isopropoxynorbornane having, respectively, the structures:

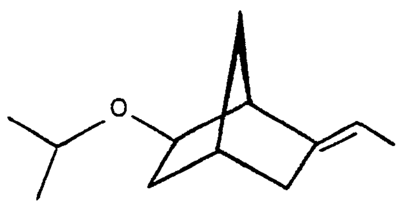

and

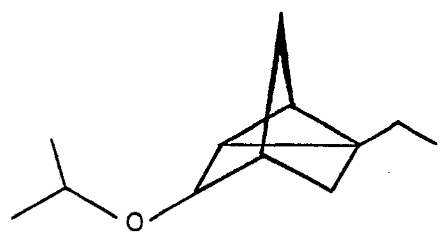

Figure 2(A) sets forth the NMR spectrum for peak 2 of the GLC profile of figure 1(A) consisting of the compound having the structure:

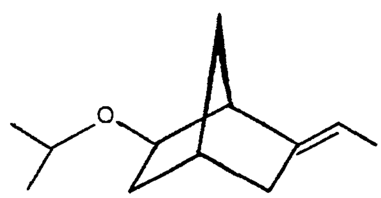

Figure 2(B) sets forth the NMR spectrum for peak 3 of the GLC profile of figure 1(A) consisting of the compound having the structure:

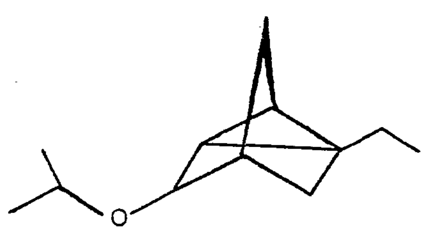

Figure 3 sets forth the infra-red spectrum of the product mixture consisting of 2-ethyl-5-propyltricyclo[2.2.1.0$^{(2,6)}$]heptane and 2-ethylidene-6- isopropoxynorborname.

Figure 3(A) sets for the infra-red spectrum for peak 2 of the GLC profile of figure 1(A) consisting of the compound having the structure:

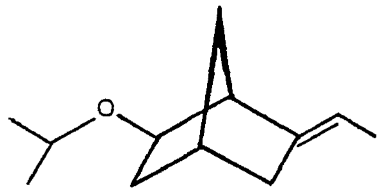

Figure 3(B) sets forth the infra-red spectrum for peak 3 of the GLC profile of figure 1(A) consisting of the compound having the structure:

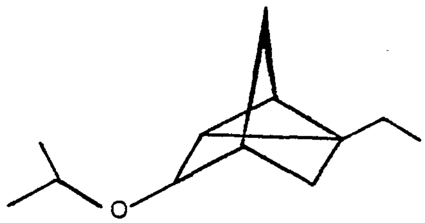

Example I(B)

(Reaction using sulfuric acid catalyst)

Into a 2 liter reaction flask equipped with stirrer, thermometer, reflux condenser and heating mantle is placed 16 grams (0.16 moles) of concentrated sulfuric acid and 480 grams (8.00 moles) of isopropyl alcohol. The resulting mixture is heated to 80°C, reflux temperature. While refluxing, 640 grams (5.33 moles) of vinylidene norbornene is added to the reaction mass and over a period of 2 hours, the reaction mass is heated at 85°C. The reaction mass is then admixed with 400 ml of 10% sodium hydroxide. The organic layer is separated from the aqueous layer and the organic layer is rushed over and then fractionally distilled on a 12" × 1—1/2" Goodloe column yielding the following fractions:

| Fraction Number | Vapor Temp. (°C) | Liquid Temp. (°C) | Pressure mm/Hg. | Reflux Ratio | Weight of Fraction (g.) |
|---|---|---|---|---|---|
| 1 | 45/48 | 69/69 | 1.6 | 4.1 | 43.5 |
| 2 | 48 | 69 | 1.6 | 4.1 | 61.6 |
| 3 | 49 | 69 | 1.6 | 4:1 | 18.2 |
| 4 | 42 | 69 | 1.6 | 4:1 | 21.0 |
| 5 | 47 | 66 | 1.4 | 4:1 | 25.0 |
| 6 | 47 | 67 | 1.4 | 4:1 | 40.6 |
| 7 | 50 | 69 | 1.4 | 100% | 51.1 |
| 8 | 50 | 69 | 1.4 | 100% | 14.3 |
| 9 | 50 | 70 | 1.4 | 100% | 49.8 |
| 10 | 51 | 72 | 1.4 | 100% | 43.4 |
| 11 | 51 | 74 | 1.4 | 100% | 45.5 |
| 12 | 52 | 74 | 1.4 | 100% | 52.0 |
| 13 | 52 | 73 | 1.4 | 100% | 50.6 |
| 14 | 54 | 75 | 1.4 | 100% | 46.9 |
| 15 | 54 | 77 | 1.4 | 100% | 52.7 |
| 16 | 52 | 74 | 1.4 | 100% | 49.7 |
| 17 | 52 | 80 | 1.4 | 100% | 52.3 |
| 18 | 52 | 85 | 1.4 | 100% | 43.8 |
| 19 | 51 | 100 | 1.4 | 100% | 36.3 |
| 20 | 50 | 200 | 3 | 100% | 38.1 |

Figure 3(C) represents the GLC profile for the reaction product of this example containing the compounds having the structures:

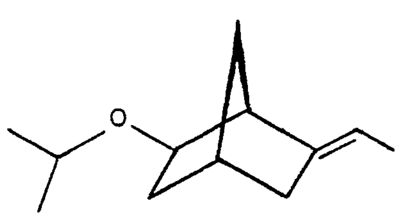

and

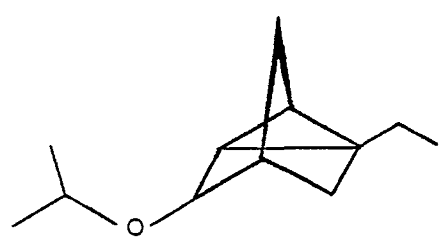

Figure 3(D) represents the NMR spectrum for the reaction product of this example containing the compounds having the structures:

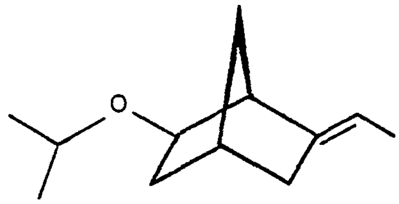

and

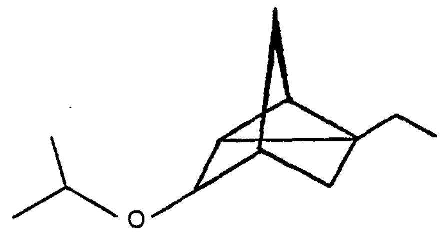

Example II

*Preparation of a mixture consisting of 2-ethyl-5-allyltricyclo[2.2.1.0$^{(2,6)}$]heptane and 2-ethylidene-6-allylnorbornane*

Reaction:

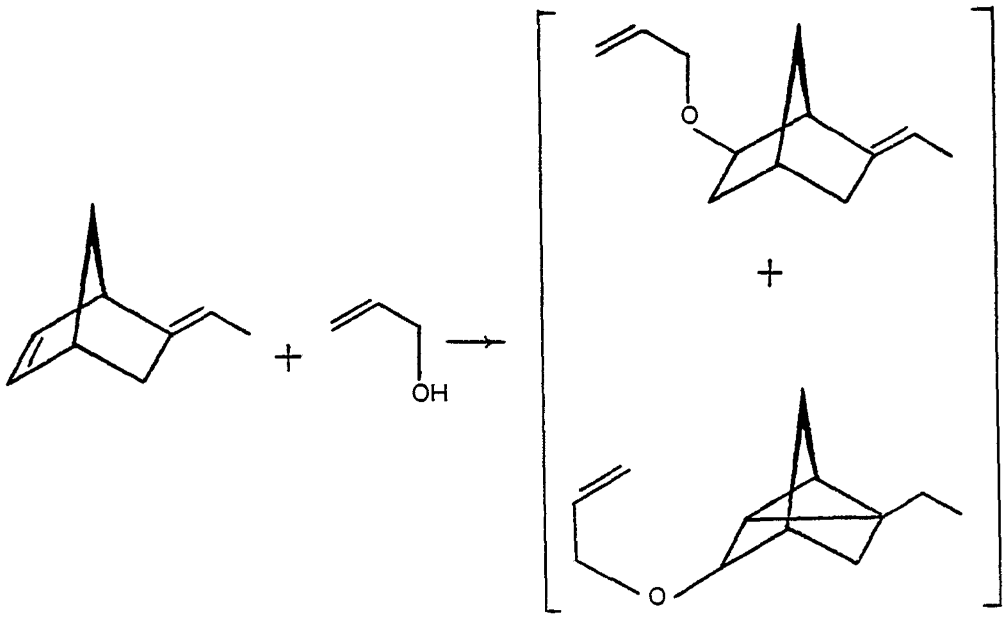

Ethylidene norbornene (480 grams) is added over a 90 minute period to a stirred solution of toluene (250 ml), allyl alcohol (267 grams), and $BF_3$ etherate (15 ml) at reflux. The reaction mass is quenched with 1 liter of water and the organic layer is washed with 500 ml of 10% sodium carbonate. The toluene is removed on a rotary evaporator. The organic solution is distilled through a 1—1/2″ x 12″ Goodloe packed column to afford 490 grams of product (b.p. 60°C at 3 mm/Hg pressure).

Figure 4 represents the GLC profile of the crude product (conditions: 150°C isothermal, 10′ x 1/4″, 10% SE—30 packed column).

Figure 5 shows the NMR spectrum of the mixture consisting of 2-ethyl-5-allyltricyclo[2.2.1.0$^{(2,6)}$]heptane and 2-ethylidene-6-allylnorbornane, respectively, having the structures:

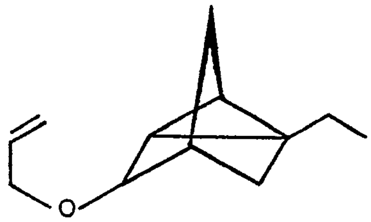

and

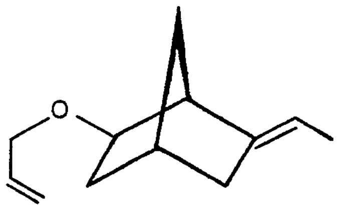

Figure 6 sets forth the infra-red spectrum of the mixture consisting of 2-ethyl-5-allyltricyclo-[2.2.1.$0^{(2,6)}$]heptane and 2-ethylidene-6-allylnorbornane.

Example III

*Preparation of a mixture consisting of 2-ethyl-5-(β-*hydroxyethoxy)-tricyclo[2.2.1.$0^{(2,6)}$]heptane and 2-ethylidene-6-(β-hydroxyethoxy)-norbornane

Reaction:

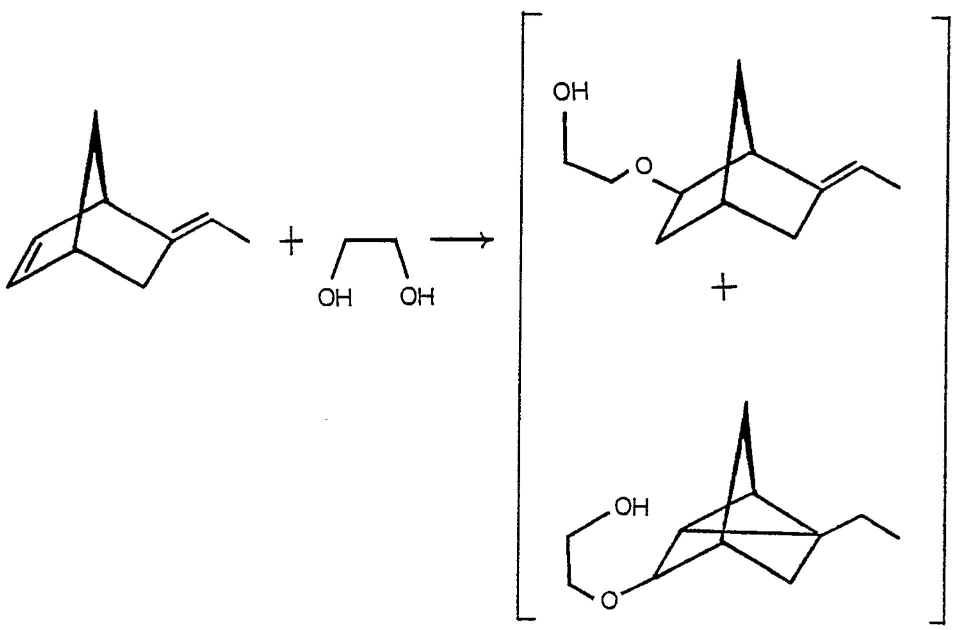

Ethylidene norbornene (480 grams) is added over a 4—1/2 hour period to a stirred solution of ethylene glycol (186 grams) and boron trifluoride etherate (15 ml) at 60°C. At the end of this period the resulting organic reaction mass is washed two times with 500 ml of water and washed once with 500 ml of 10% sodium carbonate solution.

The resulting organic layer is then distilled through a 1—1/2″ x 12″ Goodloe packed column to afford 168 grams of product (b.p. 100°C at 2 mm/Hg. pressure).

Figure 7 sets forth the GLC profile of the crude reaction product (conditions: 180°C, isothermal, 10′ x 1/4″, 10% SE—30 packed column).

Figure 8 sets forth the NMR spectrum for the reaction product consisting of 2-ethyl-5-(β-hydroxy-ethoxy)-tricyclo-[2.2.1.$0^{(2,6)}$]heptane and 2-ethylidene-6-(β-hydroxyethoxy)-norbornane having, respectively, the structures:

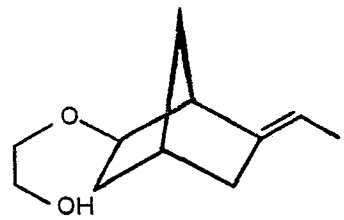

and

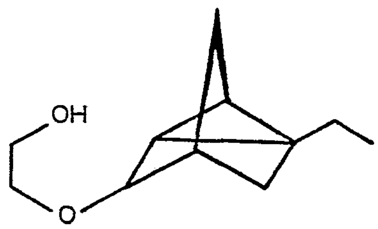

Figure 9 sets forth the infra-red spectrum for the product mixture consisting of 2-ethyl-5-(β-hydroxyethoxy)-tricyclo[2.2.1.0$^{(2,6)}$]heptane and 2-ethylidene-6-(β-hydroxyethoxy)-norbornane.

Example IV

*Preparation of a mixture consisting of 2-ethoxy-5-hexyloxytricyclo[2.2.1.0$^{(2,6)}$]heptane and 2-ethylidene-6-hexyloxynorbornane*

Reaction:

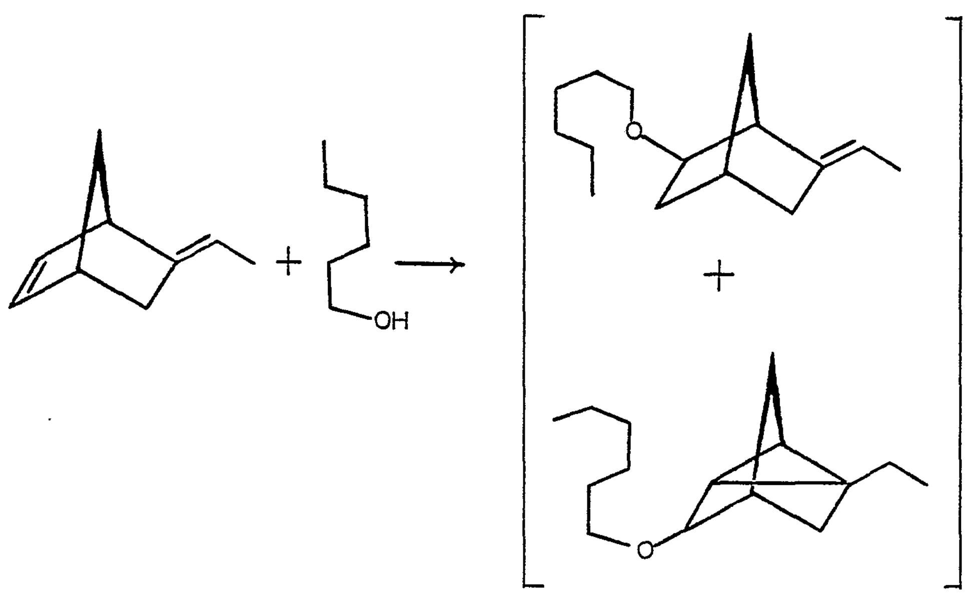

Ethylidene norbornene (480 grams) is added over a two hour period to a stirred solution of hexyl alcohol (510 grams) and boron trifluoride etherate at 95°C. The reaction mass is poured into one liter of water and the organic layer is subsequently washed with 500 mls of a 10% sodium carbonate solution. Distillation through a 1—1/2" x 12" Goodloe packed column affords 523 grams of product (b.p. 6°C at 1 mm/Hg pressure).

Figure 10 represents the GLC profile for the crude reaction product (conditions: 180°C, isothermal, 10' x 1/4" 10%, SE—30 packed column).

Figure 11 sets forth the NMR spectrum of the product mixture consisting of the compounds having the structures:

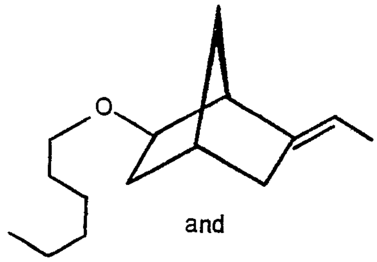

and

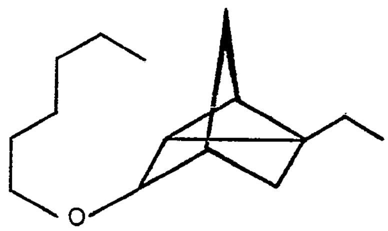

Figure 12 sets forth the infra-red spectrum of the product mixture consisting of the compounds having the structures:

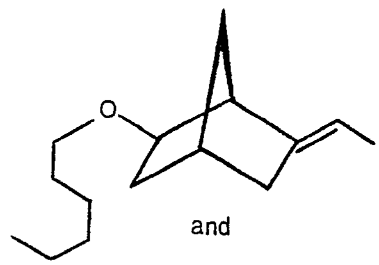

and

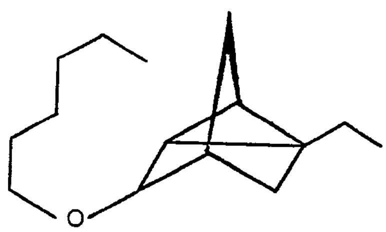

Example V

*Preparation of a mixture consisting of 2-ethyl-5-s-butyloxytricyclo[2.2.1.0$^{(2,6)}$]heptane and 2-ethylidene-6-s-butyloxynorbornane*

Reaction:

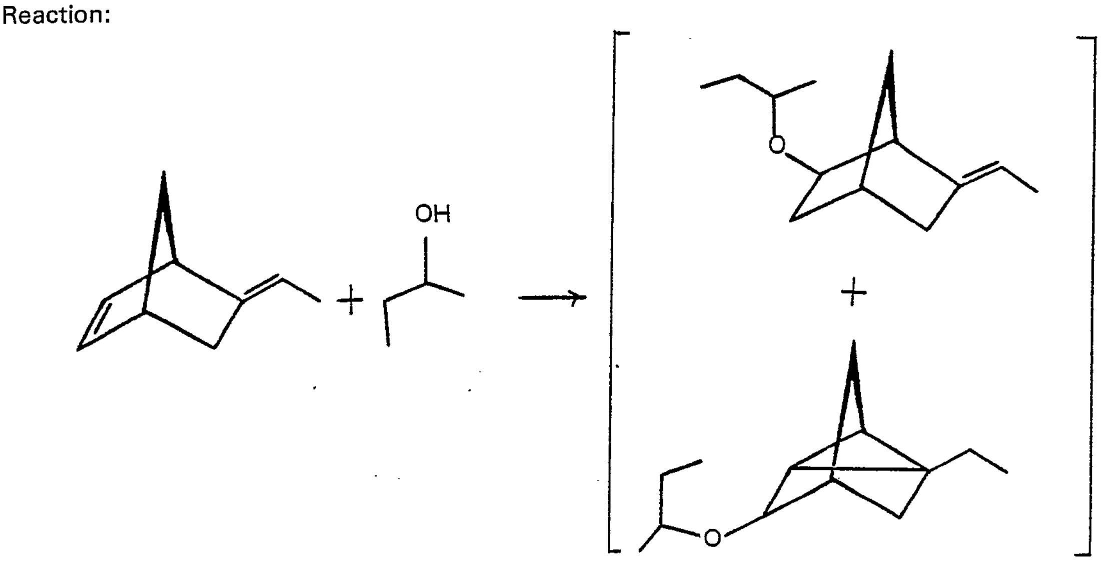

Ethylidene norbornene (480 grams) is added over a two hour period to a stirred solution of s-butyl

alcohol (444 grams) and boron trifluoride etherate at 85°C. The reaction mass is poured into one liter of water and the organic layer is washed with 500 ml of a 10% sodium carbonate solution. Distillation through a 1′ x 1—1/2″ Goodloe packed column affords 513 grams of product (b.p. 78°C at 5 mm/Hg. pressure).

Figure 13 sets forth the NMR spectrum of the product mixture consisting of the compounds having the structures:

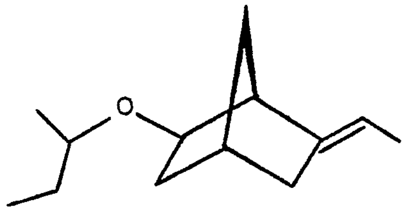

and

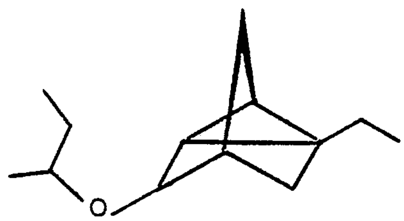

Figure 14 sets forth the infra-red spectrum of the product mixture consisting of the compounds having the structures:

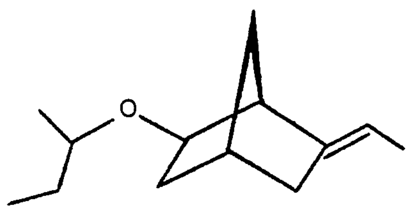

and

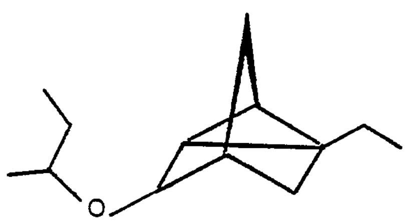

Example VI

*Preparation of a mixture consisting of 2-ethyl-5-(β-methoxyethoxy)tricyclo[2.2.1.0[(2,6)]]heptane and 2-ethylidene-6-(β-methoxyethoxy)norbornane*

Reaction:

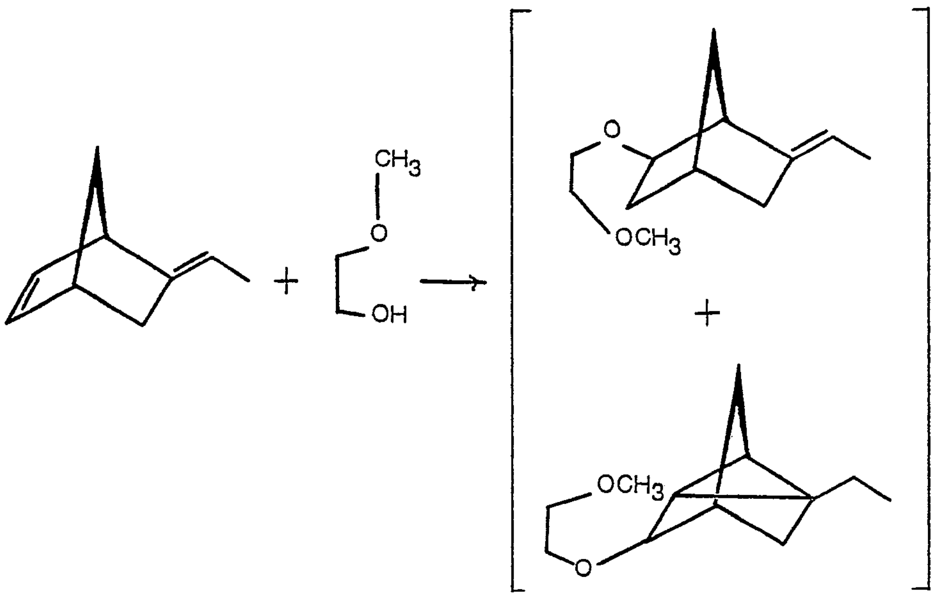

Ethylidene norbornene (360 grams) is added to a stirred solution of β-methoxyethanol (304 grams) and boron trifluoride etherate (30 ml) at 60°C over a two hour period. The reaction mass is then poured into 500 ml of water and washed with 10% sodium carbonate solution. Distillation through a 1" x 12" Goodloe packed column affords 348 grams of product (b.p. 75°C at 0.3 mm/Hg.).

Figure 15 sets forth GLC profile of the crude reaction product (conditions: 180°C, isothermal, 10' x 1/4" 10% SE—30 packed column).

Figure 16 sets forth the MMR spectrum of the product mixture consisting of the compounds having the structures:

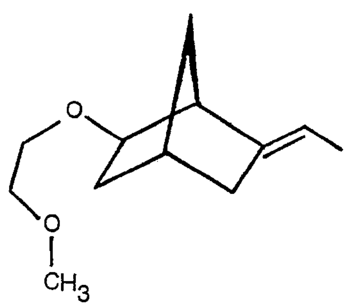

and

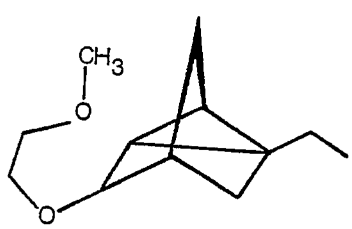

Figure 17 sets forth the infra-red spectrum of the product mixture consisting of the compounds having the structures:

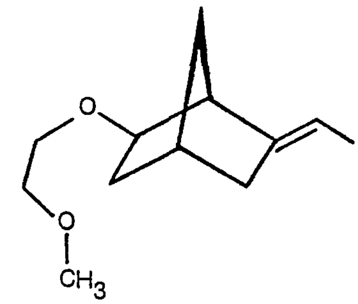

and

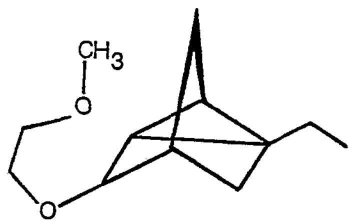

Example VII

*Preparation of a mixture consisting of 2-ethyl-5-isoamyloxytricyclo[2.2.1.$0^{(2,6)}$]heptane and 2-ethylidene-6-isoamyloxynorbornane*

Reaction:

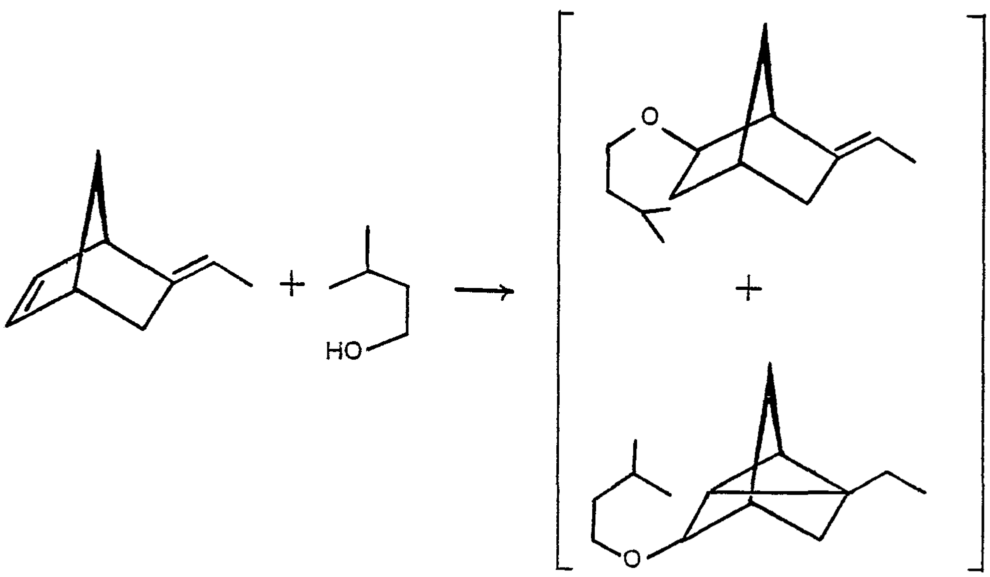

Ethylidene norbornene (408 grams) is added over a two hour period to a solution of isoamyl alcohol (360 grams) and boron trifluoride etherate (30 ml) at 75°C. The reaction mixture is heated at 75°C for a period of two hours whereupon it is quenched with one liter of water. The reaction mass is then washed with a 10% sodium carbonate solution. Distillation through a 1—1/2" x 12" Goodloe packed column affords 446 grams of product (b.p. 79°C at 0.4 mm/Hg. pressure).

Figure 18 represents the GLC profile of the crude reaction product (conditions: 220°C, isothermal, 10' x 1/4" 10% SE—30 packed column).

Figure 19 sets forth the NMR spectrum of the product mixture consisting of the compounds having the structures:

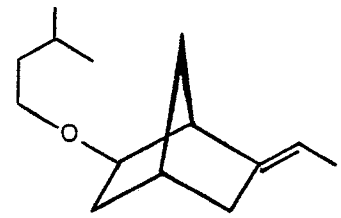

and

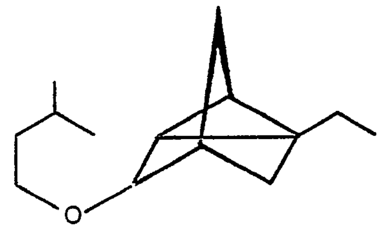

Figure 20 sets forth the infra-red spectrum of the product mixture consisting of the compounds having the structures:

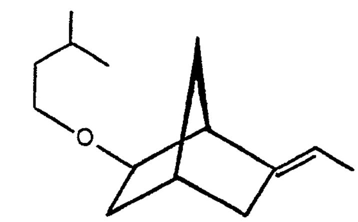

and

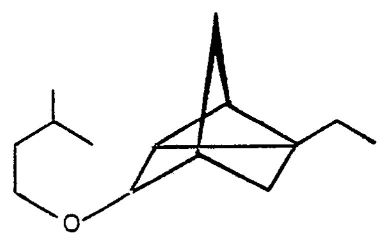

Example VIII

*Preparation of a mixture consisting of 2-ethyl-5-phenethyloxytricyclo[2.2.1.$0^{(2,6)}$]heptane and 2-ethylidene-6-phenethyloxynorbornane*

Reaction:

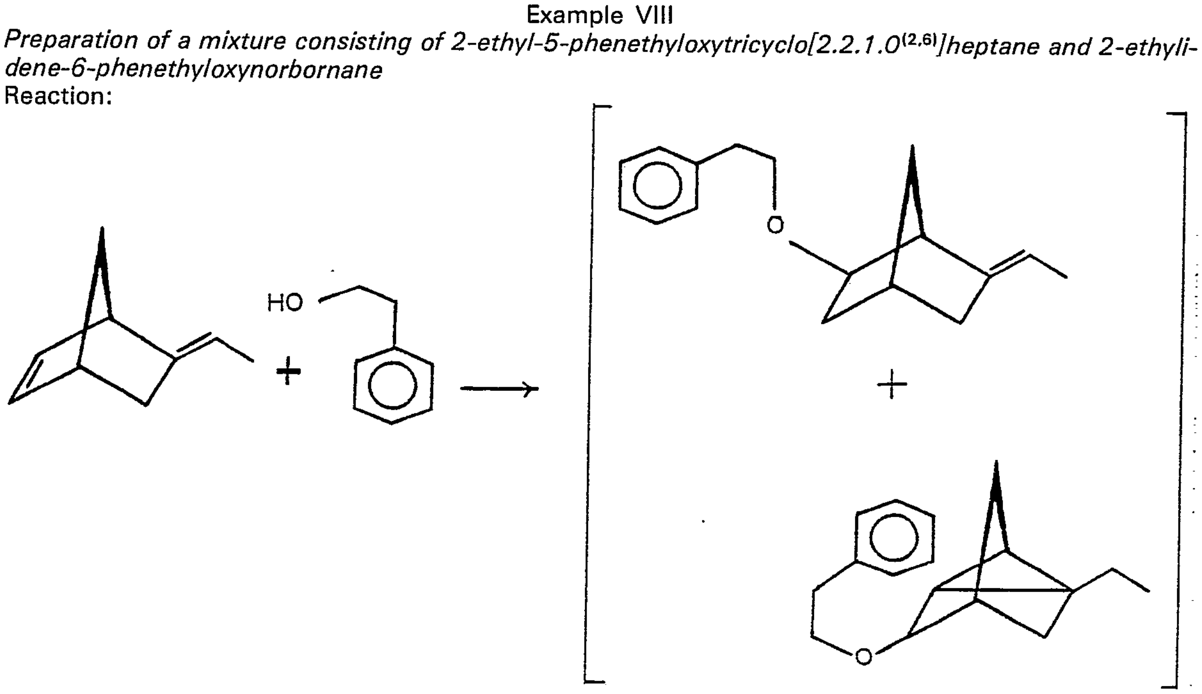

Ethylidene norbornane (427 grams) is added over a two hour period to a solution of phenylethyl alcohol (379 grams) and boron trifluoride etherate (30 ml). The reaction mass is then heated to 100°C for 90 minutes. Water (one liter) is added and the reaction mass is washed with a 10% sodium carbonate solution. Distillation through a 1—1/2" x 12" Goodloe packed column affords 192 grams of product (b.p.: 105°C—128°C at 0.7 mm/Hg pressure).

Figure 21 represents the GLC profile of the crude reaction product (conditions: 220°C, isothermal; 10' x 1/4" 10%, SE—30 packed column).

Figure 22 sets forth the NMR spectrum of the product mixture consisting of the compounds having the structures:

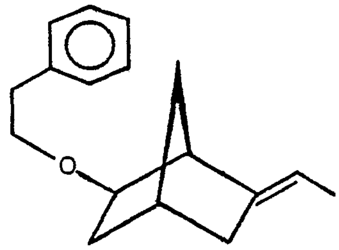

and

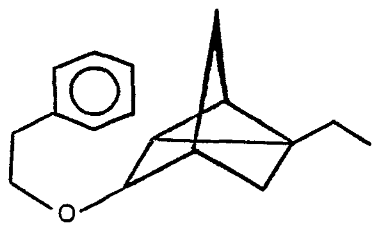

Figure 23 sets forth the infra-red spectrum of the product mixture consisting of the compounds having the structures:

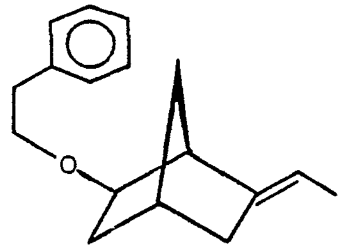

and

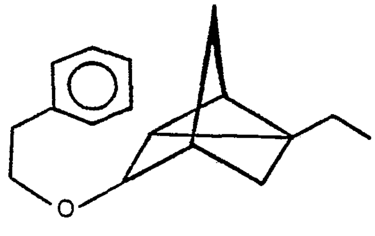

Example IX

*Preparation of a mixture consisting of 2-ethyl-5-(1',3'-dimethylbutyloxy)tricyclo[2.2.1.0$^{(2,6)}$]heptane and 2-ethylidene-6-(1',3'-dimethylbutyloxy)norbornane*

Reaction:

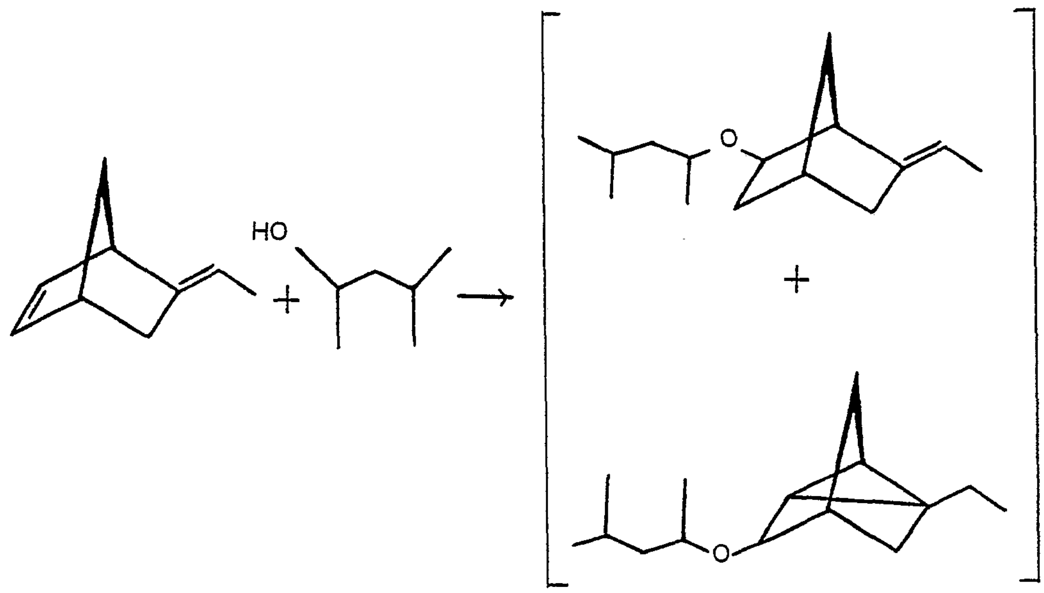

Ethylidene norbornene (360 grams) is added to a solution of 4-methyl-2-pentanol (408 grams) and boron trifluoride etherate (30 ml) over a two hour period at 60°C. The reaction mass is then heated at 75°C for two hours and quenched in 500 ml of water. The resulting organic layer is washed with 500 ml of a 10% sodium carbonate solution. Distillation through a 1—1/2" x 12" Goodloe packed column yields 236 grams of product (b.p. 70°C at 0.4 mm/Hg. pressure).

Figure 24 sets forth the GLC profile of the crude reaction mixture (conditions: 220°C, isothermal; 10' x 1/4", 10% SE—30 packed column).

Figure 25 sets forth the NMR spectrum of the product consisting of the compounds having the structures:

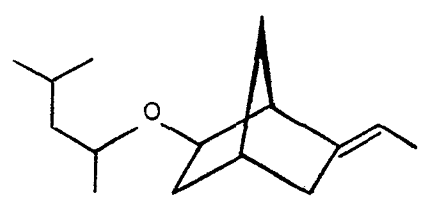

and

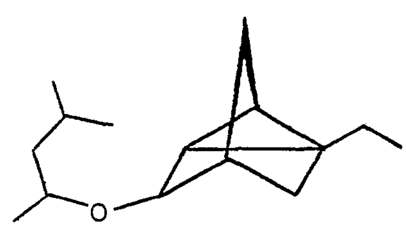

Figure 26 sets forth the infrared spectrum of the reaction product consisting of the compounds having the structures:

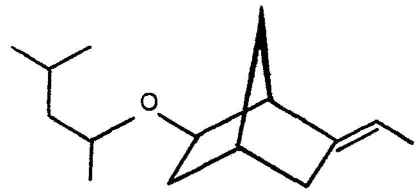

and

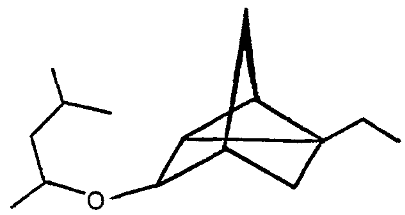

Example X

*Preparation of a mixture consisting of 2-ethyl-5-isobutyloxytricyclo[2.2.1.*$O^{(2,6)}$*]heptane and 2-ethylidene-6-isobutyloxynorbornane*

Reaction:

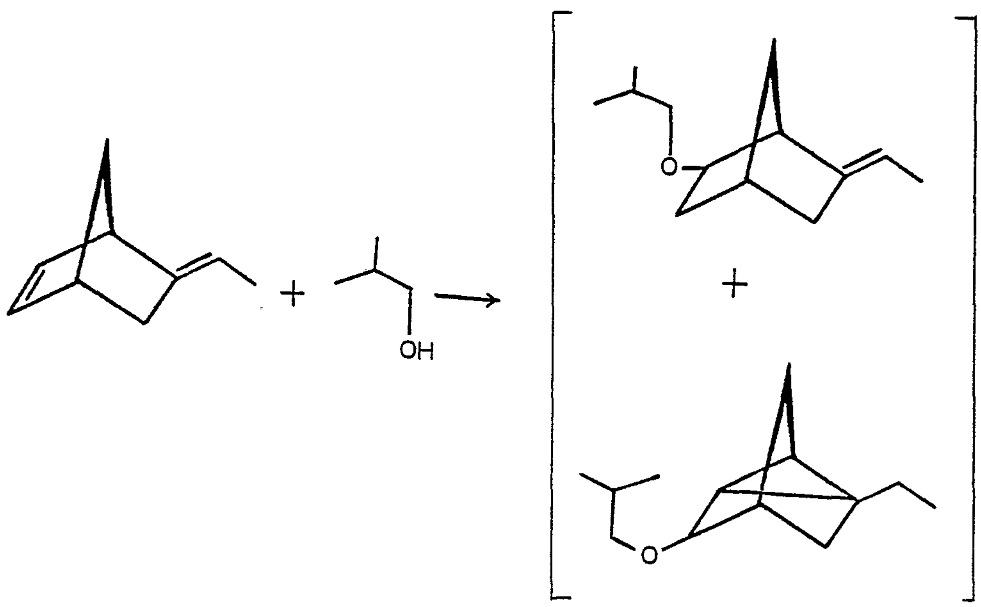

Ethylidene norbornene (480 grams) is added to a solution of isobutanol (407 grams) and boron trifluoride etherate at 80°C over a 90 minute period. The reaction mass is stirred at 80°C for one hour whereupon 700 ml of water is added thereto. The organic layer is washed with 500 ml of a 10% sodium carbonate solution and distilled through a 1/2" x 12" Goodloe packed column to afford 662 grams of product (b.p. 55°C at 1 mm/Hg. pressure).

Figure 27 represents the GLC profile of the crude reaction product (conditions:180°C, isothermal, 10' x 1/4" 10% SE—30 packed column).

Figure 28 sets forth the NMR spectrum of the product mixture consisting of the compounds having the structures:

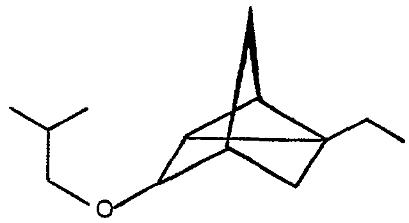

and

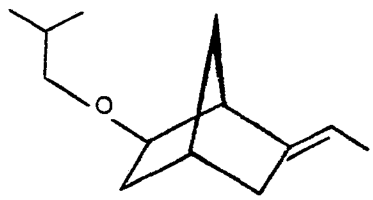

Figure 29 sets forth the infra-red spectrum of the reaction product mixture consisting of the compounds having the structures:

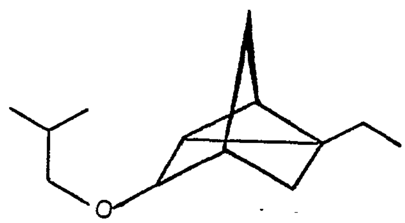

and

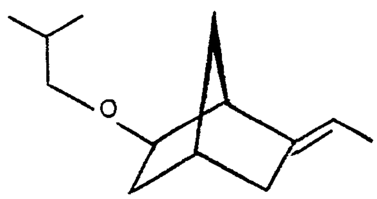

Example XI

*Preparation of a mixture consisting of 2-ethyl-5-cyclohexyloxytricyclo[2.2.1.0$^{(2,6)}$]heptane and 2-ethylidene-6-cyclohexyloxynorbornane*

Reaction:

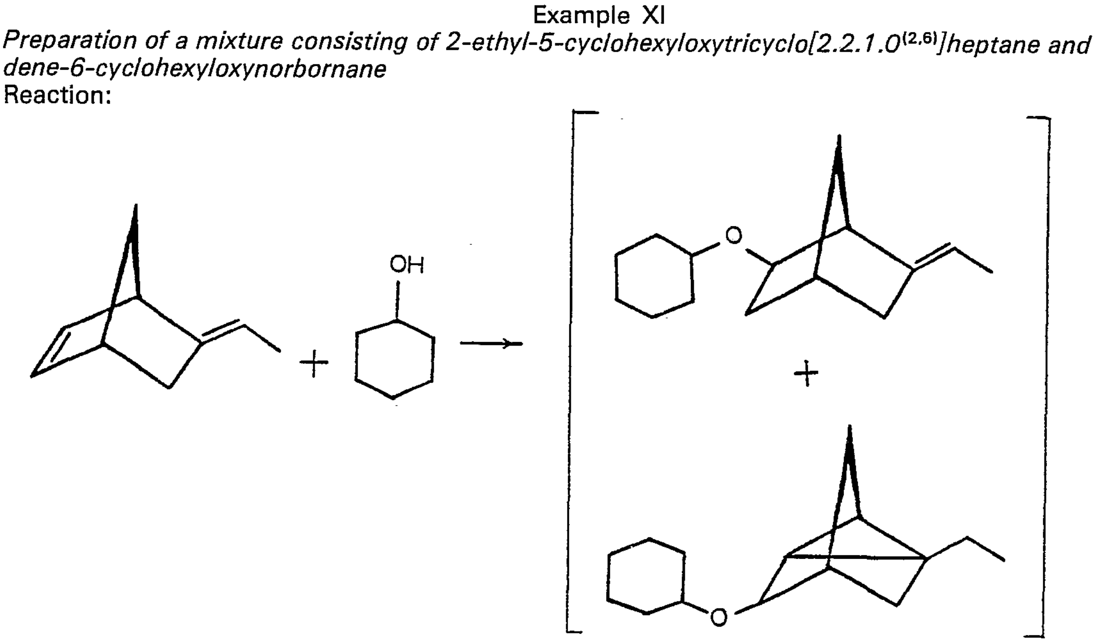

Ethylidene norbornene (360 grams) is added to a stirred solution of cyclohexanol (450 grams) and sulfuric acid (9 grams) at 80°C over a 90 minute period. The reaction mass is stirred at 90°C for a

further 90 minutes whereupon 500 ml of water are added thereto. The organic layer is washed with 500 ml of a 10% sodium carbonate solution and distilled through a 1—1/2" x 12" Goodloe packed column to afford 528 grams of product (b.p. 90°C at 0.9 mm/Hg. pressure).

Figure 30 represents the GLC profile of the crude reaction product (conditions: 180°C, isothermal, 10' x 1/4" SE—30 packed column).

Figure 31 represents the NMR spectrum of the reaction product mixture consisting of the compounds having the structures:

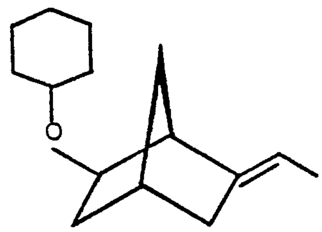

and

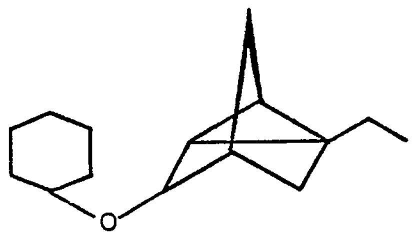

Figure 32 sets forth the infra-red spectrum of the reaction product mixture consisting of the compounds having the structures:

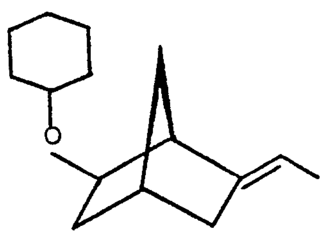

and

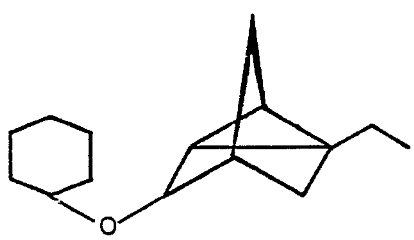

Example XII

*Preparation of norbornyl methyl ether of prior art*

Reaction:

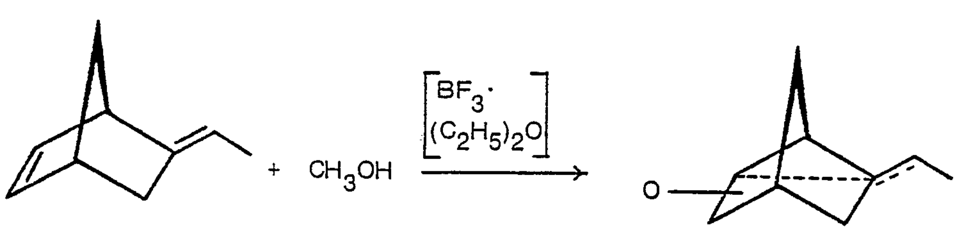


wherein in the mixture produced in each of the compounds, one of the dashed lines is a carbon-carbon bond and the other of the dashed lines represents no bond.

Into a one liter reaction flask equipped with stirrer, thermometer, reflux condenser and heating mantle is placed 20 grams of boron trifluoride diethyl ether complex and 192 grams of anhydrous methanol. The resulting mixture is heated to reflux (approximately 61 °C). Over a period of one hour, dropwise, is added to the reaction mass, 480 grams of ethylidene norbornene (4 moles). The reaction mass is stirred for a period of four hours at reflux. The reaction mass is then quenched with 400 ml water. The resulting two phases are separated and the oil phase is washed with 200 ml of 10% sodium hydroxide solution. The reaction product is then distilled on a 12″ x 1.5″ Goodloe column yielding the following fractions:

| Fraction Number | Vapor Temp. (°C) | Liquid Temp. (°C) | Pressure mm/Hg. | Reflux Ratio | Weight of Fraction (grams) |
|---|---|---|---|---|---|
| 1 | 35/54 | 58/62 | 11 | 4:1 | 58.4 |
| 2 | 52 | 65 | 11 | 4:1 | 31.8 |
| 3 | 54 | 64 | 10 | 4:1 | 31.7 |
| 4 | 57 | 64 | 10 | 100% | 45.6 |
| 5 | 57 | 63 | 10 | 100% | 44.7 |
| 6 | 57 | 64 | 10 | 100% | 48.5 |
| 7 | 58 | 65 | 10 | 100% | 46.7 |
| 8 | 58 | 66 | 10 | 100% | 54.7 |
| 9 | 58 | 68 | 10 | 100% | 52.6 |
| 10 | 60 | 93 | 10 | 100% | 53.1 |
| 11 | 60 | 101 | 10 | 100% | 36.3 |
| 12 | 61 | 112 | 10 | 100% | 4.7 |

Fractions 6—10 are bulked for evaluation

Figure 33 is the GLC profile for the reaction mass just prior to distillation.

Figure 35 is the NMR spectrum for ethylidene norbornene methyl ether, for peak 2 of the GLC profile of figure 33 and it is the NMR spectrum for the compound having the structure:

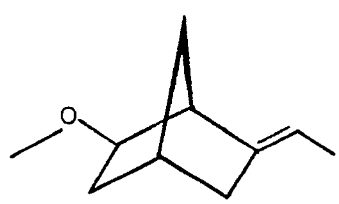

Figure 34 is the NMR spectrum for ethylidene norbornene methyl ether, peak 1 of the GLC profile of figure 33 and it is for the compound having the structure:

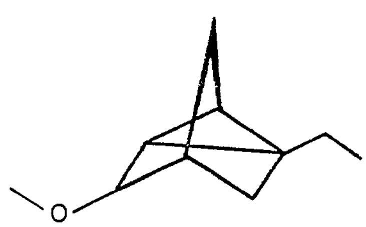

The mixture prior to distillation has a very powerful solventy, harsh, metallic, ketonic aroma profile. Peak 1 having the structure:

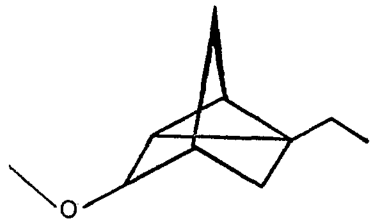

has a solventy (low molecular weight ketone-like), low-keyed, dusty, slightly woody, heptaldehyde-like aroma profile.

The compound having the structure:

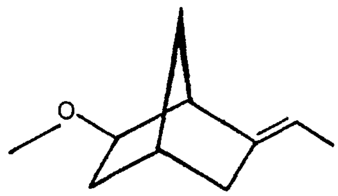

has a harsh, green, ketonic, slight linalyl-like aroma profile.

Example XIII

*Preparation of norbornyl ethyl ether of the prior art*

Reaction:

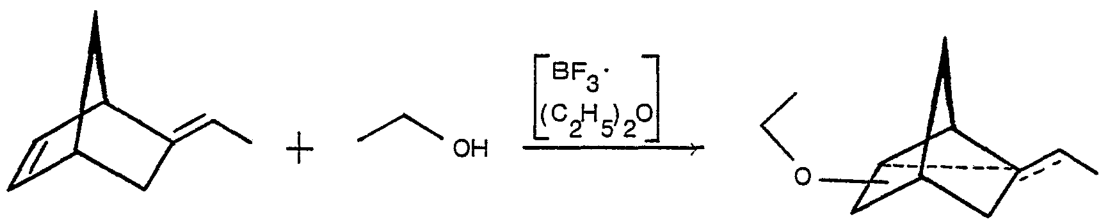


wherein in the resulting mixture in each of the molecules, one of the dashed lines represents a carbon-carbon bond and the other of the dashed lines represents no bond.

Into a one liter reaction flask equipped with reflux condenser, stirrer, thermometer and heating mantle is placed 276 grams of anhydrous ethanol (6 moles) and 20 ml boron trifluoride diethyl ether complex. The resulting mixture is heated to reflux and over a forty minute period while refluxing, ethylidene norbornene (480 grams; 4 moles) is added to the reaction mass. The reaction mass is then refluxed for a period of 2.5 hours. At the end of the refluxing period, 300 ml water is added to the reaction mass. The resulting mixture separates into two phases, an aqueous phase and an organic phase. The organic phase is separated and washed with 200 ml 10% NaOH solution. The resulting product is then dried and distilled on a 12" x 1.5" Goodloe column yielding the following fractions:

| Fraction Number | Vapor Temp. (°C) | Liquid Temp. (°C) | Pressure mm/Hg. | Reflux Ratio | Weight of Fraction (grams) |
|---|---|---|---|---|---|
| 1 | 28/62 | 119/71 | 10 | 4.1 | 49.0 |
| 2 | 61 | 72 | 10 | 4:1 | 34.3 |
| 3 | 61 | 72 | 10 | 4:1 | 45.0 |
| 4 | 66 | 73 | 10 | 100% | 48.0 |
| 5 | 67 | 73 | 10 | 100% | 52.0 |
| 6 | 67 | 73 | 10 | 100% | 57.2 |
| 7 | 67 | 73 | 10 | 100% | 55.4 |
| 8 | 68 | 77 | 10 | 100% | 41.0 |
| 9 | 69 | 78 | 10 | 100% | 56.5 |
| 10 | 70 | 80 | 10 | 100% | 47.4 |
| 11 | 70 | 130 | 10 | 100% | 56.5 |
| 12 | 68 | 137 | 10 | 100% | 22.0 |

Fractions 7—10 are bulked

Figure 36 is the GLC profile for the reaction mass after 2 hours and 40 minutes of reaction.

Figure 38 is the NMR spectrum for ethylidene norbornene peak 2 and is for the compound having the structure:

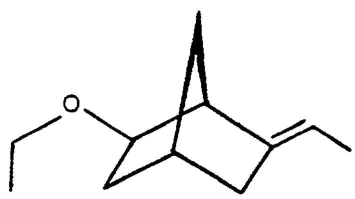

Figure 37 is the NMR spectrum for ethylidene norbornene ethyl ether, peak 1 of the GLC profile of figure 36 and it is for the compound having the structure:

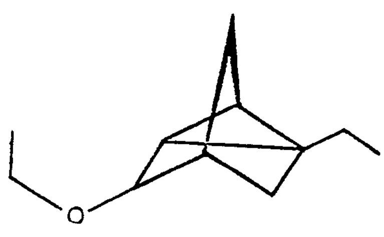

The mixture containing the compounds having the structures:

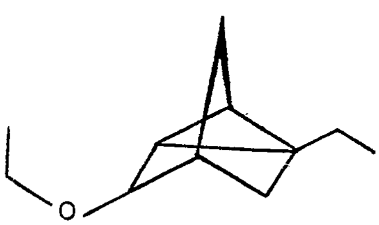

and

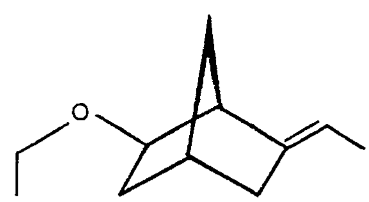

has a green, gassy aroma with catonic (e.g., acetone-like) notes; lacking green bean and lemon-like notes. The compound having the structure:

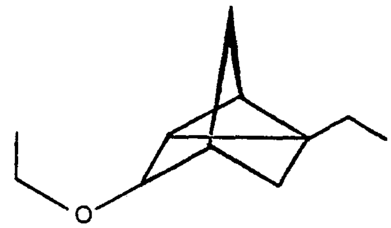

has a very volatile green, aniseed-like aroma. The compound having the structure:

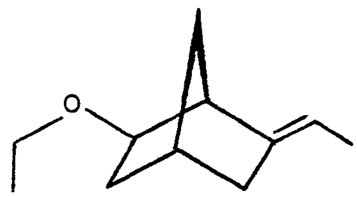

has a pyridene-like, fruity, nutty, dirty hair-like aroma with camphoraceous undertones.

Example XIV

*Preparation of n-propyl ether of norbornene*

Reaction:

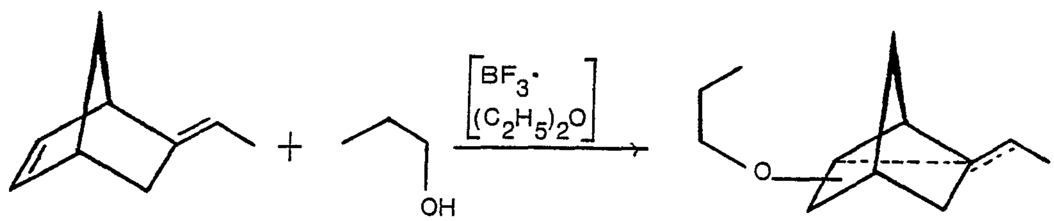


wherein in the mixture in each of the molecules, one of the dashed lines represents a carbon-carbon bond and the other of the dashed lines represents no bond.

Into a one-liter reaction flask equipped with stirrer, thermometer, reflux condenser and heating mantle, is placed 348 grams (6 moles) of n-propanol and 20 ml boron trifluoride diethyl ether complex. The resulting mixture is heated to reflux (95°C). Over a 45 minute period, is added 480 grams (4 moles) of ethylidene norbornene dropwise. The reaction mass is then quenched with water. The resulting organic phase is separated from the aqueous phase and the organic phase is washed with 200 ml 10% sodium hydroxide. The resulting product is dried and distilled on a 12″ x 1.5″ Goodloe column to yield the following fractions:

| Fraction Number | Vapor Temp. (°C) | Liquid Temp. (°C) | Pressure mm/Hg. | Reflux Ratio | Weight of Fraction (grams) |
|---|---|---|---|---|---|
| 1 | 26/78 | 54/87 | 10 | 4:1 | 58.7 |
| 2 | 78 | 88 | 10 | 4:1 | 45.0 |
| 3 | 78 | 88 | 10 | 4:1 | 46.8 |
| 4 | 79 | 89 | 10 | 4:1 | 56.5 |
| 5 | 78 | 92 | 10 | 4:1 | 38.6 |
| 6 | 81 | 95 | 10 | 100% | 113.3 |
| 7 | 81 | 97 | 10 | 100% | 80.7 |
| 8 | 81 | 120 | 10 | 100% | 79.9 |
| 9 | 70 | 175 | 10 | 100% | 43.8 |

Fractions 4—7 are bulked

Figure 39 is the GLC profile of the reaction product subsequent to quenching but prior to distillation.

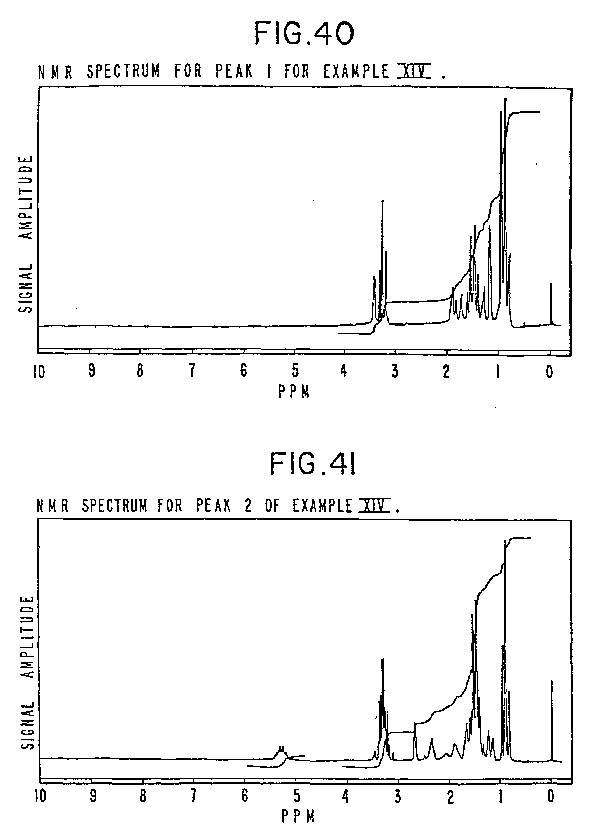

Figure 40 is the NMR spectrum for ethylidene norbornene n-propyl ether, peak 1 of the GLC profile of figure 39 and it is for the compound having the structure:

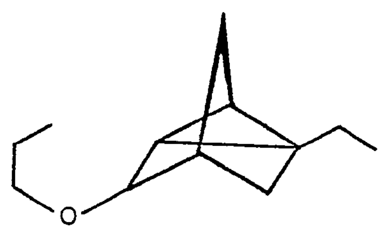

Figure 41 is the NMR spectrum for ethylidene norbornene n-propyl ether, peak 2 and is for the compound having the structure:

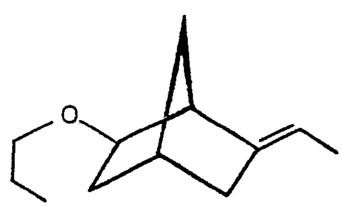

The mixture of compounds prior to distillation has a harsh, anise-like, sweet, metallic aroma lacking fresh, clean, herbaceous notes but having a strong fruity nuance. The compound having the structure:

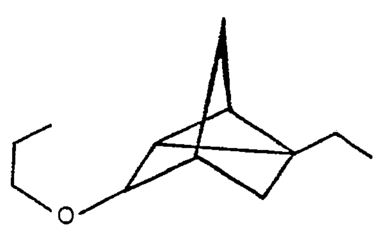

has an aniseed-like, woody aroma with a slight fruity character. The compound having the structure:

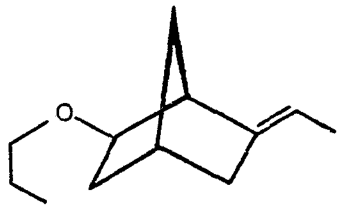

has a light, fresh, slightly herbaceous aroma which is relatively long-lasting.

Example XV

The following is prepared:

| Ingredients | Parts by Weight |
|---|---|
| Turpentine gum oil | 100 |
| Limonene | 70 |
| Gum camphor | 10 |
| Isobornyl acetate | 50 |
| Borneol | 30 |
| 2-(2-butenoyl)-3,3-dimethylnorbornane (produced according to Example XII of U.S. Patent No. 4,148,826) | 40 |
| Mixture of 2-(3-butenoyl)-3,3-dimethylnorbornane and 2-(2-butenoyl)-3,3-dimethylnorbornane (produced according to the process of Example III of U.S. Patent No. 4,148,826) | 100 |
| Alpha-allyl-3,3-dimethyl-2-norbornanemethanol (produced according to the process of Example II of U.S. Patent No. 4,148,826) | 70 |

EXAMPLE XV, (Continued)

| Ingredient | Example | Parts by Weight |
|---|---|---|
| The products produced according to Example I(A) or I(B) containing compounds having the structures: | "A" | 60 |
| | "B" | 0 |
| 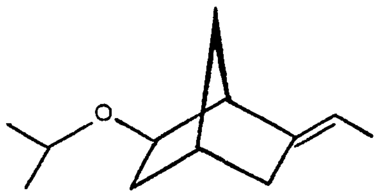 | "C" | 0 |
| | "D" | 0 |
| | "E" | 0 |
| | "F" | 0 |
| | "G" | 0 |
| and | "H" | 0 |
| 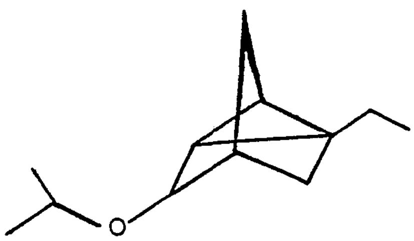 | "J" | 0 |
| | "K" | 0 |
| | "L" | 0 |
| | "M" | 0 |
| The product produced according to Example II, containing compounds having the structures: | "A" | 0 |
| | "B" | 80 |
| 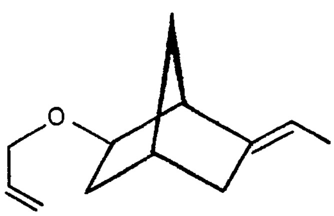 | "C" | 0 |
| | "D" | 0 |
| | "E" | 0 |
| | "F" | 0 |
| | "G" | 0 |
| and | "H" | 0 |
| 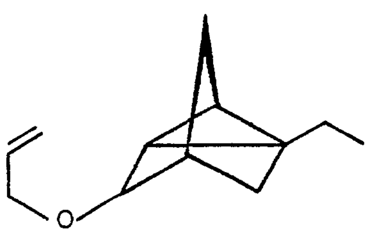 | "J" | 0 |
| | "K" | 0 |
| | "L" | 0 |
| | "M" | 0 |

EXAMPLE XV, (Continued)

| Ingredient | Example | Parts by Weight |
|---|---|---|
| The product produced according to Example III, containing compounds having the structures: | "A" | 0 |
| | "B" | 0 |
| 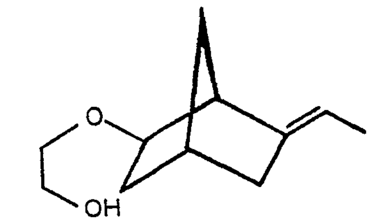 | "C" | 90 |
| | "D" | 0 |
| | "E" | 0 |
| | "F" | 0 |
| | "G" | 0 |
| and | "H" | 0 |
| 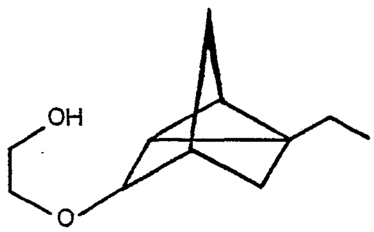 | "J" | 0 |
| | "K" | 0 |
| | "L" | 0 |
| | "M" | 0 |
| The product produced according to Example IV containing the compounds having the structures: | "A" | 0 |
| | "B" | 0 |
| 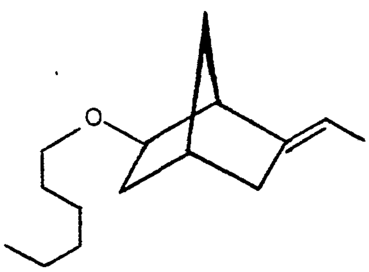 | "C" | 0 |
| | "D" | 90 |
| | "E" | 0 |
| | "F" | 0 |
| | "G" | 0 |
| and | "H" | 0 |
| 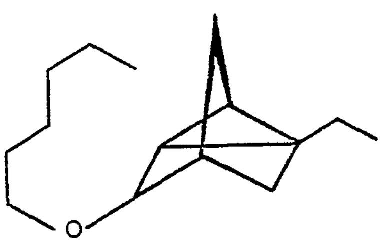 | "J" | 0 |
| | "K" | 0 |
| | "L" | 0 |
| | "M" | 0 |

EXAMPLE XV, (Continued)

| Ingredient | Example | Parts by Weight |
|---|---|---|
| The product produced according to Example V, containing the compounds having the structures: | "A" | 0 |
| | "B" | 0 |
| 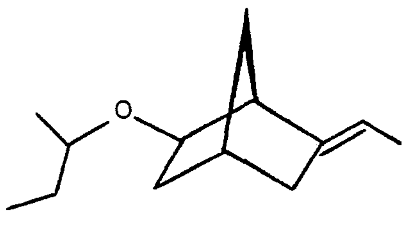 | "C" | 0 |
| | "D" | 0 |
| | "E" | 80 |
| | "F" | 0 |
| | "G" | 0 |
| and | "H" | 0 |
| | "J" | 0 |
| 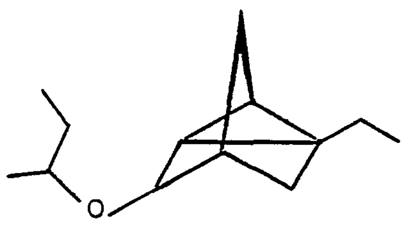 | "K" | 0 |
| | "L" | 0 |
| | "M" | |
| The product produced according to Example VI, containing the compounds having the structures: | "A" | 0 |
| | "B" | 0 |
| 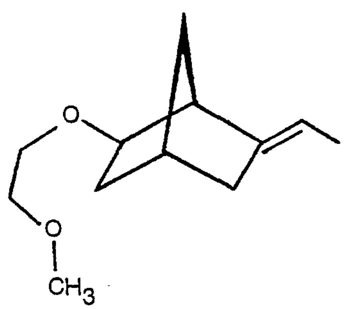 | "C" | 0 |
| | "D" | 0 |
| | "E" | 0 |
| | "F" | 80 |
| | "G" | 0 |
| | "H" | 0 |
| and | "J" | 0 |
| | "K" | 0 |
| 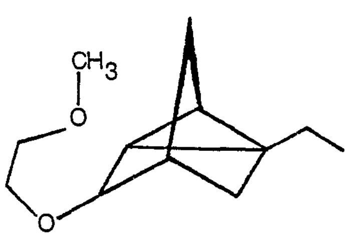 | "L" | 0 |
| | "M" | 0 |

EXAMPLE XV (Continued)

| Ingredient | Example | Parts by Weight |
|---|---|---|
| The product produced according to Example VII, containing the compounds having the structures: | "A" | 0 |
| | "B" | 0 |
| 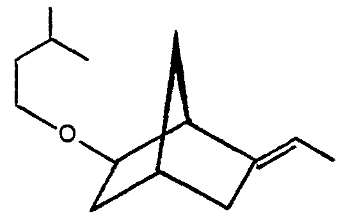 | "C" | 0 |
| | "D" | 0 |
| | "E" | 0 |
| | "F" | 0 |
| | "G" | 90 |
| and | "H" | 0 |
| 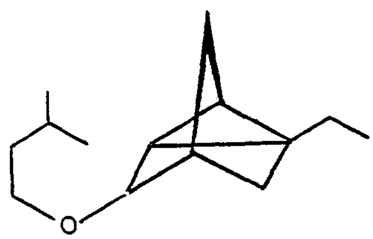 | "J" | 0 |
| | "K" | 0 |
| | "L" | 0 |
| | "M" | 0 |
| The product produced according to the process of Example VIII, containing the compounds having the structures: | "A" | 0 |
| | "B" | 0 |
| 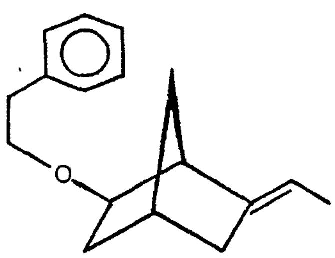 | "C" | 0 |
| | "D" | 0 |
| | "E" | 0 |
| | "F" | 0 |
| | "G" | 0 |
| | "H" | 80 |
| and | "J" | 0 |
| 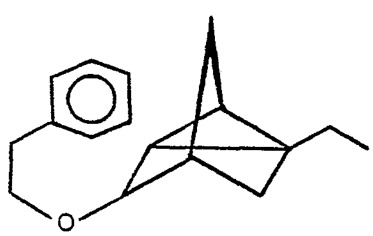 | "K" | 0 |
| | "L" | 0 |
| | "M" | 0 |

EXAMPLE XV, (Continued)

| Ingredient | Example | Parts by Weight |
|---|---|---|
| The product produced according to the process of Example IX, containing the compounds having the structures:<br>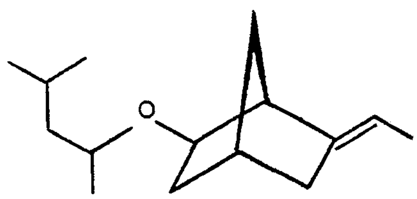<br>and<br>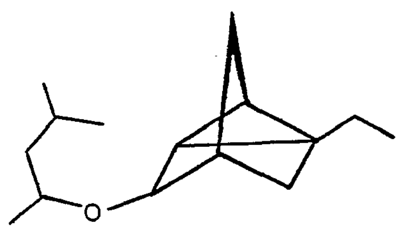 | "A" | |
| | "B" | 0 |
| | "C" | 0 |
| | "D" | 0 |
| | "E" | 0 |
| | "F" | 0 |
| | "G" | 0 |
| | "H" | 0 |
| | "J" | 70 |
| | "K" | 0 |
| | "L" | 0 |
| | "M" | 0 |
| The product produced according to the process of Example X, containing the compounds having the structures:<br>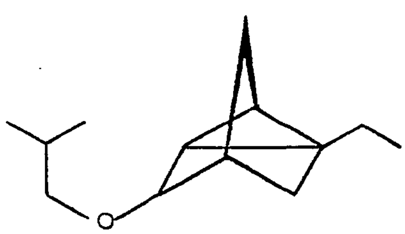<br>and<br>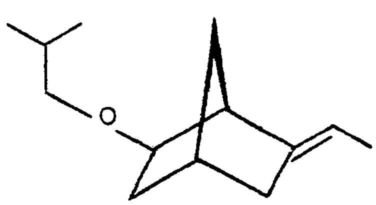 | "A" | 0 |
| | "B" | 0 |
| | "C" | 0 |
| | "D" | 0 |
| | "E" | 0 |
| | "F" | 0 |
| | "G" | 0 |
| | "H" | 0 |
| | "J" | 0 |
| | "K" | 80 |
| | "L" | 0 |
| | "M" | 0 |

EXAMPLE XV, (Continued)

| Ingredient | Example | Parts by Weight |
|---|---|---|
| The product produced according to the process of Example XI, containing the compounds having the structures: 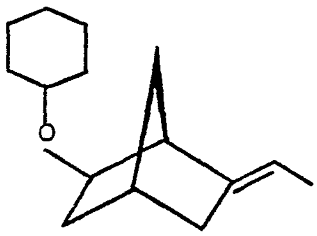 and 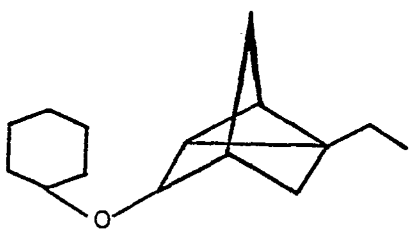 | "A" | 0 |
| | "B" | 0 |
| | "C" | 0 |
| | "D" | 0 |
| | "E" | 0 |
| | "F" | 0 |
| | "G" | 0 |
| | "H" | 0 |
| | "J" | 0 |
| | "K" | 0 |
| | "L" | 90 |
| | "M" | 0 |
| The product produced according to the process of Example XIV, containing the compounds having the structures: 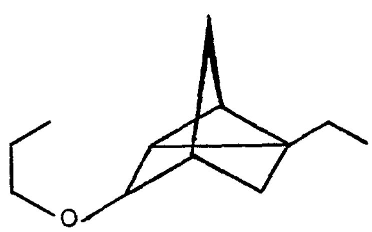 and 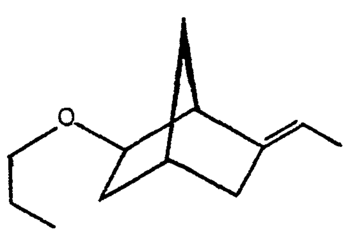 | "A" | 0 |
| | "B" | 0 |
| | "C" | 0 |
| | "D" | 0 |
| | "E" | 0 |
| | "F" | 0 |
| | "G" | 0 |
| | "H" | 0 |
| | "J" | 0 |
| | "K" | 0 |
| | "L" | 0 |
| | "M" | 95 |

- Each of Examples XV (A—M) has interesting pine needle oil aromas with various nuances described as follows:

TABLE II

| Example | Aroma Nuance |
|---|---|
| XV(A) | A fresh, green bean-like, rosey, citrus (petitgrain-like) aroma. |
| XV(B) | A fruity, anisic, green aroma. |
| XV(C) | A green, raw potato aroma with galbanum topnotes. |
| XV(D) | A long-lasting, green, twiggy, fruity and herbaceous aroma. |
| XV(E) | A sweet, fruity aroma. |
| XV(F) | An excellent green aroma. |
| XV(G) | A green, sweaty aroma. |
| XV(H) | A green pea-like and green aroma. |
| XV(J) | A green and chocolate-like aroma with anther-like undertones. |
| XV(K) | A floral, green and carrot-like aroma. |
| XV(L) | A green, creamy, stringbean-like, herbaceous and floral-rosey aroma with anise-like undertones. |
| XV(M) | A harsh, anise-like, sweet, metallic aroma having a strong, fruity nuance. |

Example XVI

*Preparation of a cosmetic powder preparation*

A cosmetic powder is prepared by mixing in a ball mill, 100 grams of talcum powder with 0.25 grams of one of the substances set forth in Table III below. The resulting cosmetic powder has a pleasant aroma as set forth in Table III below.

TABLE III

| Structure of Compound | Perfumery Evaluation |
|---|---|
| 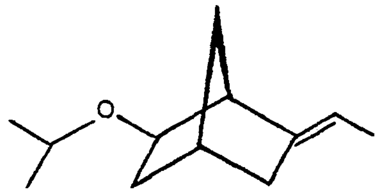<br>and<br>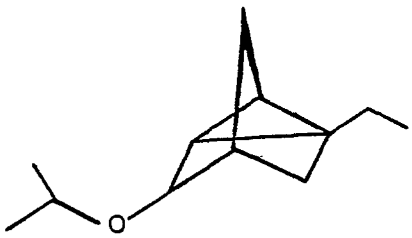<br>Produced according to Example I(A) or I(B) | A fresh, green bean-like, rosey, citrus (petitgrain-like) aroma. |
| 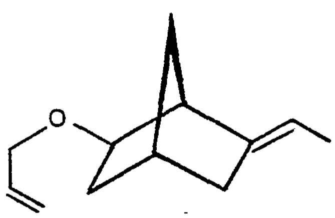<br>and<br>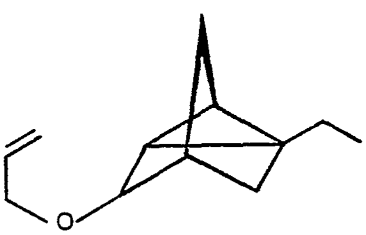<br>Produced according to Example II | A fruity, anisic, green aroma. |

TABLE III (Continued)

| Structure of Compound | Perfumery Evaluation |
|---|---|
| 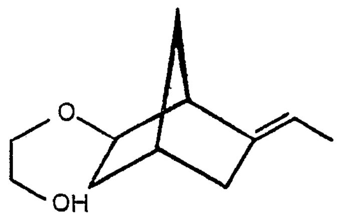<br>and<br>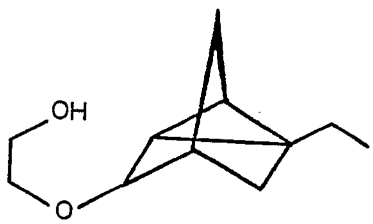<br>Produced according to Example III | A green, raw potato aroma with galbanum topnotes. |
| 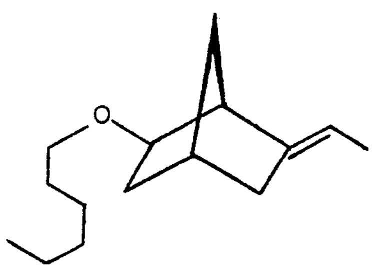<br>and<br>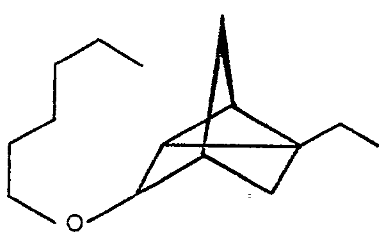<br>Produced according to Example IV | A long-lasting, green, twiggy, fruity and herbaceous aroma. |

TABLE III (Continued)

| Structure of Compound | Perfumery Evaluation |
|---|---|
| 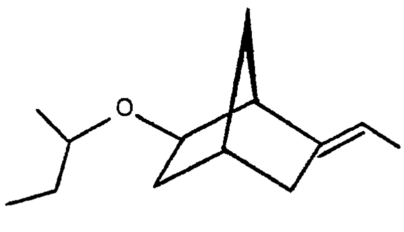<br>and<br>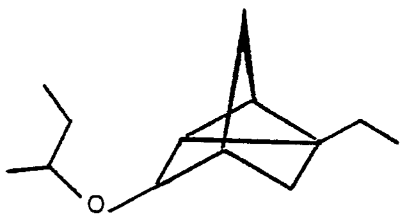<br>Produced according to Example V | A sweet, fruity aroma. |
| 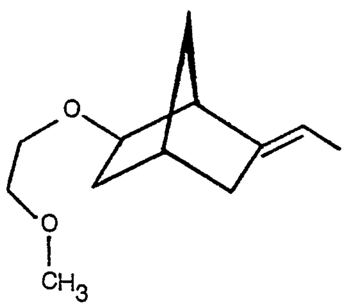<br>and<br>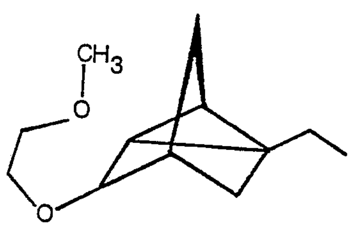<br>Produced according to Example VI | An excellent green aroma. |

TABLE III (Continued)

| Structure of Compound | Perfumery Evaluation |
| --- | --- |
| 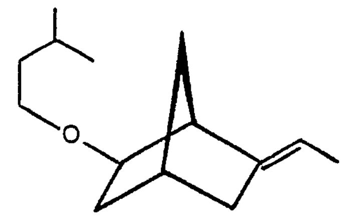<br>and<br>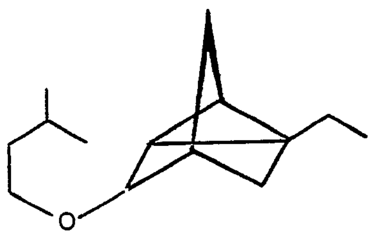<br>Produced according to Example VII | A green, sweaty aroma. |
| 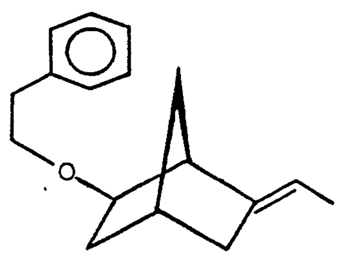<br>and<br>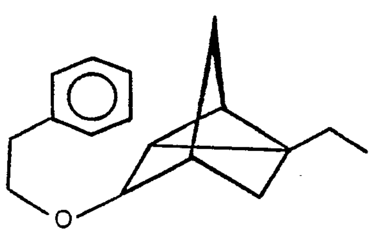<br>Produced according to Example VIII | A green pea-like and green aroma. |

TABLE III (Continued)

| Structure of Compound | Perfumery Evaluation |
|---|---|
| 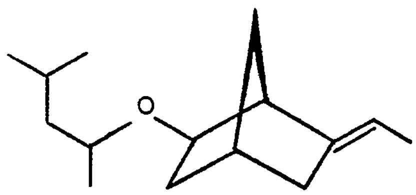<br>and<br>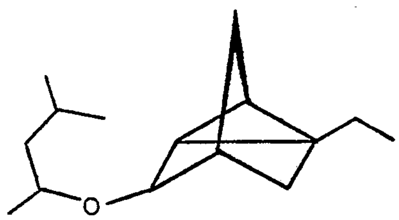<br>Produced according to Example IX | A green and chocolate-like aroma with anther-like undertones. |
| 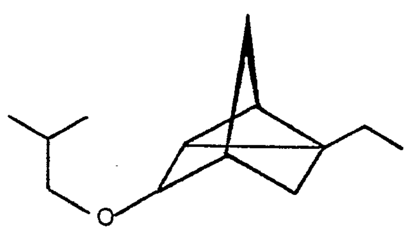<br>and<br>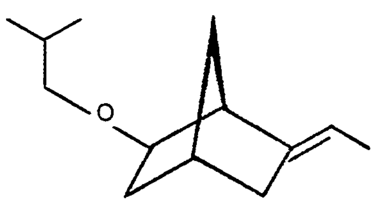<br>Produced according to Example X | A floral, green and carrot-like aroma. |

TABLE III (Continued)

| Structure of Compound | Perfumery Evaluation |
|---|---|
| 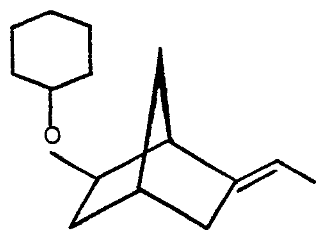 and 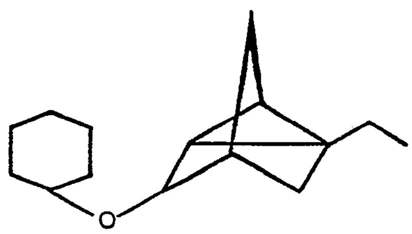 Produced according to Example XI | A green, creamy, string-bean-like, herbaceous and floral-rosey aroma with anise-like undertones. |
| 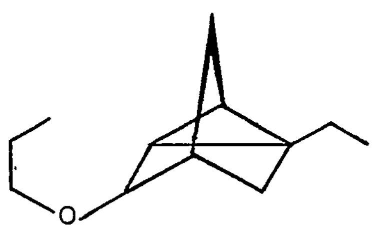 and 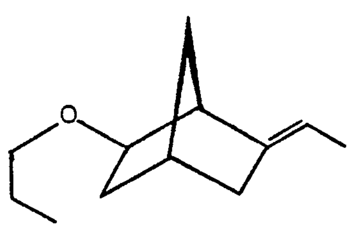 Produced according to Example XIV | A harsh, anise-like, sweet, metallic aroma having a strong, fruity nuance. |

Each of Examples XV(A—M) has interesting pine needle oil aromas with various nuances described as follows:

| Example | Aroma Nuance |
|---|---|
| XV(A) | A fresh, green bean-like rosey, citrus, (petitgrain-like) aroma. |
| XV(B) | A fruity, anisic, green aroma. |
| XV(C) | A green raw potato aroma with galbanum topnotes. |
| XV(D) | A long-lasting, green, twiggy, fruity and herbaceous aroma. |
| XV(E) | A sweet, fruity aroma. |
| XV(F) | An excellent green aroma. |
| XV(G) | A green, sweaty aroma. |
| XV(H) | A green pea-like and green aroma. |
| XV(J) | A green and chocolate-like aroma with anther-like undertones. |
| XV(K) | A floral, green and carrot-like aroma. |
| XV(L) | A green, creamy, stringbean-like, herbaceous and floral-rosey aroma with anise-like undertones. |
| XV(M) | A harsh, anise-like, sweet aroma having a strong, fruity nuance with a woody undertone. |

## Example XVII

Concentrated liquid detergents (lysine salt of n-dodecylbenzene sulfonic acid as more specifically described in U.S. Patent No. 3,948,818, issued on April 6, 1976) with fragrance profiles as defined in Table III of Example XVI are prepared containing 0.10%, 0.15%, 0.20%, 0.25%, 0.30% and 0.35% of the substance as set forth in Table III of Example XVI. They are prepared by adding and homogeneously mixing the appropriate quantity of substance as set forth in Table III of Example XVI in the liquid detergent. The detergents all posess excellent intense aromas as defined according to the profiles of Table III of Example XVI, the intensity increasing with greater concentrations of said substance as set forth in Table III of Example XVI.

## Example XVIII

*Preparation of a cologne and handkerchief perfume*

Substances set forth in Table III of Example XVI are each individually incorporated into colognes at concentrations of 2.0%, 2.5%, 3.0%, 3.5%, 4.0%, 4.5% and 5.0% in 75%, 80%, 85% and 90% aqueous food grade ethanol; and into handkerchief perfumes at concentrations of 15%, 20% and 30% in 95% aqueous food grade ethanol. Distinctive and definitive long-lasting warm aromas as defined according to Table III of Example XVI are all imparted to the cologne and to the handkerchief perfumes at all levels as indicated above.

## Example XIX

*Preparation of soap composition*

One hundred grams of soap chips (IVORY®, produced by the Procter & Gamble Company, Cincinnati, Ohio) are admixed with one gram of the substance as set forth in Table III of Example XVI until homogeneous compositions are obtained. In each of the cases, the homogeneous compositions are heated under three atmospheres pressure at 180°C for a period of three hours and the resulting liquids are placed into soap molds. The resulting soap cakes, on cooling, manifest excellent, long-lasting, warm aromas as set forth in Table III of Example XVI.

Example XX

*Preparation of solid detergent compositions*

Detergents are prepared from the following ingredients according to Example I of Canadian Patent No. 1,007,948:

| Ingredient | Percent by Weight |
|---|---|
| Neodol® 45—11 (a $C_{14}$—$C_{15}$ alcohol ethoxylated with 11 moles of ethylene oxide) | 12 |
| Sodium carbonate | 55 |
| Sodium citrate | 20 |
| Sodium sulfate, water brighteners | q.s. |

This detergent is a "phosphate-free" detergent. A total of 100 grams of said detergent is admixed with 0.10, 0.15, 0.20 and 0.25 grams of the substance as set forth in Table III of Example XVI. Each of the detergent samples has excellent, warm aromas as indicated in Table III of Example XVI.

Example XXI

Utilizing the procedure of Example I of column 15 of U.S. Patent No. 3,632,396, a non-woven cloth substrate useful as a dryer-added fabric softening article of manufacture prepared wherein the substrate, the substrate coating and the outer coating and the perfuming material are as follows:

1. A water "dissolvable" paper ("Dissolvo Paper")
2. Adogen 448 (m.p. about 140°F) as the substrate coating and
3. An outer coating having the following formulation (m.p. about 150°F):
   - 57% $C_{20}$—$C_{22}$HAPS
   - 22% Isopropyl alcohol
   - 20% antistatic agent
   - 1% of one of the substances as set forth in Table III of Example XVI

Fabric softening compositions containing substances as set forth in Table III of Example XVI essentially consist of a substrate having a weight of about 3 grams per 100 square inches of substrate coating, of about 1.85 grams per 100 square inches of substrate, and an outer coating of about 1.4 grams per 100 square inches of substrate, thereby providing a total aromatized substrate and outer coating weight ratio of about 1:1 by weight of the substrate. The aromas as set forth in Table III of Example XVI are imparted in a pleasant manner to the head space in the dryer on operation thereof, using the said dryer-added fabric softening non-woven fabric.

In the case of fabric softener articles, the entire U.S. Patent No. 3,632,396 is herewith incorporated by reference. Thus, all of the articles of 3,632,396 acting as fabric softener articles in said U.S. Patent may be perfumed in their outer coating with from 0.25% up to 5% by weight of the norbornyl ether derivatives of my invention.

In the following examples, Aromox® DMC—W and Aromox® DMMC-W are 30% aqueous solutions of dimethyl cocoamine oxide; and Aromox® NCMDW is a 40% aqueous solution of N-cocomorpholine oxide produced by Armac Division of AKZO of Chicago, Illinois.

Example XXII

Four drops of one of the substances set forth in Table III of Example XVI is added to two grams of Aromox® DMC-W to produce a clear premix. The clear premix is added to 200 grams of Clorox® with stirring resulting in a clear stable, single-phase solution. Sufficient 1M aqueous NaOH is added to bring the pH of the mixture up to 12.8. The solution remains substantially stable at 120°F for a period of seven days. When the 5% aqueous sodium hypochlorite solution is used as a laundry bleach, the resulting laundry, on dry-out in an atmosphere of 65% relative humidity yields substantially no characteristic "hypochlorite" odor, but does have a faint, pleasant aroma as set forth in Table III of Example XVI. Furthermore, no such characteristic "hypochlorite" aroma is retained on the hands of the individual handling such laundry in both the wet and the dry states.

Example XXIII

Aromox® DMMC-W in various quantities is mixed with 0.1 grams of one of the substances set forth in Table III of Example XVI. The resulting premixes are then added to 200 grams of an aqueous 5% sodium hypochlorite solution. Sufficient 12.5M aqueous NaOH is added to bring the pH of the mixture up to 13. The following results are obtained:

| Percentage Aromox® DMMC—W | Clarity of hypochlorite solution after addition of premix |
|---|---|
| 0.23% | Clear after three days |
| 0.15% | Clear after three days |
| 0.08% | Initially slightly turbid; two phase exist after three days. |

When the 5% aqueous sodium hypochlorite solution is used as a laundry bleach, the resulting laundry on dry-out, in an atmosphere of 65% relative humidity, yields substantially no characteristic "hypochlorite" odor, but does have a faint, pleasant aroma as set forth in Table III of Example XVI. Furthermore, no such characteristic "hypochlorite" aroma is retained on the hands of the individual handling such laundry in both the wet and the dry states.

## Example XVIV

Two grams of Aromox® DMMC-W is admixed with eight drops of one of the substances set forth in Table III of Example XVI. The premix is then added with stirring to 200 grams of a 7% aqueous solution of lithium hypochlorite. Sufficient 3M aqueous LiOH is added to bring the pH of the solution to 13.4. The mixture is then heated to 120°F and maintained at that temperature with stirring for a period of 1 week. The resulting solution remains clear in a single phase. When used as a laundry bleach, the resulting bleached laundry, on dry-out in an atmosphere of 50% relative humidity, retains a "clean" warm aroma as set forth in Table III of Example XVI; whereas without the use of the substance set forth in Table III of Example XVI the bleached laundry has a faint characteristic disagreeable "hypochlorite" aroma.

## Example XXV

Two grams of Aromox® DMMC-W is admixed with eight drops of one of the substances of Table III of Example XVI. This premix is then added, with stirring, to 200 grams of a mixture containing 4.5% aqueous sodium hypochlorite and 4.5% aqueous lithium hypochlorite. Sufficient 4M aqueous LiOH is added to bring the pH of the solution to 13.4. The mixture is then heated to 120°F and maintained at that temperature for a period of one week. The resulting solution remains clear in a single phase. When used as a laundry bleach, the resulting bleached laundry on dry-out in an atmosphere of 50% relative humidity retains a "clean fresh" warm aroma as set forth in Table III of Example XVI; whereas without the use of the substance set forth in Table III of Example XVI, the bleached laundry has a faint characteristic disagreeable "hypochlorite" aroma.

## Example XXVI

Two grams of Aromox® DMMC-W is admixed with eight drops of one of the substances set forth in Table III of Example XVI. This premix is then added with stirring to 200 grams of a mixture containing 4.5% aqueous sodium hypochlorite and 4.5% aqueous lithium hypochlorite. Sufficient 2M aqueous NaOH is added to bring the pH of the solution to 13.4. The mixture is then heated to 100°F and maintained at that temperature with stirring for a period of 2 weeks. The resulting solution remains clear as a single phase when used as a laundry bleach. The resulting laundry bleach. on dry-out in an atmosphere of 50% relative humidity, retains an aroma as set forth in Table III of Example XVI, whereas without the use of the substance set forth in Table III of Example XVI, the bleached laundry has a faint characteristic disagreeable "hypochlorite" aroma.

## Example XXVII

Four drops of one of the substances set forth in Table III of Example XVI is added to 1.5 grams of Aromox® to produce a clear premix. The clear premix is added to 200 grams of Clorox® with stirring resulting in a clear stable single phase solution. Sufficient 1M aqueous NaOH is added to bring the pH of the mixture up to 12.8. The solution remains substantially stable at 120°F for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant warm longlasting aroma as set forth in Table III of Example XVI. Furthermore, no such characteristic "hypochlorite" aroma is retained on the hands of the individual handling such laundry in both the wet and the dry states.

## Example XXVIII

Four drops of one of the substances set forth in Table III of Example XVI is added to 1 gram of n-undecyl dimethyl amine oxide to produce a clear premix. The clear premix is added to 200 grams of Clorox® with stirring resulting in a clear stable single phase solution. Sufficient 1M aqueous NaOH is

added to bring the pH of the mixture up to 12.8. The solution remains substantially stable at 120°F for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant warm aroma as set forth in Table III of Example XVI. Furthermore, no such characteristic "hypochlorite" aroma is retained on the hands of the individual handling such laundry in both the wet and the dry states.

Example XXIX

Four drops of one of the substances as set forth in Table III of Example XVI are added to 1 gram of n-dodecyl dimethyl amine oxide to produce a clear premix. The clear premix is added to 200 grams of Clorox® with stirring resulting in a clear, stable single phase solution. Sufficient 1M aqueous NaOH is added to bring the pH of the mixture up to 12.8. The solution remains substantially stable at 120°F for a period of 7 days. When the 5% aqueous sodium hypochlorite solution is used as a laundry bleach, the resulting laundry on dry-out in an atmosphere of 65% relative humidity yields substantially no characteristic "hypochlorite" aroma, but does have a warm, pleasant, long-lasting aroma as set forth in Table III of Example XVI. Furthermore, no such characteristic "hypochlorite" aroma is retained on the hands of the individual handling such laundry in both the wet and the dry states.

Example XXX

One gram of n-tridecyl dimethyl amine oxide is admixed with eight drops of one of the substances as set forth in Table II of Example XVI. This premix is then added with stirring to 200 grams of a 7% aqueous solution of lithium hydrochlorite. Sufficient 3M aqueous LiOH is added to bring the pH of the solution to 13.4. The mixture is then heated to 120°F and maintained at that temperature with stirring for a period of one week. The resulting solution remains clear in a single phase. When used as a laundry bleach, the resulting bleached laundry on dry-out in an atmosphere of 50% relative humidity retains a warm, fresh aroma described in Table III of Example XVI whereas without the use of one of the substances of Table III of Example XVI, the bleached laundry has a faint characteristic disagreeable "hypochlorite" aroma.

Example XXXI

A "soft-feel, good-hold" hair spray is produced containing the following ingredients:

| Ingredients | Parts by Weight |
|---|---|
| Polyvinylpyrrilidones/vinyl acetate E-735 copolymer manufactured by the GAF Corporation of New York, N.Y. | 4.0 |
| Anhydrous ethanol | 70.90 |
| Dioctyl sebecate | 0.05 |
| Benzyl alcohol | 0.05 |
| "Propellant A46" manufactured by the GAF Corporation of New York, N.Y. | 24.95 |
| Fragrance ingredient as set forth in Table III of Example XVI | 0.05 |

The PVP/VA copolymers are first dissolved in alcohol and all other ingredients are added until uniform. The propellant is then pressurized and used as an aerosol. The resulting hair-spray has a pleasant aroma as set forth in Table III of Example XVI.

Example XXXII

*Scouring cleanser composition*

A scouring cleanser composition is prepared in accordance with Example I, at columns 11 and 12 of U.S. Patent No. 4,193,888 issued on March 18, 1980. To this composition a substance as set forth in Table III of Example XVI is added at the level of 0.250% as set forth in the Table in said Example I of U.S. Patent No. 4,193,888 yielding an aroma on using said cleanser in ordinary circumstances which is quite pleasant and described in said Table III of Example XVI.

Example XXXIII

A fabric softening article prepared substantially as set forth in Example VIII of Canadian Patent No. 1,069,260 is prepared, containing 0.21 percent by weight of a perfuming substance as set forth in

Table III of Example XVI and yielding on use in a dryer, a faint aroma as set forth in Table III of Example XVI.

Brief description of the drawings

Figure 1 sets forth the GLC profile for the crude reaction product of Example I(A) containing the compounds having the structures:

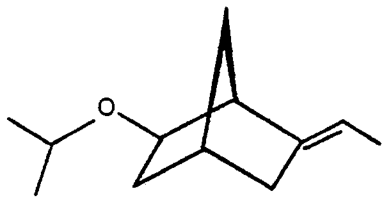

and

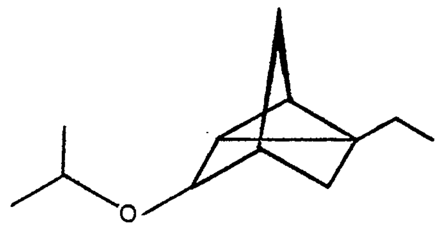

Figure 1(A) is the GLC profile of the purified reaction product of Example I(A) containing the compounds having the structures:

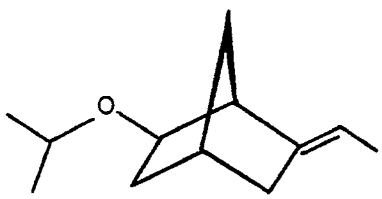

and

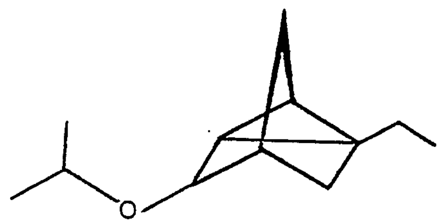

wherein peak 2 on said figure 1(A) is the compound having the structure:

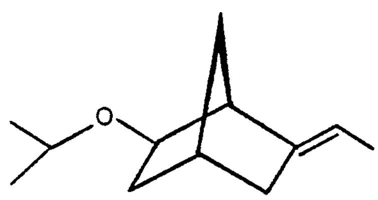

and peak 3 on said figure 1(A) consists of the compound having the structure:

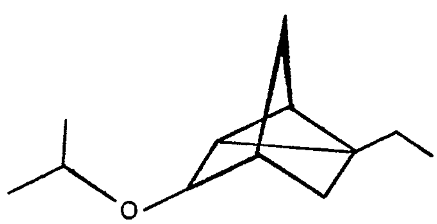

Figure 2 sets forth the NMR spectrum for the reaction product of Example 1(A) containing the compounds having the structures:

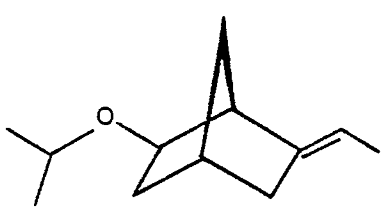

and

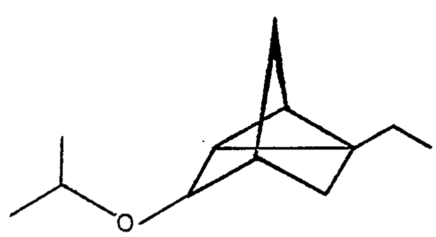

Figure 2(A) represents the NMR spectrum for peak 2 of the GLC profile of figure 1(A) and consists of the compound having the structure:

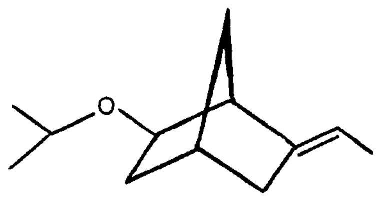

Figure 2(B) is the NMR spectrum for peak 3 of the GLC profile of figure 1(A) and consists essentially of the compound having the structure:

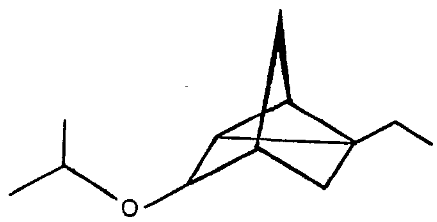

Figure 3 sets forth the infra-red spectrum for the reaction product of Example 1(A) containing the compounds having the structures:

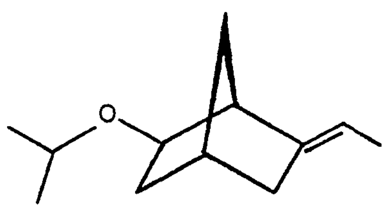

and

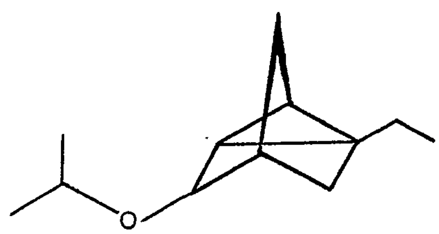

Figure 3(A) represents the infra-red spectrum for peak 2 of the GLC profile of figure 1(A) which peak consists essentially of the compound having the structure:

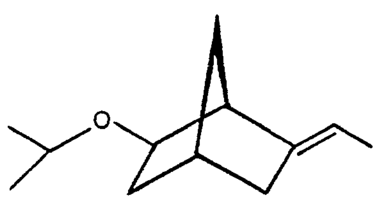

Figure 3(B) represents the infra-red spectrum for peak 3 of the GLC profile of figure 1(A) and consists essentially of the compound having the structure:

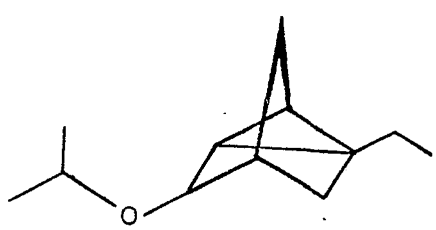

Figure 3(C) represents the GLC profile for the reaction product of Example I(B) containing the compounds having the structures:

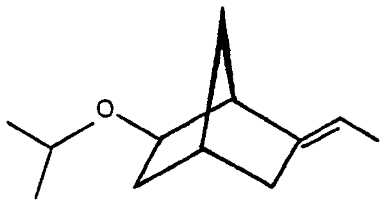 and 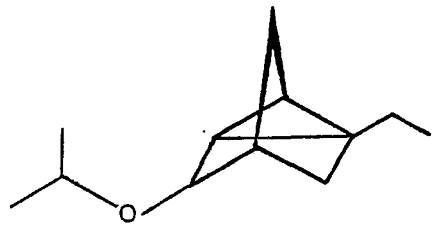

Figure 3(D) represents the NMR spectrum for the reaction product of Example I(B) containing the compounds having the structures:

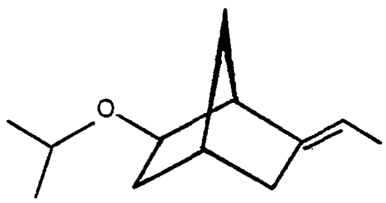 and 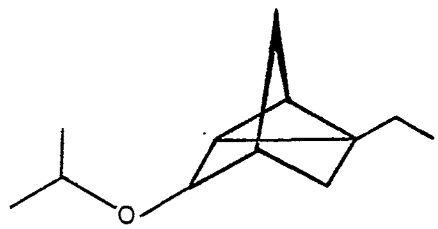

Figure 4 sets forth the GLC profile for the reaction product of Example II containing the compounds having the structures:

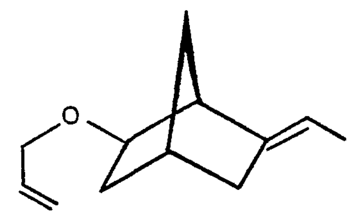

and

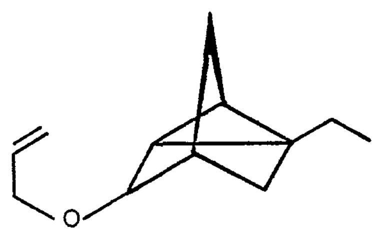

Figure 5 sets forth the NMR spectrum for the reaction product of Example II containing the compounds having the structures:

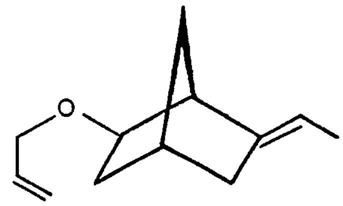

and

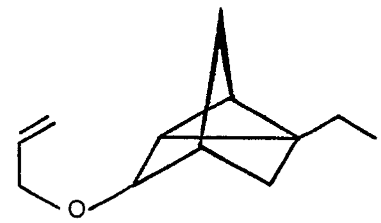

Figure 6 is the infra-red spectrum for the reaction product of Example II containing the compounds having the structures:

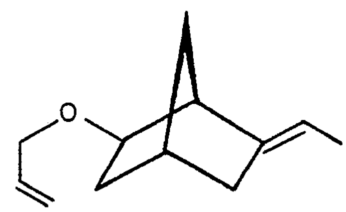

and

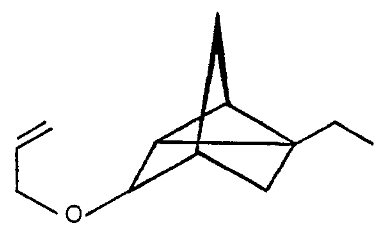

Figure 7 sets forth the GLC profile for the reaction product of Example III containing the compounds having the structures:

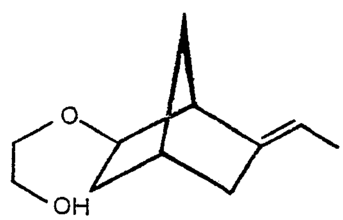

and

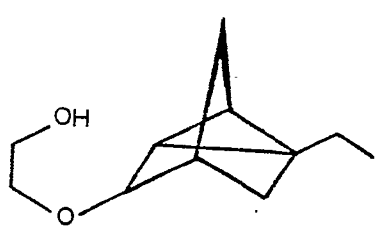

Figure 8 represents the NMR spectrum for the reaction product of Example III containing the compounds having the structures:

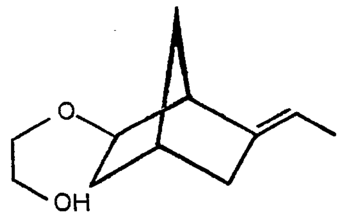

and

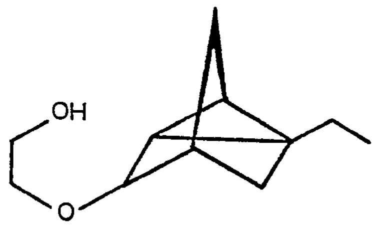

Figure 9 sets forth the infra-red spectrum for the reaction product of Example III containing the compounds having the structures:

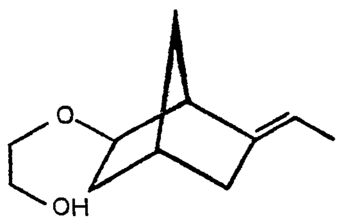

and

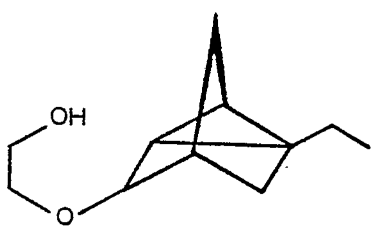

Figure 10 represents the GLC profile for the reaction product of Example IV containing the compounds having the structures:

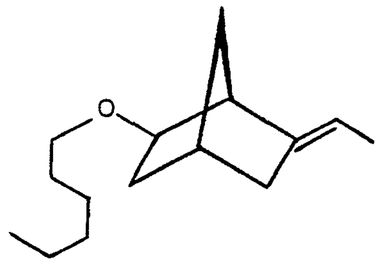

and

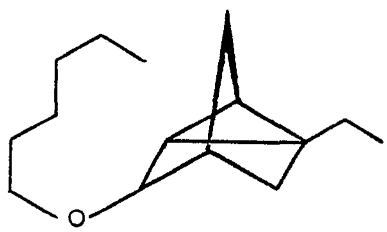

Figure 11 sets forth the NMR spectrum for the reaction product of Example IV containing the compounds having the structures:

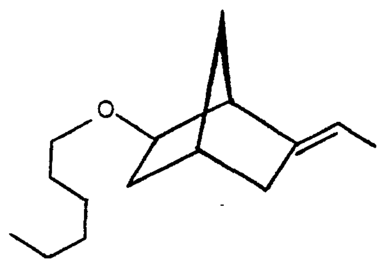

and

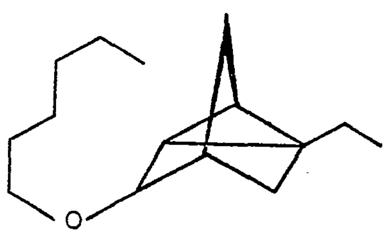

Figure 12 represents the infra-red spectrum for the reaction product of Example IV containing the compounds having the structures:

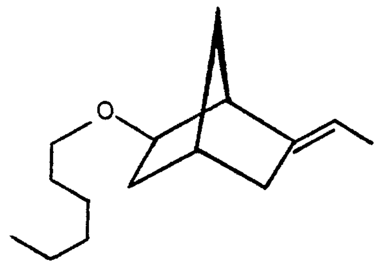

and

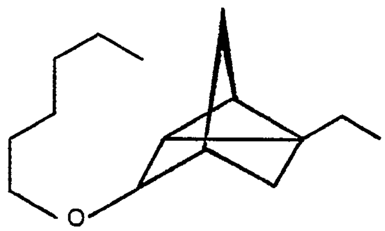

Figure 13 is the NMR spectrum for the reaction product of Example V containing the compounds having the structures:

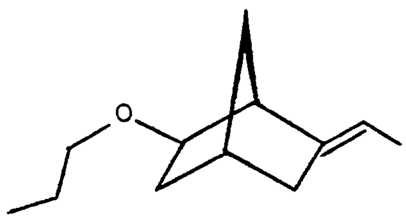

and

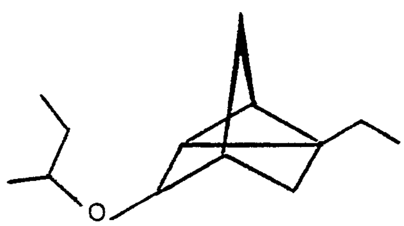

Figure 14 sets forth the infra-red spectrum for the reaction product of Example V containing the compounds having the structures:

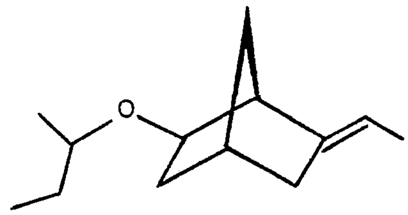

and

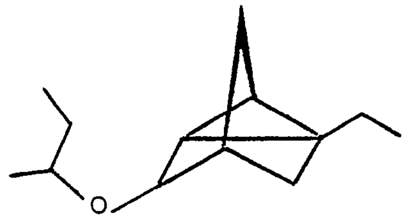

Figure 15 represents the GLC profile for the reaction product of Example VI containing the compounds having the structures:

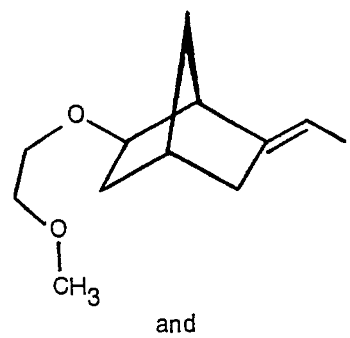

and

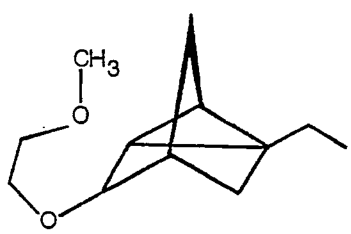

Figure 16 sets forth the NMR spectrum for the reaction product of Example VI containing the compounds having the structures:

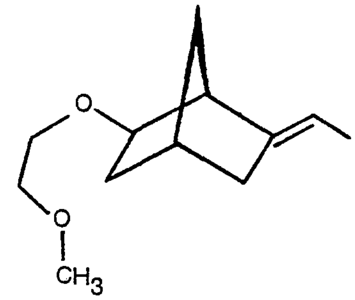

and

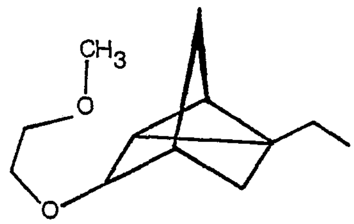

Figure 17 represents the infra-red-spectrum for the reaction product of Example VI containing the compounds having the structures:

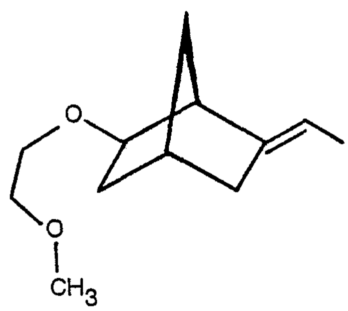

and

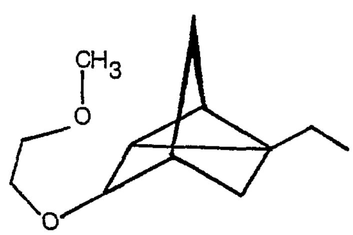

Figure 18 sets forth the GLC profile for the reaction product of Example VII containing the compounds having the structures:

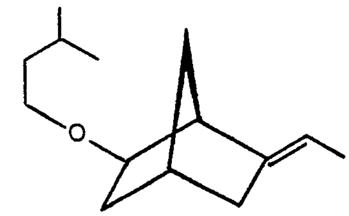

and

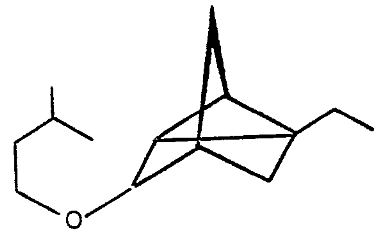

Figure 19 sets forth the NMR spectrum for the reaction product of Example VII containing the compounds having the structures:

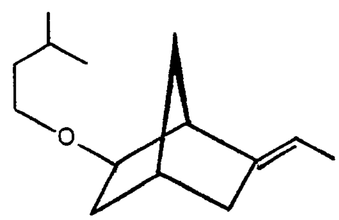

and

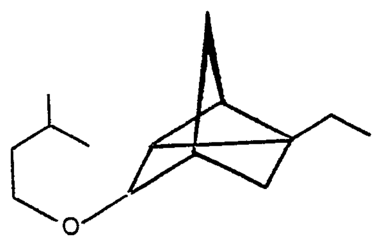

Figure 20 sets forth the infra-red spectrum for the reaction product of Example VII containing the compounds having the structures:

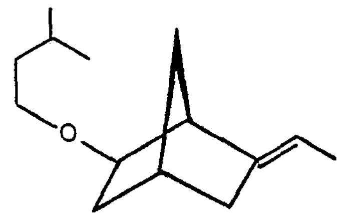

and

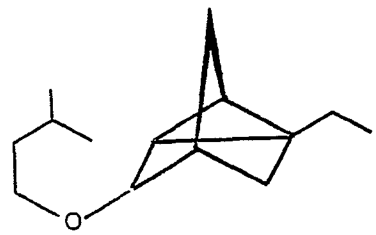

Figure 21 sets forth the GLC profile for the reaction product of Example VIII containing the compounds having the structures:

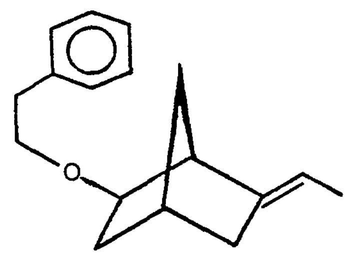

and

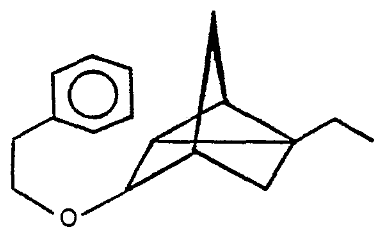

Figure 22 sets forth the NMR spectrum for the reaction product of Example VIII containing the compounds having the structures:

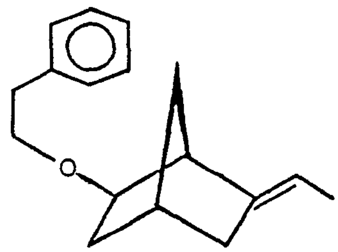

and

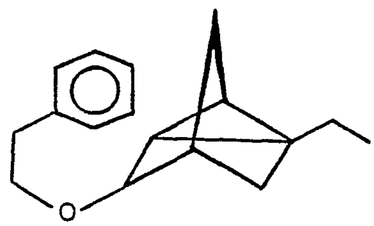

Figure 23 sets forth the infra-red spectrum for the reaction product of Example VIII containing the compounds having the structures:

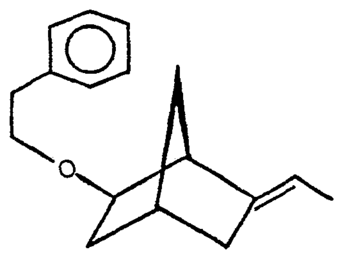

and

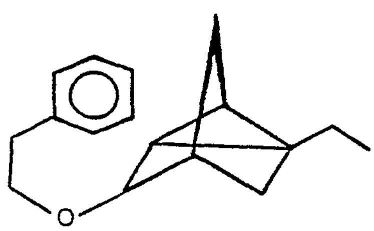

Figure 24 sets forth the GLC profile for the reaction product of Example IX containing the compounds having the structures:

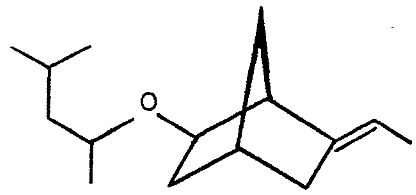

and

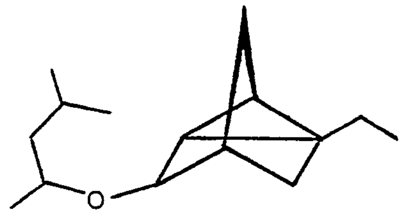

Figure 25 sets forth the NMR spectrum for the reaction product of Example IX containing the compounds having the structures:

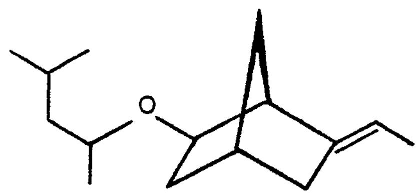

and

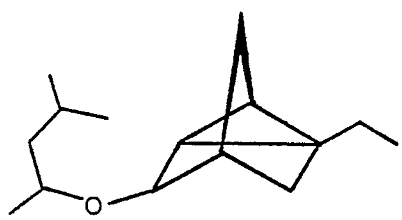

Figure 26 sets forth the infra-red spectrum for the reaction product of Example IX containing the compounds having the structures:

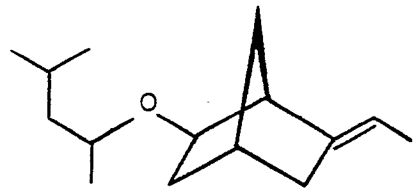

and

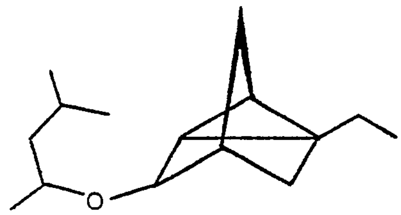

Figure 27 sets forth the GLC profile for the reaction product of Example X containing the compounds having the structures:

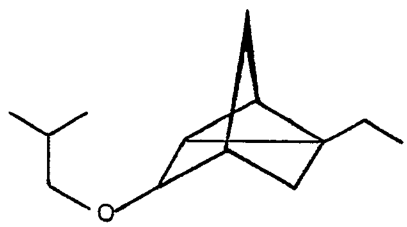

and

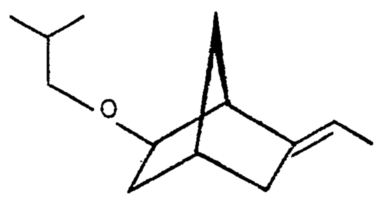

Figure 28 sets forth the NMR spectrum for the reaction product of Example X containing the compounds having the structures:

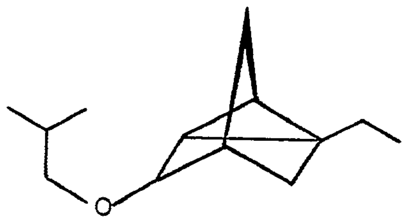

and

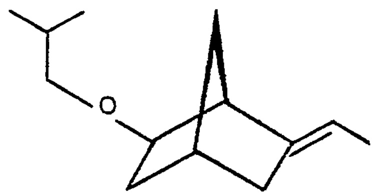

Figure 29 sets forth the infra-red spectrum for the reaction product of Example X containing the compounds having the structures:

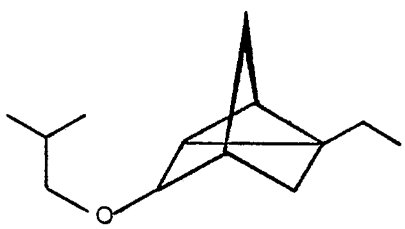

and

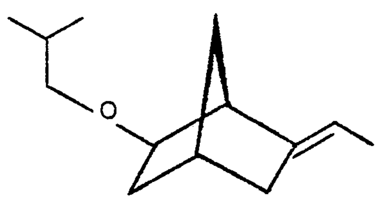

Figure 30 sets forth the GLC profile for the reaction product of Example XI containing the compounds having the structures:

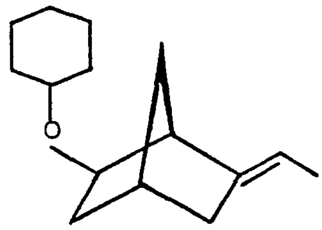

and

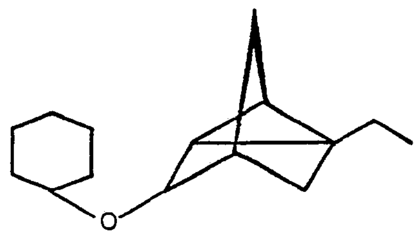

Figure 31 sets forth the NMR spectrum for the reaction product of Example XI containing the compounds having the structures:

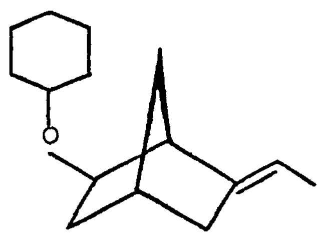

and

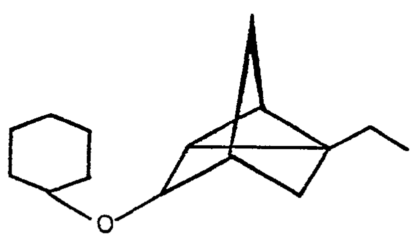

Figure 32 sets forth the infra-red spectrum for the reaction product of Example XI containing the compounds having the structures:

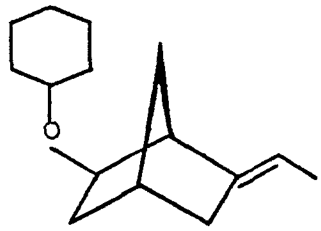

and

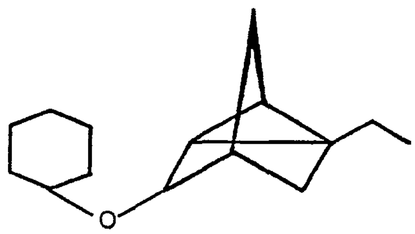

Figure 33 is the GLC profile for the reaction mass just prior to distillation of Example XII containing the compounds having the structures:

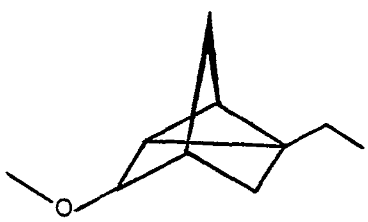

and

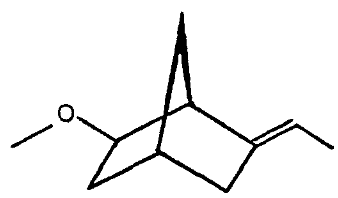

Figure 34 is the NMR spectrum for peak 1 of the GLC profile of figure 33 of the reaction product of Example XII containing the compounds defined according to the structure:

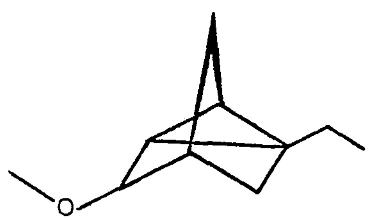

Figure 35 is the NMR spectrum for peak 2 of the GLC profile of figure 33 (of the reaction product of Example XII) and is for the structure:

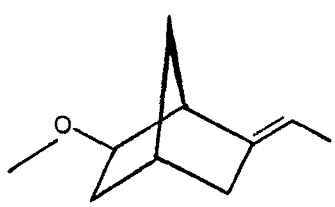

Figure 36 is the GLC profile for the reaction mass of Example XIII after 2 hours and 40 minutes of reaction; the reaction mass containing compounds having the structures:

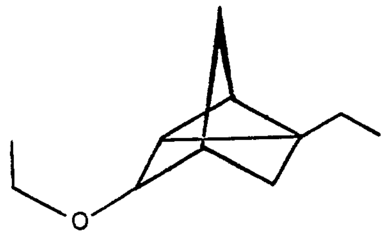

and

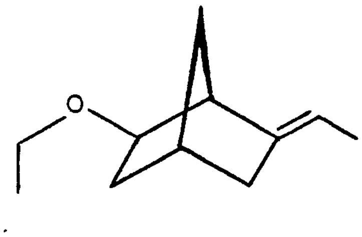

Figure 37 is the NMR spectrum for peak 1 of the GLC profile of figure 36 for the reaction mass of Example XIII and is for the compound having the structure.

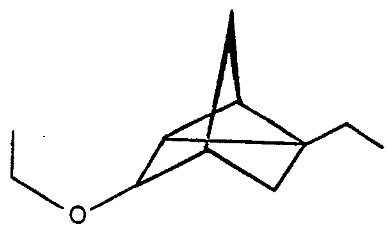

Figure 38 is the NMR spectrum for peak 2 of the GLC profile of figure 36 for the reaction product of Example XIII and is for the compound having the structure:

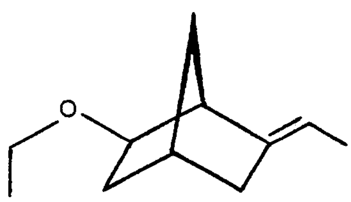

Figure 39 is the GLC profile for the reaction product subsequent to quenching but prior to distillation product for Example XIV containing the compounds having the structures:

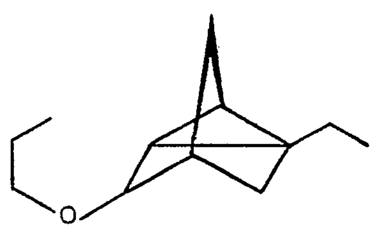

and

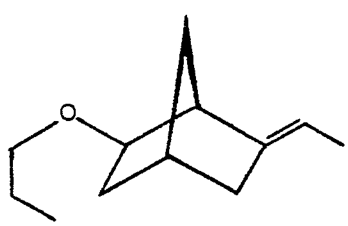

Figure 40 is the NMR spectrum for peak 1 of the GLC profile of figure 39 for the reaction product of Example XIV containing the compound having the structure:

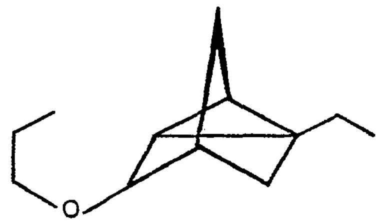

Figure 41 is the NMR spectrum for peak 2 of the GLC profile of figure 39 for the reaction product of Example XIV and is for the compound having the structure:

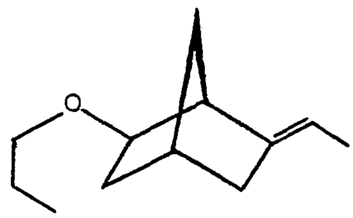

Detailed description of the drawings

Figure 1(A) is the GLC profile for the reaction product produced according to Example I(A) and contains starting material having the structure:

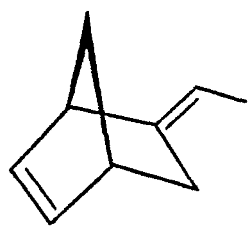

as well as reaction products:

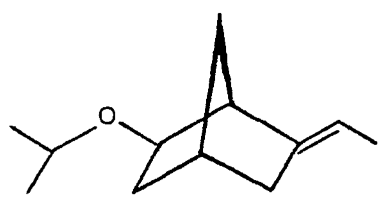

and

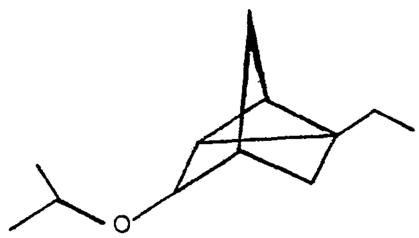

Reference "1" indicates the peak of this GLC profile which consists of the compound having the structure:

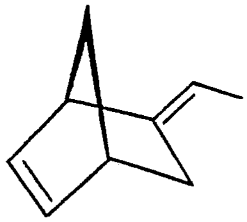

Reference "2" indicates the peak of the GLC profile which consists essentially of the compound having the structure:

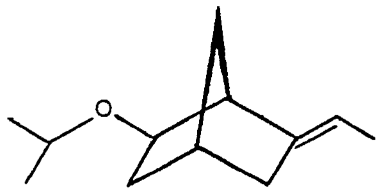

Reference "3" indicates the peak of the GLC profile which consists essentially of the compound having the structure:

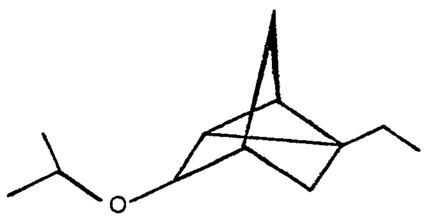

**Claims**

1. A composition of matter having organoleptic property-modifying capability, which comprises at least one compound having a structure selected from the group consisting of:

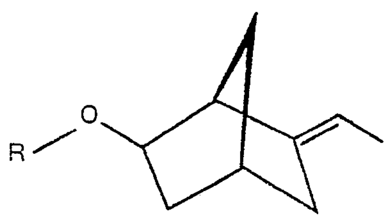

and

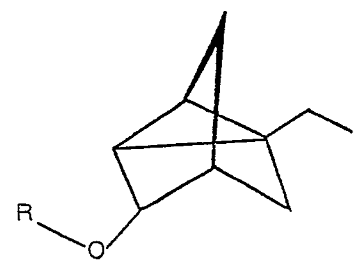

wherein R represents $C_3$—$C_6$ alkyl; $C_3$—$C_6$ alkenyl; $C_6$—$C_{10}$ aralkyl; $C_6$—$C_{10}$ alkaryl; hydroxy-$C_2$—$C_6$-alkyl; $C_1$—$C_4$-alkoxy-$C_2$—$C_6$ alkyl; and $C_5$—$C_8$ cycloalkyl; and wherein the R moieties in each structure are the same, with the proviso that in the compound comprising the double bond R cannot be an $C_6$ alkyl.

2. The composition of Claim 1 wherein the compound has the structure:

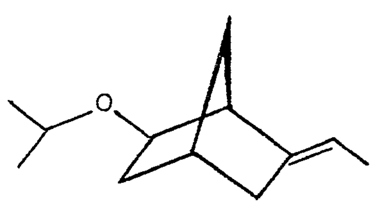

3. The composition of Claim 1 wherein the compound has the structure:

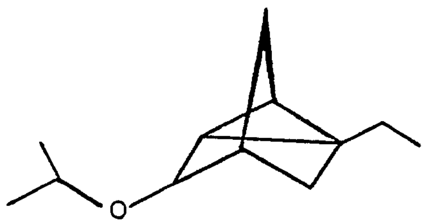

4. The composition of Claim 1 wherein the compound has the structure:

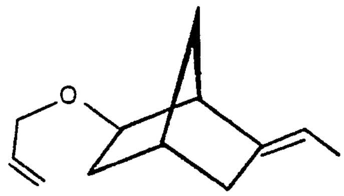

5. The composition of Claim 1 wherein the compound has the structure:

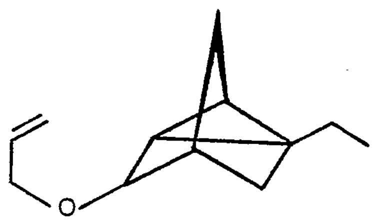

6. The compositon of Claim 1 wherein the compound has the structure:

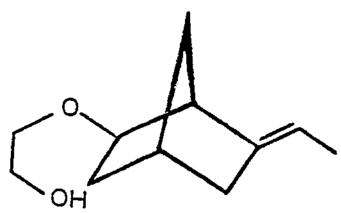

7. The composition of Claim 1 wherein the compound has the structure:

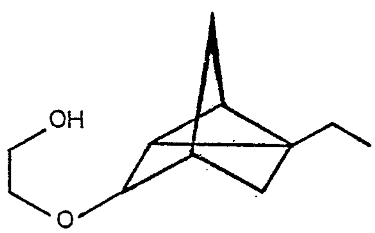

8. The composition of Claim 1 wherein the compound has the structure:

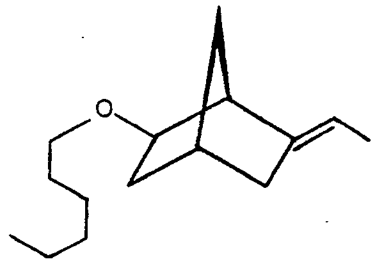

9. The composition of Claim 1 wherein the compound has the structure:

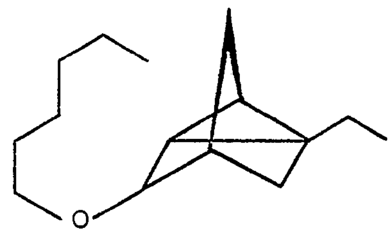

10. The composition of Claim 1 wherein the compound has the structure:

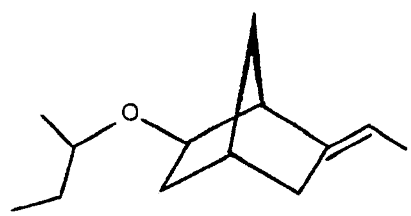

11. The composition of Claim 1 wherein the compound has the structure:

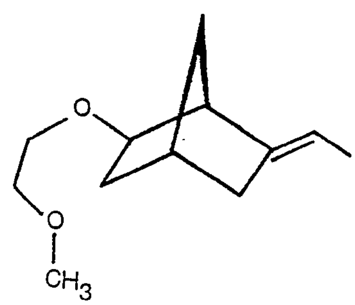

12. The composition of Claim 1 wherein the compound has the structure:

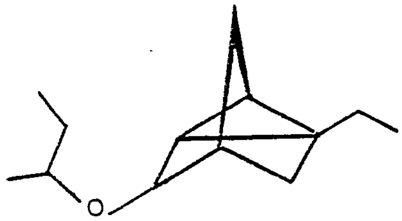

13. The composition of Claim 1 wherein the compound has the structure:

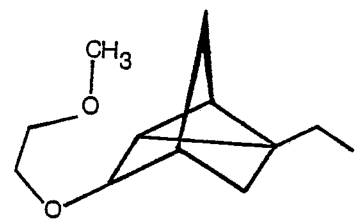

14. The composition of Claim 1 wherein the compound has the structure:

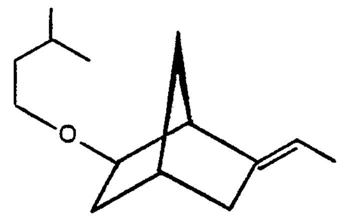

15. The composition of Claim 1 wherein the compound has the structure:

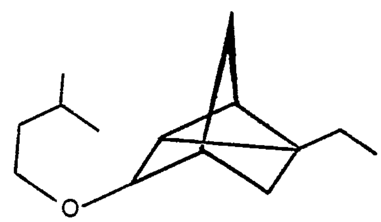

16. The composition of Claim 1 wherein the compound has the structure:

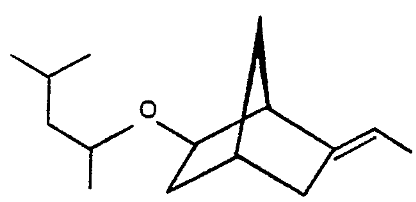

17. The composition of Claim 1 wherein the compound has the structure:

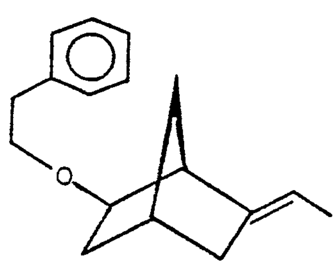

18. The composition of Claim 1 wherein the compound has the structure:

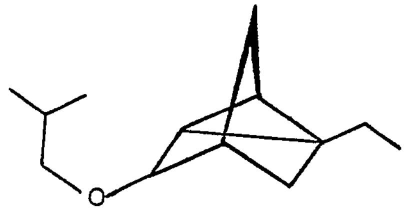

19. The composition of Claim 1 wherein the compound has the structure:

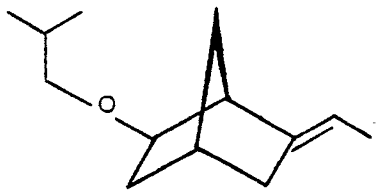

20. The composition of Claim 1 wherein the compound has the structure:

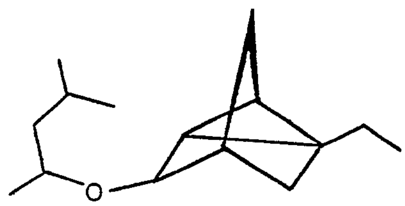

21. The composition of Claim 1 wherein the compound has the structure:

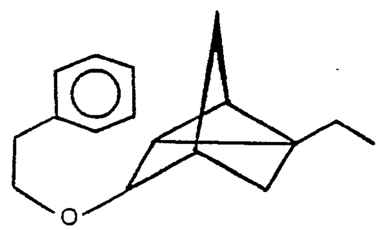

22. The composition of Claim 1 wherein the compound has the structure:

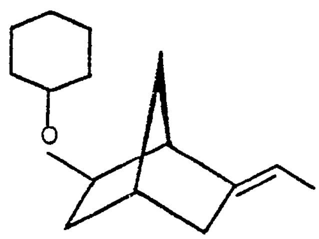

23. The composition of Claim 1 wherein the compound has the structure:

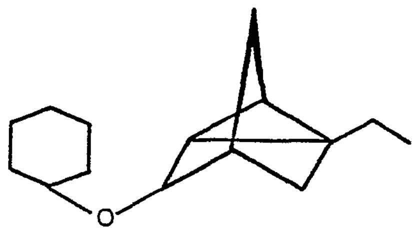

24. The composition of Claim 1 wherein the compound has the structure:

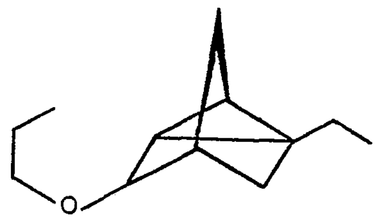

25. The composition of Claim 1 wherein the compound has the structure:

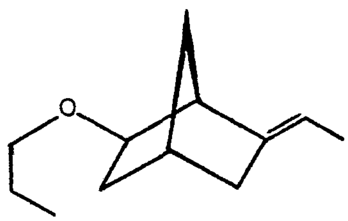

26. A process for the production of compound(s) in accordance with Claim 1 comprising the step of intimately admixing ethylidene norbornene defined according to the structure:

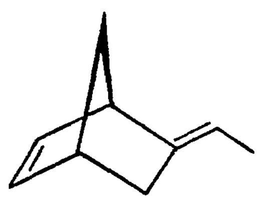

with an alcohol defined according to the structure:

R—OH

characterised in that the moiety R is selected from the group consisting of $C_3$—$C_6$ alkyl; $C_3$—$C_6$ alkenyl; $C_6$—$C_{10}$ aralkyl; $C_6$—$C_{10}$ alkaryl; hydroxy-$C_2$—$C_6$-alkyl; $C_1$—$C_4$-alkoxy-$C_2$—$C_6$ alkyl; and $C_5$—$C_8$ cycloalkyl, and that the reaction is carried out in the presence of a mineral acid catalyst or a Lewis acid catalyst and in the presence of an inert solvent or in the presence of an excess of the alcohol reactant, the reaction temperature varying from 25°C up to 120°C, the mole ratio of catalyst to ethylidene norbornene having the structure:

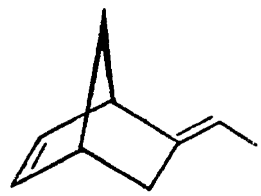

varying from 1:99 up to 1:10; the mole ratio of ethylidene norbornene reaction to ROH alcohol reactant varying from 1:1 up to 1:2.

27. The process comprising the step of reacting ethylidene norbornene having the structure:

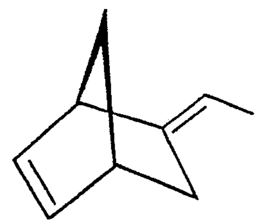

with ROH wherein R represents $C_3$—$C_6$ alkyl; $C_3$—$C_6$ alkenyl; $C_6$—$C_{10}$ aralkyl; $C_6$—$C_{10}$ alkaryl; hydroxy-$C_2$—$C_6$-alkyl; $C_1$—$C_4$-alkoxy-$C_2$—$C_6$ alkyl and $C_5$—$C_8$ cycloalkyl in the presence of a catalyst selected from the group consisting of mineral acids and Lewis acids at a reaction temperature of from 25°C up to 120°C, the mole ratio of acid catalyst: ethylidene norbornene varying from 1:99 up to 1:10; the mole ratio of ethylidene norbornene reactant to ROH reactant varying from 1:1 up to 1:2 according to the reaction scheme:

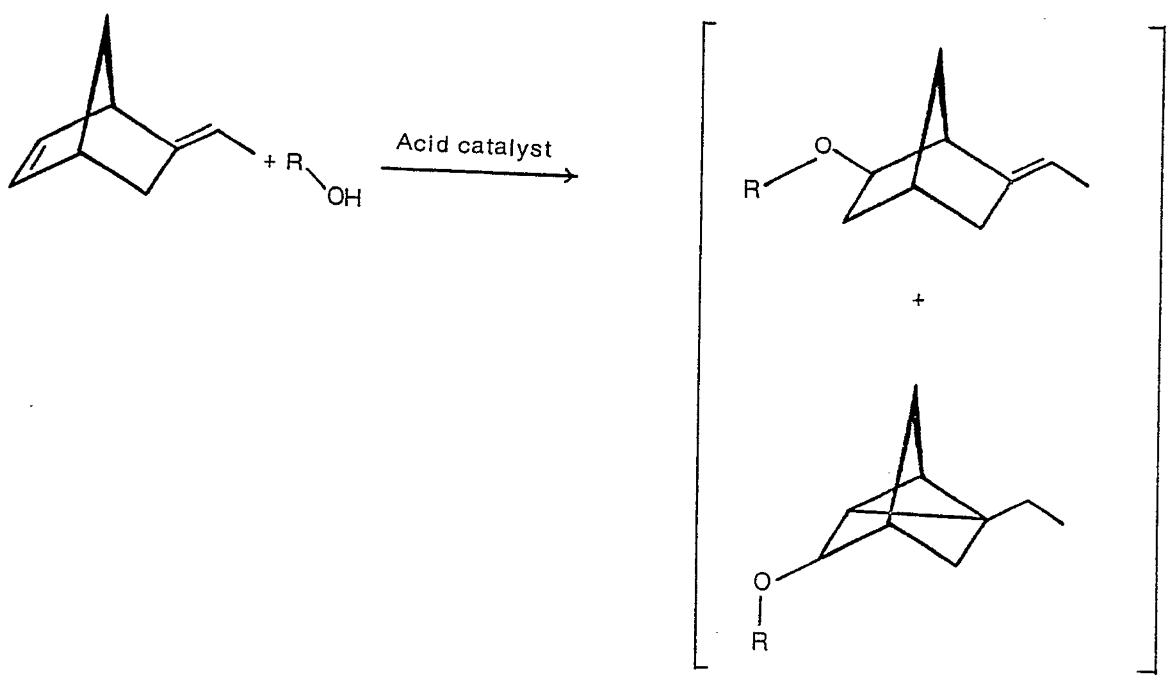


and then recovering the compounds having the structures:

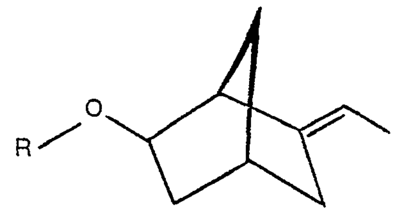

and

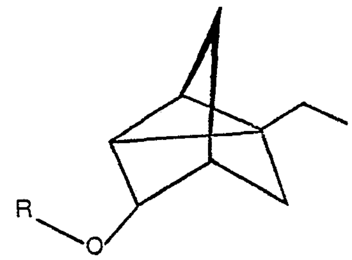

wherein R represents $C_3$—$C_6$ alkyl; $C_3$—$C_6$ alkenyl; $C_6$—$C_{10}$ aralkyl; $C_6$—$C_{10}$ alkaryl; hydroxy-$C_2$—$C_6$-alkyl; $C_1$—$C_4$-alkoxy-$C_2$—$C_6$ alkyl; and $C_5$—$C_8$ cycloalkyl; and wherein the R moieties in each structure are the same.

28. The process of Claim 27 wherein the catalyst used is selected from the group consisting of boron trifluoride etherate and sulfuric acid.

29. The process of Claim 27 wherein R is isopropyl.

30. The process of Claim 28 wherein R is isopropyl.

31. A product produced according to the process of Claim 26.

32. The product defined according to Claim 31 wherein R is isopropyl.

33. The product defined according to Claim 31 wherein R is cyclohexyl.

34. The product defined according to Claim 31 wherein R represents allyl.

35. The product defined according to Claim 31 wherein R represents phenethyl.

36. A process for augmenting or enhancing the aroma of a consumable material selected from the group consisting of perfume compositions, colognes and perfumed articles comprising the step of adding to a perfume base, a cologne base or a perfumed article base on organoleptic property-modifying quantity of at least one compound having a structure defined according to Claim 1.

37. The process of Claim 36 wherein the consumable material is a perfume base.

38. The process of Claim 36 wherein the consumable material is a cologne.

39. The process of Claim 36 wherein the consumable material is a perfumed article.

40. The process of Claim 36 wherein the consumable material is a perfumed article and the perfumed article is a detergent which is a solid or liquid anionic, cationic, nonionic or zwitterionic detergent.

41. The process of Claim 36 wherein the consumable material is a perfumed article and the perfumed article is a fabric softener composition or dryer-added fabric softener article.

42. A dryer-added fabric softener article consisting of a cloth substrate, a substrate coating and an outer coating, said outer coating being a composition of matter comprising an outer coating base composition and intimately admixed therewith from 0.01% up to 1% of the outer coating composition of a composition of matter consisting essentially of at least one compound having a structure selected from the group consisting of:

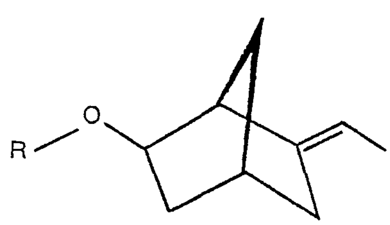

and

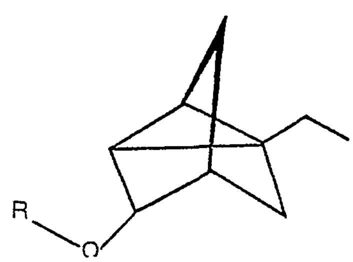

wherein R represents $C_3$—$C_6$ alkyl; $C_3$—$C_6$ alkenyl; $C_6$—$C_{10}$ aralkyl; $C_6$—$C_{10}$ alkaryl; hydroxy-$C_2$—$C_6$-alkyl; $C_1$—$C_4$-alkoxy-$C_2$—$C_6$ alkyl and $C_5$—$C_8$ cycloalkyl; and wherein the R moieties in each structure are the same.

43. A process for augmenting or enhancing the aroma of fabric subjected to the softening action of a dryer-added fabric softener article comprising the steps of admixing a coating base composition with from 0.01% up to 1% of a composition of matter consisting essentially of at least one compound having a structure selected from the group consisting of:

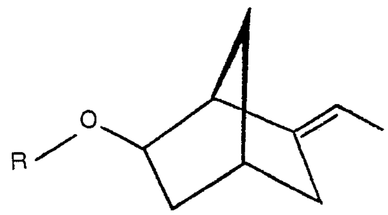 and 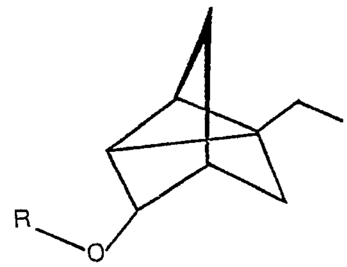

wherein R represents $C_3$—$C_6$ alkyl; $C_3$—$C_6$ alkenyl; $C_6$—$C_{10}$ aralkyl; $C_6$—$C_{10}$ alkaryl; hydroxy-$C_2$—$C_6$-alkyl; $C_1$—$C_4$-alkoxy-$C_2$—$C_6$ alkyl; and $C_5$—$C_8$ cycloalkyl; and wherein the R moieties in each structure are the same.

44. The process of Claim 43 wherein R is isopropyl.

45. The dryer-added fabric softener article of Claim 43 wherein R is isopropyl.

**Revendications**

1. Composition apte à modifier les propriétés organoleptiques comprenant au moins un composé de structure choisie dans le groupe constitué par:

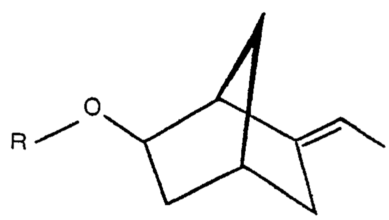 et 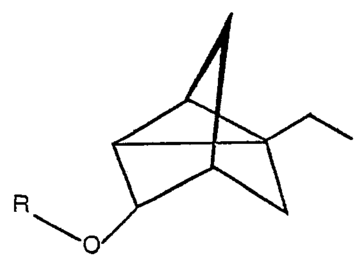

où R représente un alcoyle en $C_3$ à $C_6$; un alcényle en $C_3$ à $C_6$; un aralcoyle en $C_6$ à $C_{10}$; un alcaryle en $C_6$ à $C_{10}$; un hydroxy-alcoyle en $C_2$ à $C_6$; un alcoxy en $C_1$ à $C_4$-alcoyle en $C_2$ à $C_6$; et un cycloalcoyle en $C_5$ à $C_8$; et où les fractions R dans toutes les structures sont semblables, avec la précision que dans le composé comprenant la double liaison R ne peut être un alcoyle en $C_6$.

2. Composition de la revendication 1 où le composé est de structure:

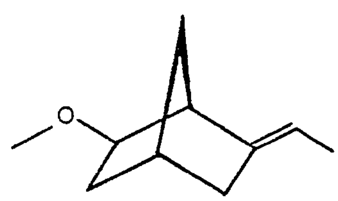

3. Composition de la revendication 1 où le composé est de structure:

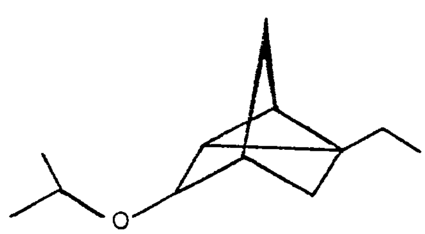

4. Composition de la revendication 1 où le composé est de structure:

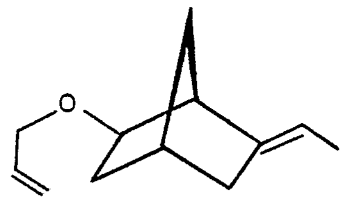

5. Composition de la revendication 1 où le composé est de structure:

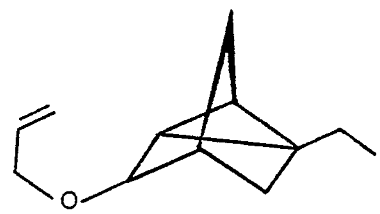

6. Composition de la revendication 1 où le composé est de structure:

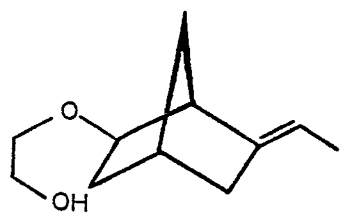

7. Composition de la revendication 1 où le composé est de structure:

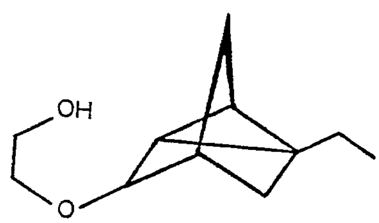

8. Composition de la revendication 1 où le composé est de structure:

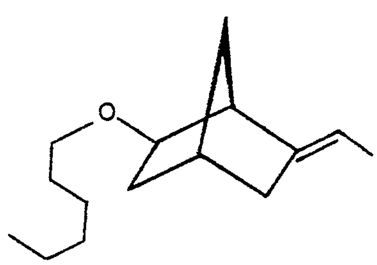

9. Composition de la revendication 1 où le composé est de structure:

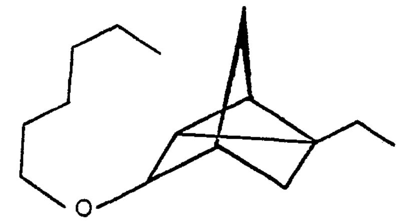

10. Composition de la revendication 1 où le composé est de structure:

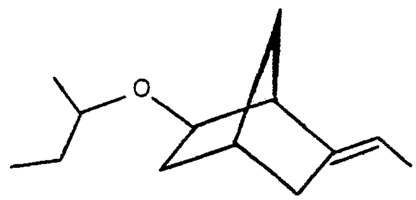

11. Composition de la revendication 1 où le composé est de structure:

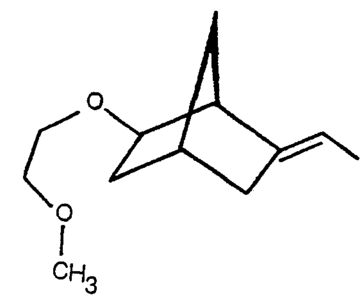

12. Composition de la revendication 1 où le composé est de structure:

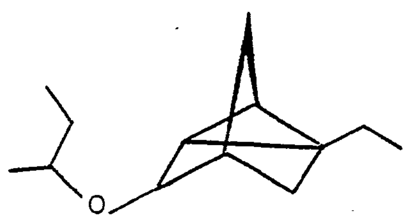

13. Composition de la revendication 1 où le composé est de structure:

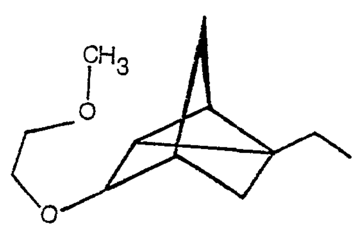

14. Composition de la revendication 1 ou le composé est de structure:

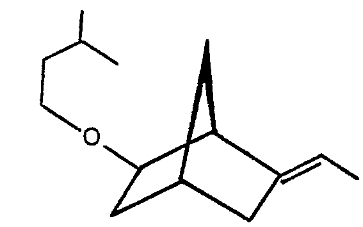

15. Composition de la revendication 1 où le composé est de structure:

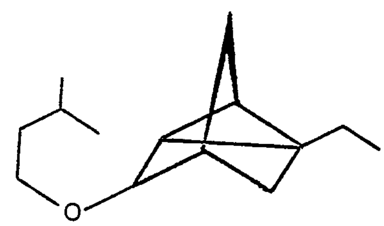

16. Composition de la revendication 1 où le composé est de structure:

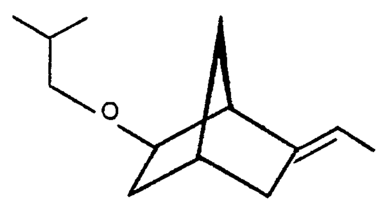

17. Composition de la revendication 1 où le composé est de structure:

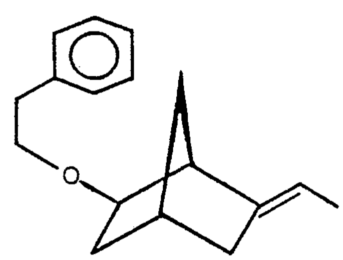

18. Composition de la revendication 1 où le composé est de structure:

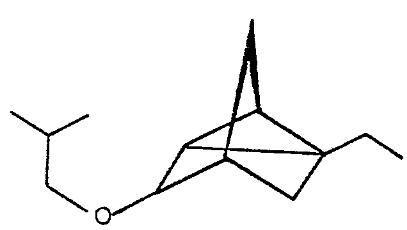

19. Composition de la revendication 1 où le composé est de structure:

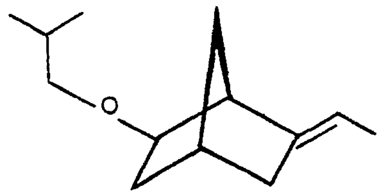

20. Composition de la revendication 1 où le composé est de structure:

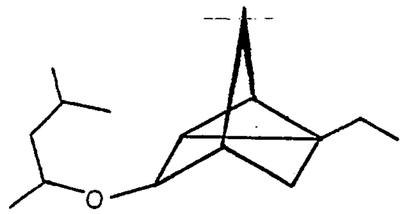

21. Composition de la revendication 1 où le composé est de structure:

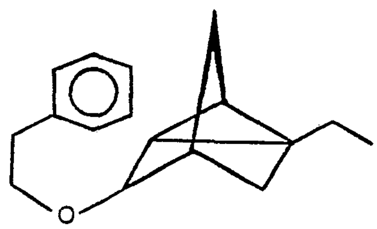

22. Composition de la revendication 1 où le composé est de structure:

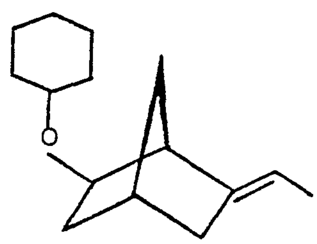

23. Composition de la revendication 1 où le composé est de structure:

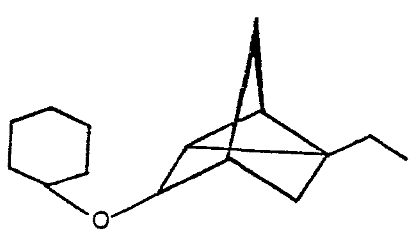

24. Composition de la revendication 1 où le composé est de structure:

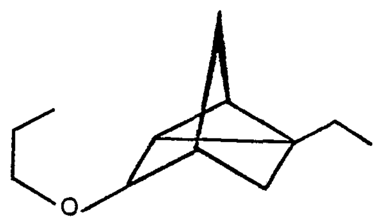

25. Composition de la revendication 1 où le composé est de structure:

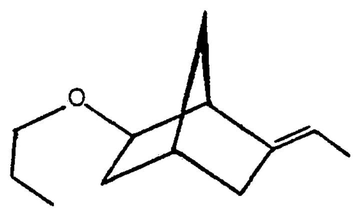

26. Procédé de production de composé(s) selon la revendication 1 comprenant l'étape consistant à mélanger intimement de l'éthylidène-norbornène défini selon la structure:

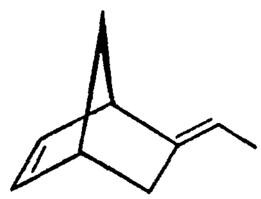

avec un alcool défini selon la structure:

R—OH

caractérisé en ce qu'on choisit la fraction R dans le groupe constitué par alcoyle en $C_3$ à $C_6$; alcényle en $C_3$ à $C_6$; aralcoyle en $C_6$ à $C_{10}$; alcaryle en $C_6$ à $C_{10}$; hydroxy-alcoyle en $C_2$ à $C_6$; alcoxy en $C_1$ à $C_4$-alcoyle en $C_2$ à $C_6$; et cyclo-alcoyle en $C_5$ à $C_8$, et en ce que la réaction est conduite en présence d'un catalyseur acide minéral ou d'un catalyseur acide de Lewis et en présence d'un solvant inerte ou en présence d'un excès du réactif alcool, la température de la réaction variant de 25°C à 120°C, le rapport molaire du catalyseur à l'éthylidène-norbornène de structure:

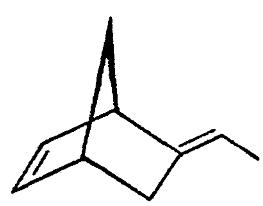

variant de 1:99 à 1:10; le rapport molaire de la réaction d'éthylidène-norbornène au réactif alcool ROH variant de 1:1 à 1:2.

27. Procédé comprenant l'étape de réaction de l'éthylidène-norbornène de structure:

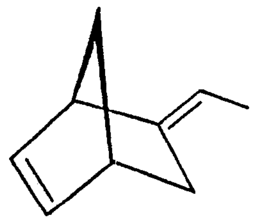

avec ROH où R représente un alcoyle en $C_3$ à $C_6$; un alcényle en $C_3$ à $C_6$; un aralcoyle en $C_2$ à $C_{10}$; un alcaryle en $C_6$ à $C_{10}$; un hydroxy-alcoyle en $C_2$ à $C_6$; un alcoxy en $C_1$ à $C_4$-alcoyle en $C_2$ à $C_6$ et un cycloalcoyle en $C_5$ à $C_8$ en présence d'un catalyseur choisi dans le groupe constitué par les acides minéraux et les acides de Lewis à une température de réaction allant de 25°C à 120°C, le rapport molaire catalyseur acide: éthylidène-norbornène variant de 1:99 à 1:10; le rapport molaire du réactif éthylidène-norbornène au réactif ROH variant de 1:1 à 1:2 selon le schéma réactionnel:

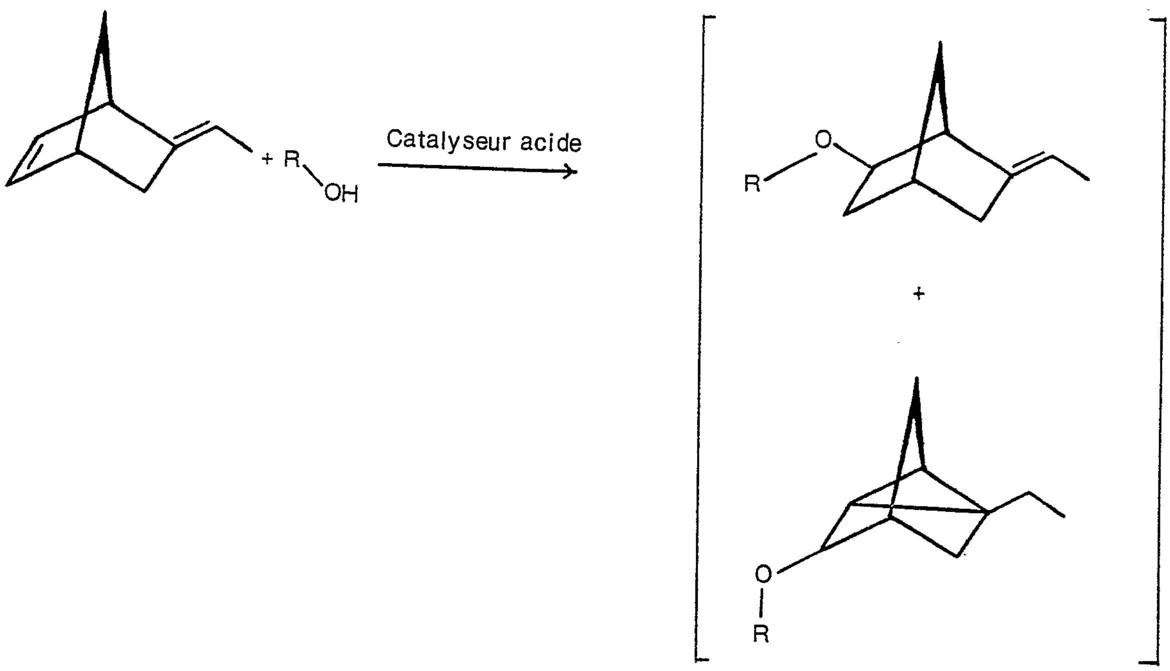


puis l'étape de récupération des composés de structure:

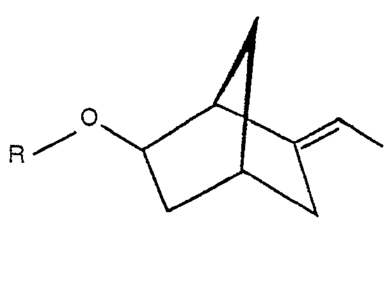

et

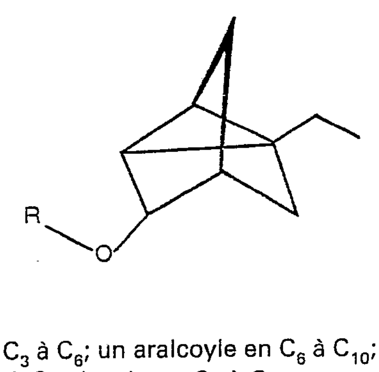

où R représente un alcoyle en $C_3$ à $C_6$; un alcényle en $C_3$ à $C_6$; un aralcoyle en $C_6$ à $C_{10}$; un alcaryle en $C_6$ à $C_{10}$; un hydroxy-alcoyle en $C_2$ à $C_6$; un alcoxy en $C_1$ à $C_4$-alcoyle en $C_2$ à $C_6$; et un cycloalcoyle en $C_5$ à $C_8$; et où les fractions R dans toutes les structures sont semblables.

28. Procédé de la revendication 27 où le catalyseur utilisé est choisi dans le groupe constitué par l'éthérate de trifluorure de bore et l'acide sulfurique.

29. Procédé de la revendication 27 où R est un isopropyle.

30. Procédé de la revendication 28 où R est un isopropyle.

31. Produit produit selon le procédé de la revendication 26.

32. Produit défini selon la revendication 31 où R est un isopropyle.

33. Produit défini selon la revendication 31 où R est un cyclohexyle.

34. Produit défini selon la revendication 31 où R représente un allyle.

35. Produit défini selon la revendication 31 où R représente un phénéthyle.

36. Procédé pour accroître ou améliorer l'arôme d'un produit de consommation choisi dans le groupe constitué par les compositions de parfum, les eaux de Cologne et les articles parfumés, comprenant l'étape consistant à ajouter à une base de parfum, à une base d'eau de Cologne ou à une base d'article parfumé une quantité, modifiant les propriétés organoleptiques d'au moins un composé de structure définie selon la revendication 1.

37. Procédé de la revendication 36 où le produit de consommation est une base de parfum.

38. Procédé de la revendication 36 où le produit de consommation est une eau de Cologne.

39. Procédé de la revendication 36 où le produit de consommation est un article parfumé.

40. Procédé de la revendication 36 où le produit de consommation est un article parfumé et l'article parfumé est un détergent qui est un détergent solide ou liquide anionique, cationique, non-ionique ou hermaphrodite.

41. Procédé de la revendication 36 où le produit de consommation est un article parfumé et l'article parfumé est une composition adoucissant les tissus ou un article adoucissant les tissus additionné de dessicateur.

42. Article adoucissant les tissus additionné de dessicateur constitué d'un substrat textile, d'un revêtement de substrat et d'un revêtement extérieur, ledit revêtement extérieur étant une composition comprenant une composition de base de revêtement extérieur et, en mélange intime avec elle, de 0,01% à 1% de la composition de revêtement extérieur d'une composition constituée essentiellement d'au moins un composé de structure choisie dans le groupe constitué par:

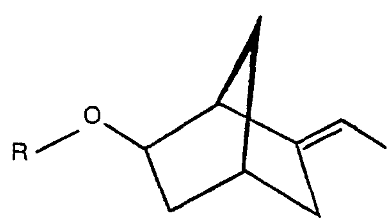

et

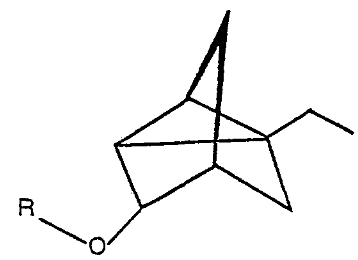

où R représente un alcoyle en $C_3$ à $C_6$; un alcényle en $C_3$ à $C_6$; un aralcoyle en $C_6$ à $C_{10}$; un alcaryle en $C_6$ à $C_{10}$; un hydroxy-alcoyle en $C_2$ à $C_6$; un alcoxy en $C_1$ à $C_4$-alcoyle en $C_2$ à $C_6$ et un cycloalcoyle en $C_5$ à $C_8$; et où les fractions R dans toutes les structures sont semblables.

43. Procédé pour accroître ou améliorer l'arôme d'un tissu soumis à l'action adoucissante d'un article adoucissant pour tissu additionné de dessicateur comprenant les étapes consistant à melanger une composition de base de revêtement avec de 0,01% à 1% d'une composition constituée essentiellement d'au moins un composé de structure choisie dans le groupe constitué par:

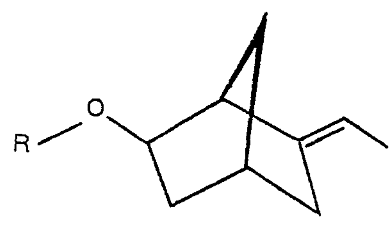

et

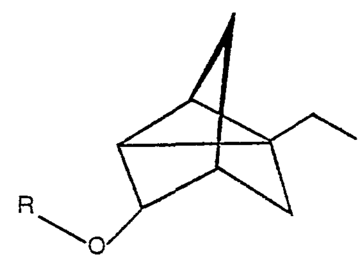

où R représente un alcoyle en $C_3$ à $C_6$; un alcényle en $C_3$ à $C_6$; un aralcoyle en $C_6$ à $C_{10}$; un alcaryle en $C_6$ à $C_{10}$; un hydroxy-alcoyle en $C_2$ à $C_6$; un alcoxy en $C_1$ à $C_4$-alcoyle en $C_2$ à $C_6$; et un cycloalcoyle en $C_5$ à $C_8$; et où les fractions R dans toutes les structures sont semblables.

44. Procédé de la revendication 43 où R est un isopropyle.

45. Article adoucissant pour tissu additionné de dessicateur de la revendication 43 où R est un isopropyle.

**Patentansprüche**

1. Materialzusammensetzung, die zur Veränderung organoleptischer Eigenschaften befähigt ist, die mindestens eine Verbindung aufweist, die eine Struktur ausgewählt aus der Gruppe bestehend aus:

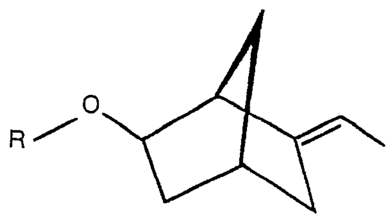

und

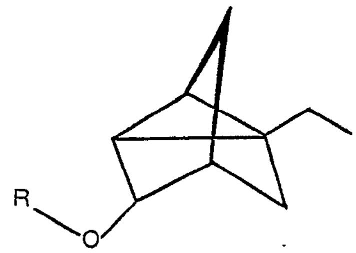

besitzt, wobei R $C_3$—$C_6$-Alkyl; $C_3$—$C_6$-Alkenyl; $C_6$—$C_{10}$-Aralkyl; $C_6$—$C_{10}$-Alkaryl; Hydroxy-$C_2$—$C_6$-Alkyl; $C_1$—$C_4$-Alkoxy-$C_2$—$C_6$-Alkyl; und $C_5$—$C_8$-Cycloalkyl bedeutet; und wobei die R-Reste in jeder Strukturformel die gleichen sind, wobei in der Verbindung, die die Doppelbindung aufweist, R kein $C_6$-Alkyl sein kann.

2. Zusammensetzung nach Anspruch 1, wobei die Verbindung folgende Struktur besitzt:

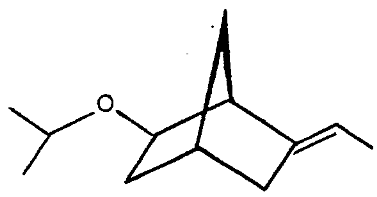

3. Zusammensetzung nach Anspruch 1, wobei die Verbindung folgende Struktur besitzt:

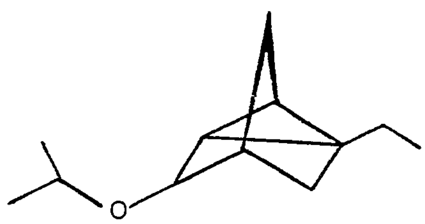

4. Zusammensetzung nach Anspruch 1, wobei die Verbindung folgende Struktur besitzt:

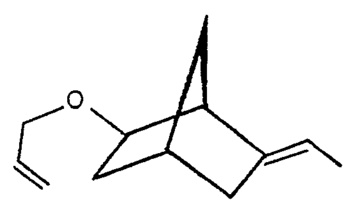

5. Die Zusammensetzung nach Anspruch 1, wobei die Verbindung folgende Struktur besitzt:

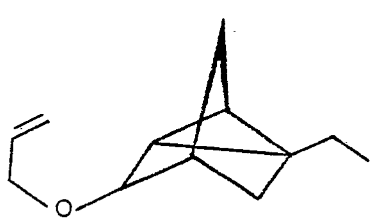

6. Die Verbindung nach Anspruch 1, wobei die Verbindung folgende Struktur besitzt:

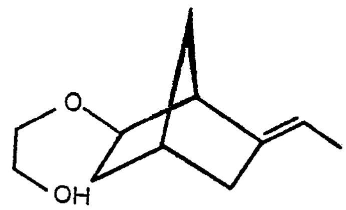

7. Die Verbindung nach Anspruch 1, wobei die Verbindung folgende Struktur besitzt:

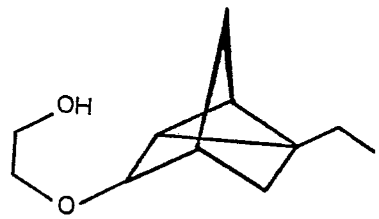

8. Die Zusammensetzung nach Anspruch 1, wobei die Verbindung folgende Struktur besitzt:

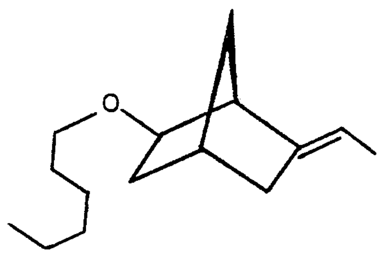

9. Die Zusammensetzung nach Anspruch 1, wobei die Verbindung folgende Struktur besitzt:

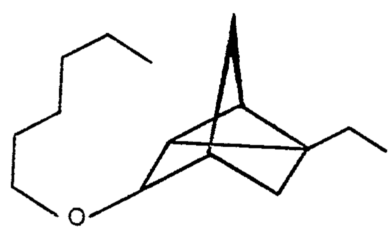

10. Die Zusammensetzung nach Anspruch 1, wobei die Verbindung folgende Struktur besitzt:

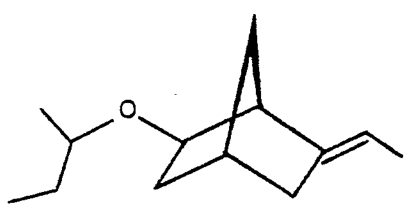

11. Die Zusammensetzung nach Anspruch 1, wobie die Verbindung folgende Struktur besitzt:

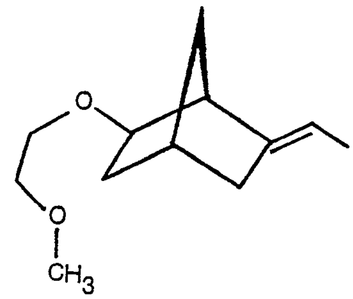

12. Die Zusammensetzung nach Anspruch 1, wobei die Verbindung folgende Struktur besitzt:

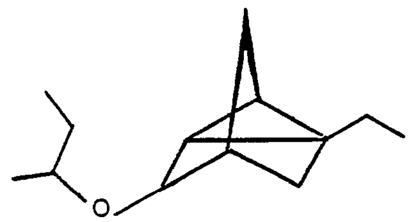

13. Die Zusammensetzung nach Anspruch 1, wobei die Verbindung folgende Struktur besitzt:

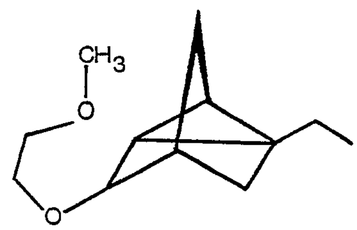

14. Die Zusammensetzung nach Anspruch 1, wobei die Verbindung folgende Struktur besitzt:

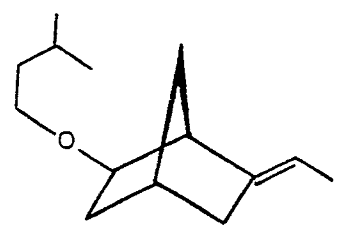

15. Die Zusammensetzung nach Anspruch 1, wobei die Verbindung folgende Struktur besitzt:

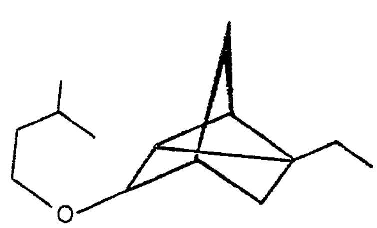

16. Die Zusammensetzung nach Anspruch 1, wobei die Verbindung folgende Struktur besitzt:

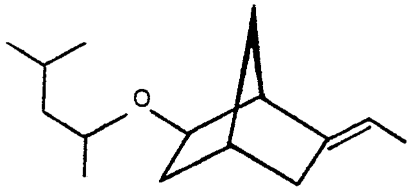

17. Die Zusammensetzung nach Anspruch 1, wobei die Verbindung folgende Struktur besitzt:

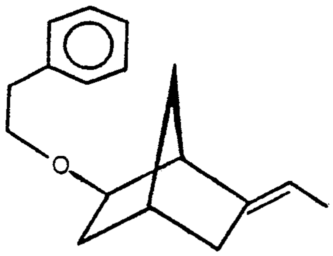

18. Die Zusammensetzung nach Anspruch 1, wobei die Verbindung folgende Struktur besitzt:

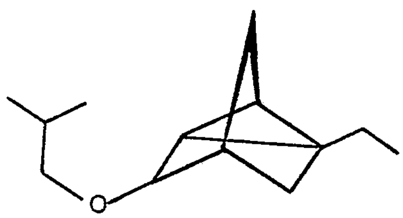

19. Die Zusammensetzung nach Anspruch 1, wobei die Verbindung folgende Struktur besitzt:

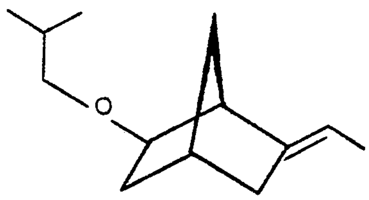

20. Die Zusammensetzung nach Anspruch 1, wobei die Verbindung folgende Struktur besitzt:

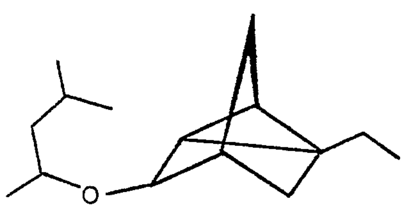

21. Die Zusammensetzung nach Anspruch 1, wobei die Verbindung folgende Struktur besitzt:

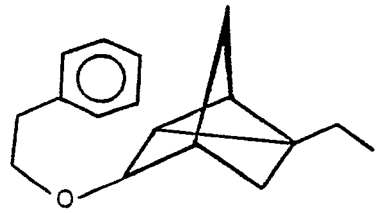

22. Die Zusammensetzung nach Anspruch 1, wobei die Verbindung folgende Struktur besitzt:

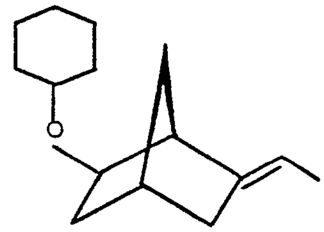

23. Die Zusammensetzung nach Anspruch 1, wobei die Verbindung folgende Struktur besitzt:

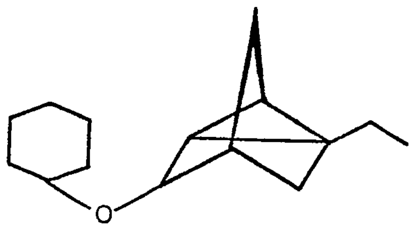

24. Die Zusammensetzung nach Anspruch 1, wobei die Verbindung folgende Struktur besitzt:

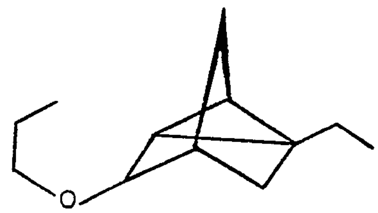

25. Die Zusammensetzung nach Anspruch 1, wobei die Verbindung folgende Struktur besitzt:

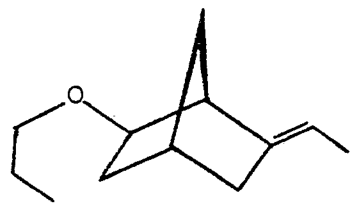

26. Verfahren zur Herstellung von (einer) Verbindung(en) gemäß Anspruch 1, welches den Verfahrensschritt des innigen Vermischens von Ethylidennorbornen, definiert durch folgende Strukturformel:

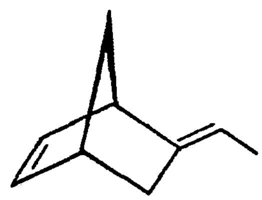

mit einen Alkohol, definiert durch folgende Strukturformel:

R—OH

aufweist, dadurch gekennzeichnet, daß der Rest R ausgewählt ist aus der Gruppe bestehend aus $C_3$—$C_6$-Alkyl; $C_3$—$C_6$-Alkenyl; $C_6$—$C_{10}$-Aralkyl; $C_6$—$C_{10}$-Alkaryl; Hydroxy-$C_2$—$C_6$-Alkyl; $C_1$—$C_4$-Alkoxy-$C_2$—$C_6$-Alkyl; und $C_5$—$C_8$-Cycloalkyl, und daß die Reaktion in Gegenwart eines Mineralsäurekatalysators oder eines Lewissäurekatalysators und in Gegenwart eines inerten Lösungsmittels oder in Gegenwart eines Überschusses des Reaktanden-Alkohols durchgeführt wird, wobei die Reaktionstemperatur zwischen 25°C bis zu 120°C liegt, und das Mol-Verhältnis von Katalysator zu Ethylidennorbornen der Struktur:

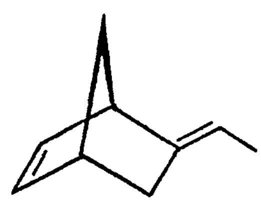

sich von 1:99 bis zu 1:10 ändert; und das Mol-Verhältnis des Ethylidennorbornenreaktanden zum ROH-Alkoholreaktanden zwischen 1:1 bis zu 1:2 variiert.

27. Verfahren, welches den Schritt des Umsetzens von Ethylidennorbornen folgender Struktur:

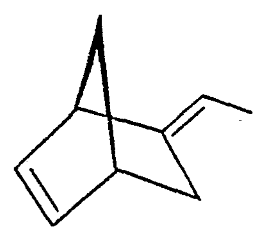

mit ROH aufweist, wobei R $C_3$—$C_6$-Alkyl; $C_3$—$C_6$-Alkenyl; $C_6$—$C_{10}$-Aralkyl; $C_6$—$C_{10}$-Alkaryl; Hydroxy-$C_2$—$C_6$-Alkyl; $C_1$—$C_4$-Alkoxy-$C_2$—$C_6$-Alkyl und $C_5$—$C_8$-Cycloalkyl ist, in Gegenwart eines Katalysators ausgewählt aus der Gruppe bestehend aus Mineralsäuren und Lewissäuren bei einer Reaktionstemperatur von 25°C bis zu 120°C, wobei das Mol-Verhältnis von saurem Katalysator zu Ethylidennorbornen zwischen 1:99 bis zu 1:10 variiert; das Mol-Verhältnis des Ethylidennorbornen-Reaktanden zum ROH-Reaktanden zwischen 1:1 bis zu 1:2 variiert, entsprechend dem Reaktionsschema:

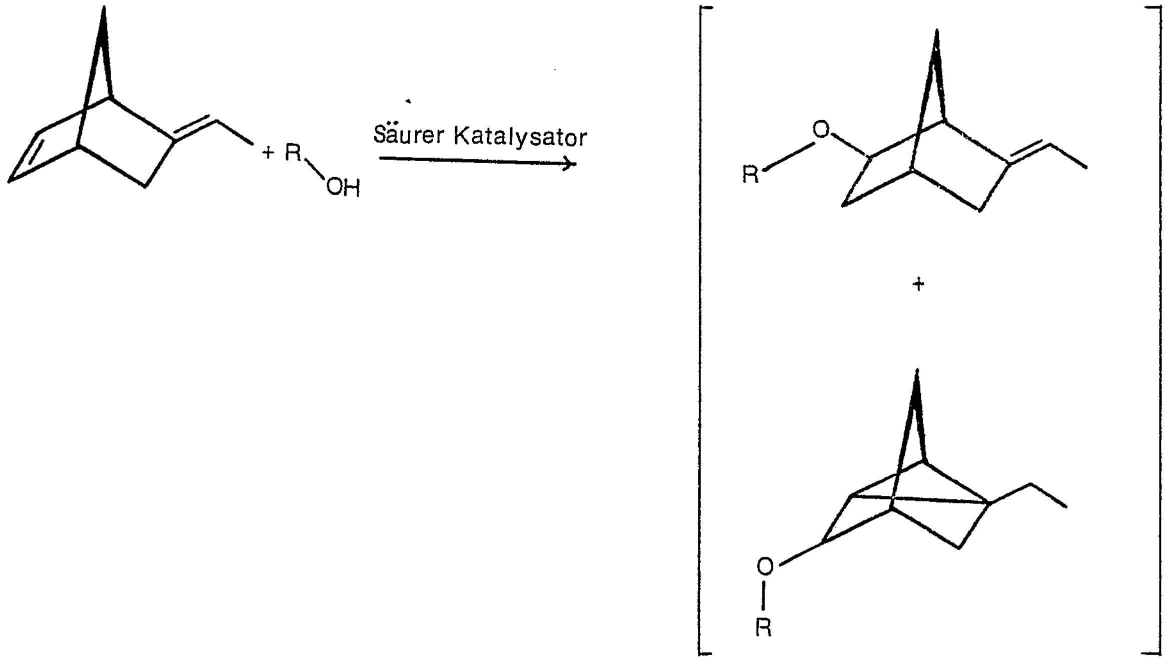


und sodann die Verbindungen folgender Strukturen gewonnen werden:

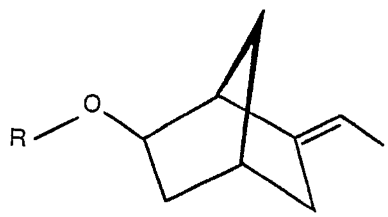

und

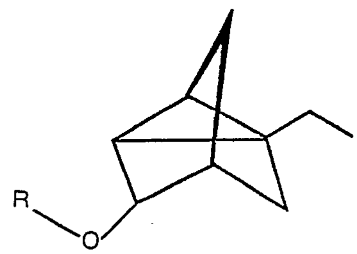

wobei R $C_3$—$C_6$-Alkyl; $C_3$—$C_6$-Alkenyl; $C_6$—$C_{10}$-Aralkyl; $C_6$—$C_{10}$-Alkaryl; Hydroxy-$C_2$—$C_6$-Alkyl; $C_1$—$C_4$-Alkoxy-$C_2$—$C_6$-Alkyl; und $C_5$—$C_8$-Cycloalkyl repräsentiert, und wobei die R-Reste in jeder Struktur die gleichen sind.

28. Verfahren nach Anspruch 27, wobei der eingesetzte Katalysator ausgewählt ist aus der Gruppe bestehend aus Bortrifluorid-Etherat und Schwefelsäure.

29. Verfahren nach Anspruch 27, wobei R Isopropyl ist.

30. Verfahren nach Anspruch 28, wobei R Isopropyl ist.

31. Ein gemäß dem Verfahren des Anspruches 26 hergestelltes Produkt.

32. Produkt gemäß Anspruch 31, wobei R Isopropyl ist.

33. Produkt gemäß Anspruch 31, wobei R Cyclohexyl ist.

34. Produkt gemäß Anspruch 31, wobei R Allyl repräsentiert.

35. Produkt gemäß Anspruch 31, wobei R Phenethyl repräsentiert.

36. Verfahren zum Verbessern oder Verstärken des Aromas eines Verbrauchs-Materials, ausgewählt aus der Gruppe bestehend aus Parfumzusammensetzungen, Kölnisch Wassern und parfümierten Artikeln, welches den Schritt der Zugabe zu einer Parfum-Basis, einer Kölnisch Wasser-Basis oder einer Basis eines parfümierten Artikels in die organoleptischen Eigenschaften ändernder Menge mindestens einer Verbindung, die eine Struktur gemäß Anspruch 1 besitzt, aufweist.

37. Verfahren nach Anspruch 36, wobei das Verbrauchs-Material eine Parfum-Basis ist.

38. Verfahren nach Anspruch 36, wobei das Verbrauchs-Material eine Kölnisch Wasser-Basis ist.

39. Verfahren nach Anspruch 36, wobei das Verbrauchs-Material eine parfümierter Artikel ist.

40. Verfahren nach Anspruch 36, wobei das Verbrauchs-Material eine parfümierter Artikel ist und der parfümierte Artikel ein Reinigungsmittel ist, das ein festes oder flüssiges anionisches, kationisches, nicht-ionisches oder zwitter-ionisches Detergens ist.

41. Verfahren nach Anspruch 36, wobei das verzehrbare Material ein parfümierter Artikel ist und der parfümierte Artikel eine Stoff-Weichmacher-Zusammensetzung oder ein in einen Trockner gegebener Textilweichmacher ist.

42. Ein in einen Trockner zu gebender Textilweichmacher, bestehend aus einem Tuchsubstrat, einem Substratüberzug und einem äußeren Überzug, wobei der äußere Überzug eine Materialzusammensetzung ist, die eine äußere Überzugsbasiszusammensetzung und innig damit vermischt — zwischen 0,01% bis zu 1% der äußeren Überzugszusammensetzung aufweist, einer Materialzusammensetzung, die im wesentlichen aus mindestens einer Verbindung einer Struktur, ausgewählt aus der Gruppe bestehend aus:

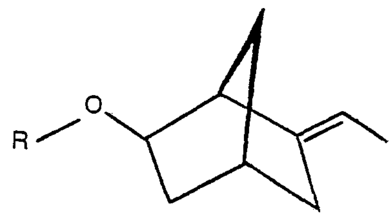

und

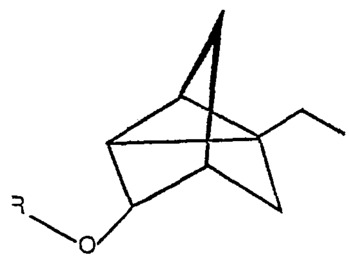

aufweist,
wobei R $C_3$—$C_6$-Alkyl; $C_3$—$C_6$-Alkenyl; $C_6$—$C_{10}$-Aralkyl; $C_6$—$C_{10}$-Alkaryl; Hydroxy-$C_2$—$C_6$-Alkyl; $C_1$—$C_4$-Alkoxy-$C_2$—$C_6$-Alkyl und $C_5$—$C_8$-Cycloalkyl repräsentiert; und wobei die R-Reste in jeder Struktur die gleichen sind.

43. Verfahren zum Verbessern oder Verstärken des Aromas von Textilien, die der Weichmacherwirkung eines in einen Trockner gegebenen Textilweichmacherartikels unterworfen werden, welches die Schritte des Vermischens einer Überzugsbasiszusammensetzung mit zwischen 0,01% bis zu 1% einer Materialzusammensetzung aufweist, die im wesentlichen aus mindestens einer Verbindung einer Struktur ausgewählt aus der Gruppe bestehend aus

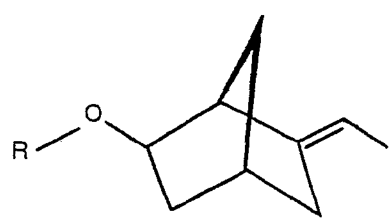

und

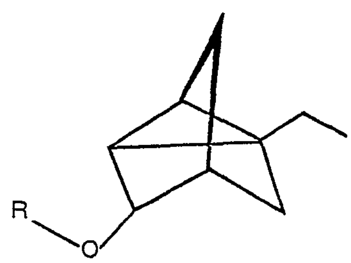

aufweist, wobei R $C_3$—$C_6$-Alkyl; $C_3$—$C_6$-Alkenyl; $C_6$—$C_{10}$-Aralkyl; $C_6$—$C_{10}$-Alkaryl; Hydroxy-$C_2$—$C_6$-Alkyl; $C_1$—$C_4$-Alkoxy-$C_2$—$C_6$-Alkyl; und $C_5$—$C_8$-Cycloalkyl präsentiert, und wobei die R-Reste in jeder Struktur die gleichen sind.

44. Verfahren nach Anspruch 43, wobei R Isopropyl ist.

45. Textilweichmacherartikel zum Trocknerzusatz gemäß Anspruch 43, wobei R Isopropyl ist.

FIG.1

GLC PROFILE FOR EXAMPLE I.

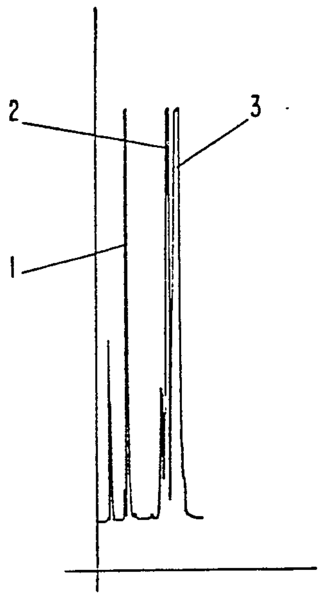


FIG.1A

GLC PROFILE FOR EXAMPLE I.

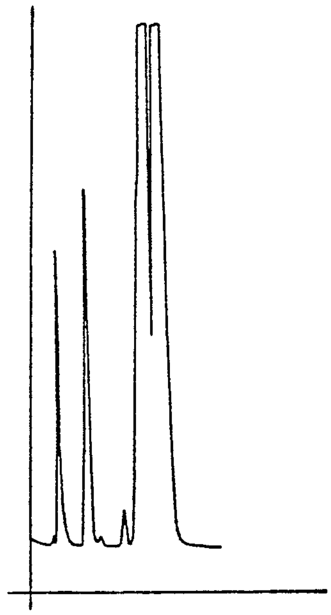

FIG.2

NMR SPECTRUM FOR EXAMPLE I.

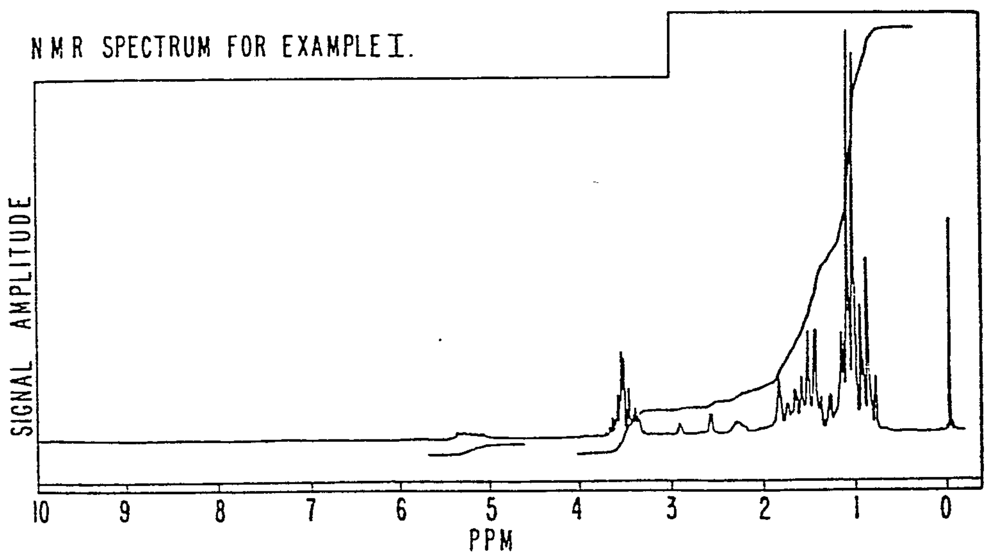

FIG.2A

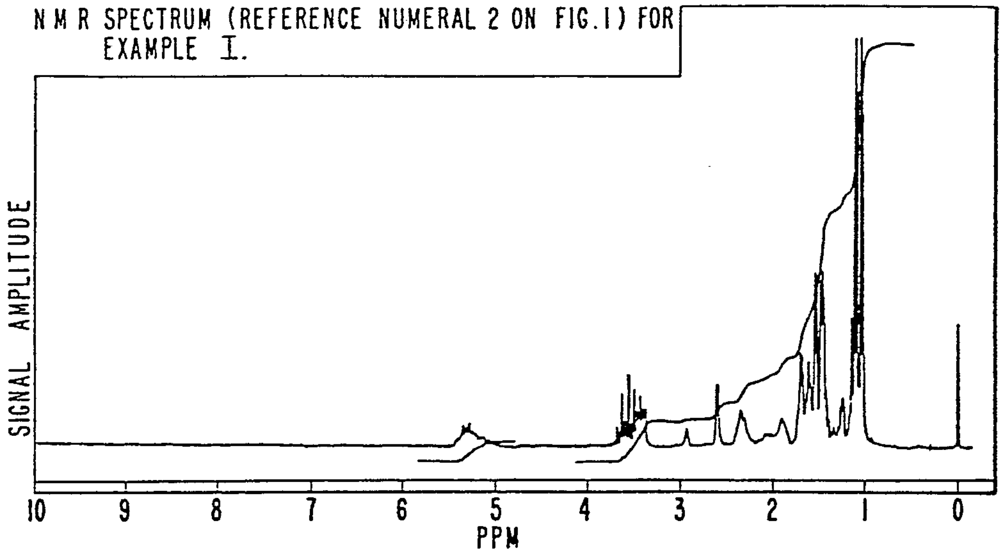
N M R SPECTRUM (REFERENCE NUMERAL 2 ON FIG.1) FOR EXAMPLE I.
SIGNAL AMPLITUDE
10 9 8 7 6 5 4 3 2 1 0
PPM

FIG.2B

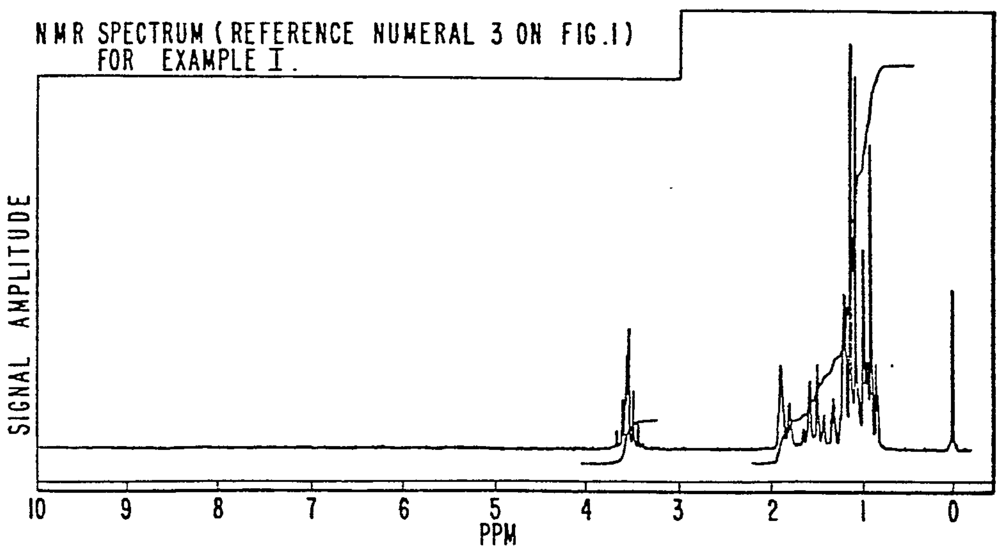
NMR SPECTRUM (REFERENCE NUMERAL 3 ON FIG.1) FOR EXAMPLE I.
SIGNAL AMPLITUDE
10 9 8 7 6 5 4 3 2 1 0
PPM

FIG. 3

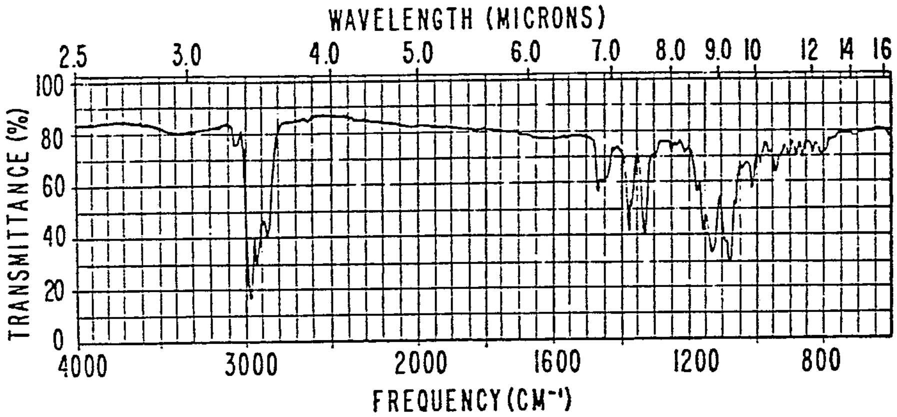


I R SPECTRUM FOR EXAMPLE I.

FIG.3A

IR SPECTRUM FOR EXAMPLE I.(REFERENCE NUMERAL 2 ON FIG.1)

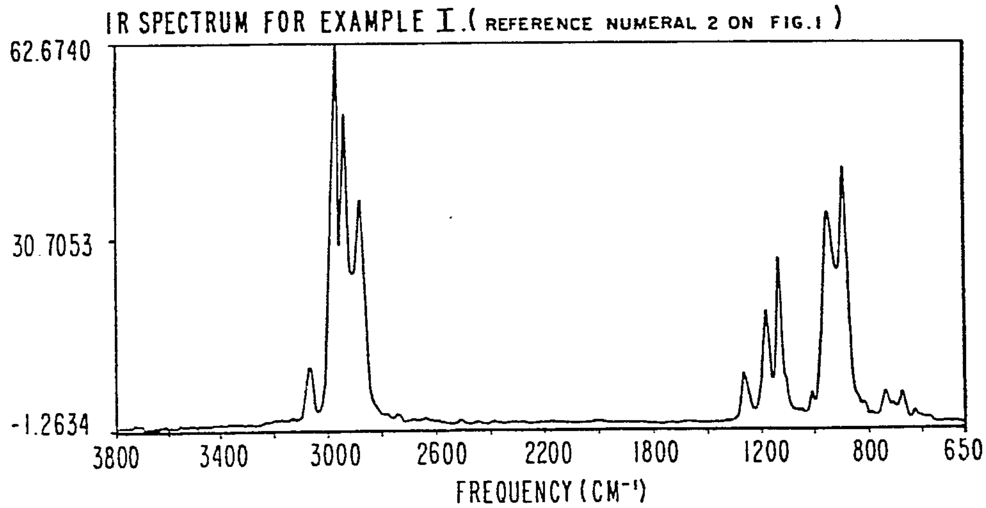

FIG.3B

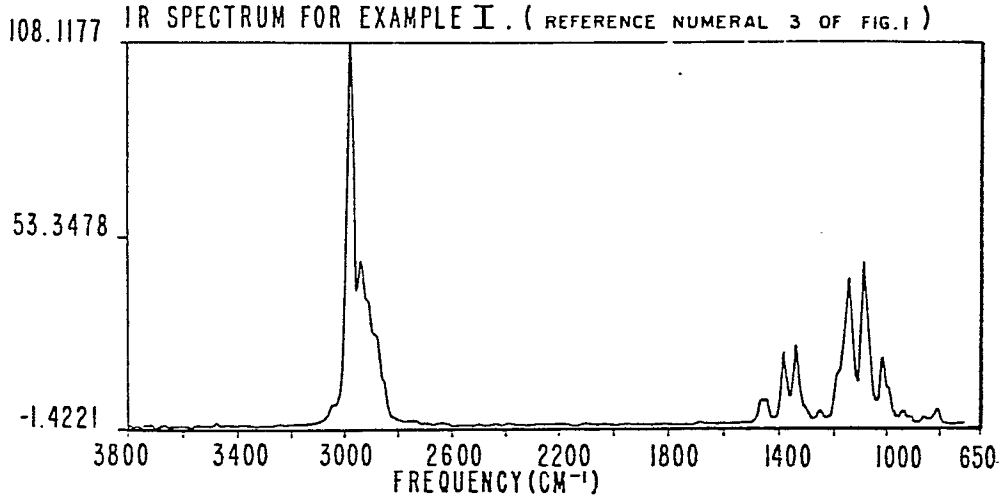
IR SPECTRUM FOR EXAMPLE I. ( REFERENCE NUMERAL 3 OF FIG.1 )
108.1177
53.3478
-1.4221
3800
3400
3000
2600
2200
1800
1400
1000
650
FREQUENCY(CM⁻¹)

FIG.3D

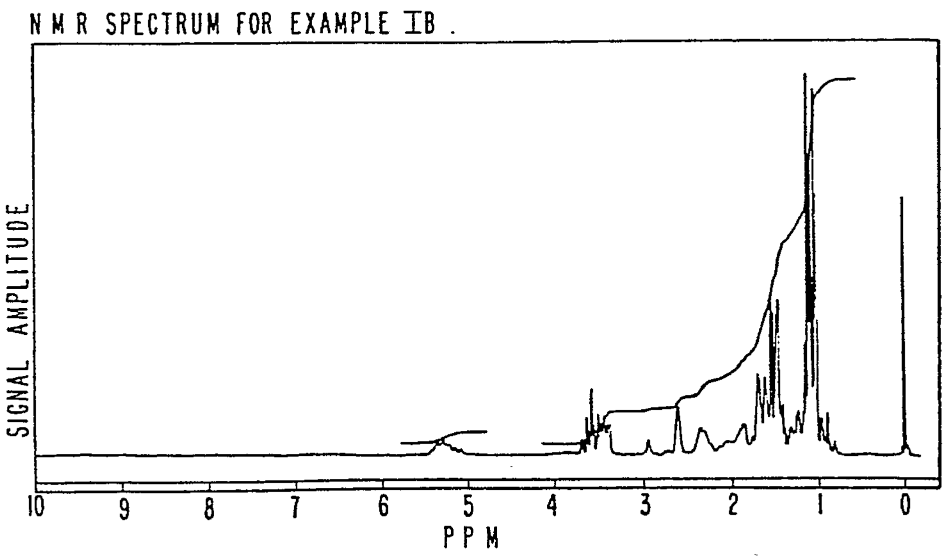
NMR SPECTRUM FOR EXAMPLE IB .
SIGNAL AMPLITUDE
10
9
8
7
6
5
4
3
2
1
0
PPM

FIG. 3C

GLC PROFILE FOR EXAMPLE I (B).

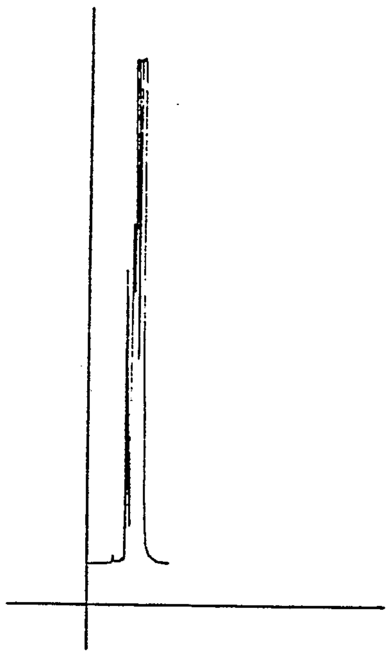

FIG.4

GLC PROFILE FOR EXAMPLE II.

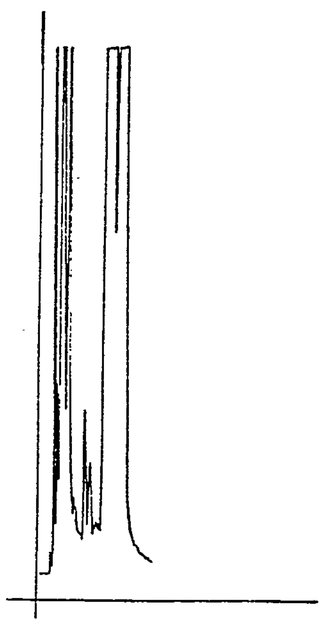

FIG.7

GLC PROFILE FOR EXAMPLE III.

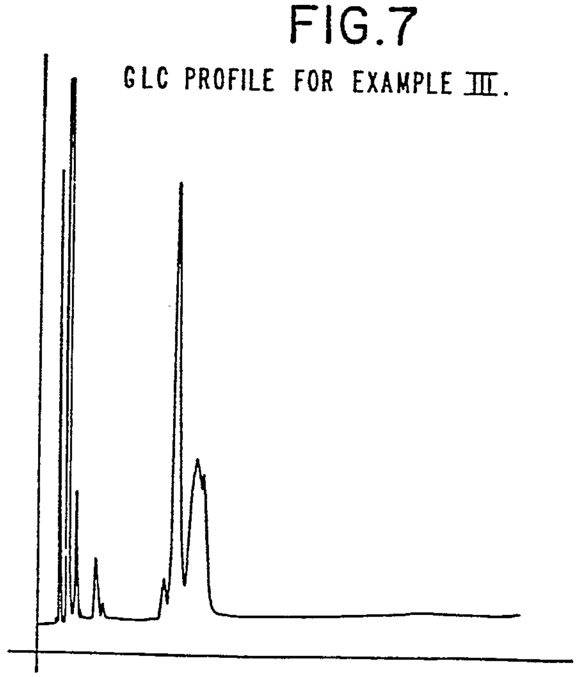

FIG. 5

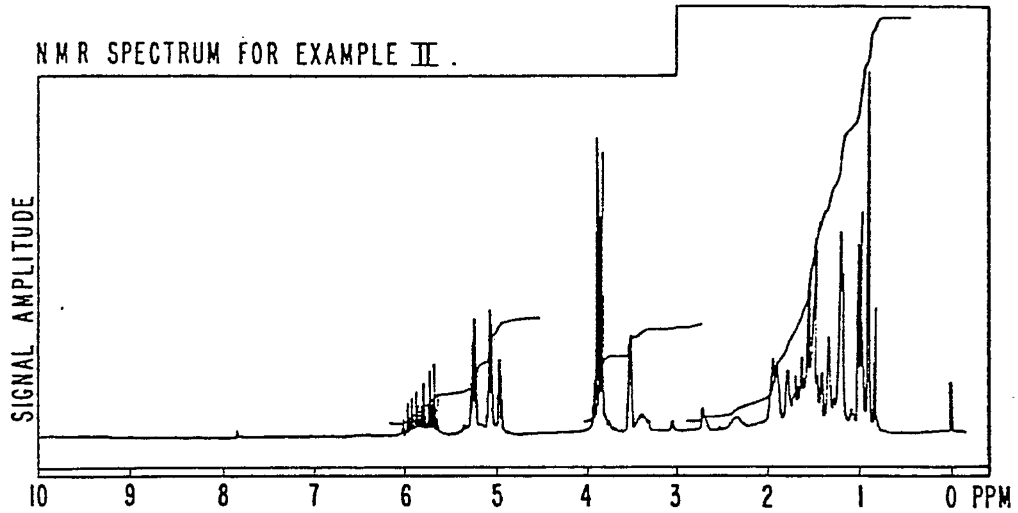


FIG. 6

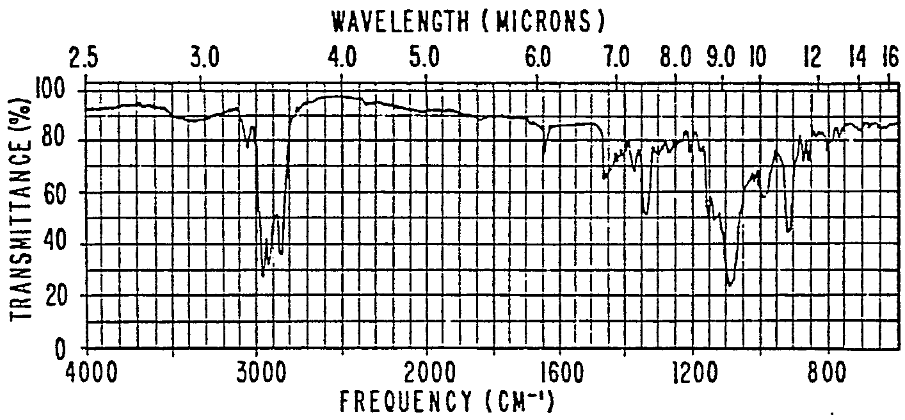


I R SPECTRUM FOR EXAMPLE II.

FIG. 8

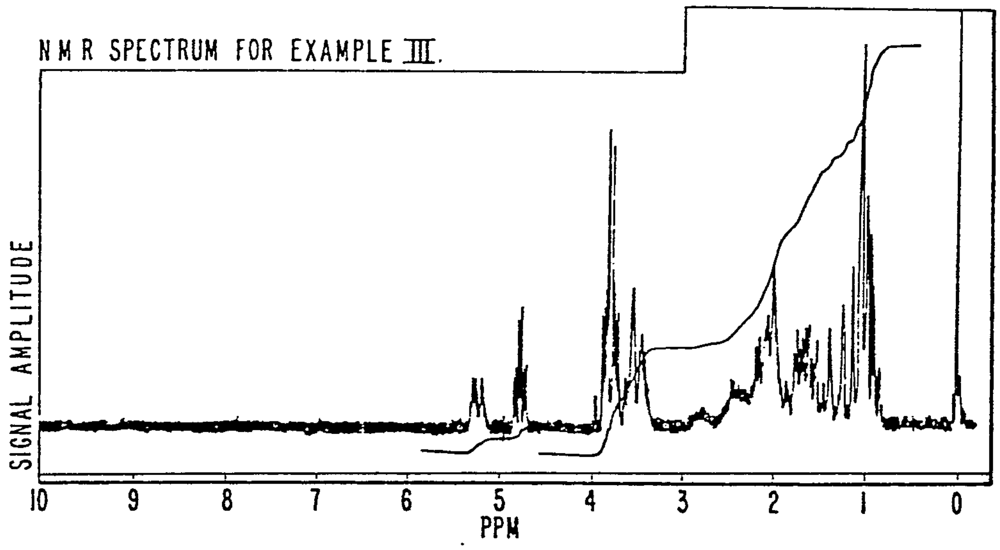


FIG. 9

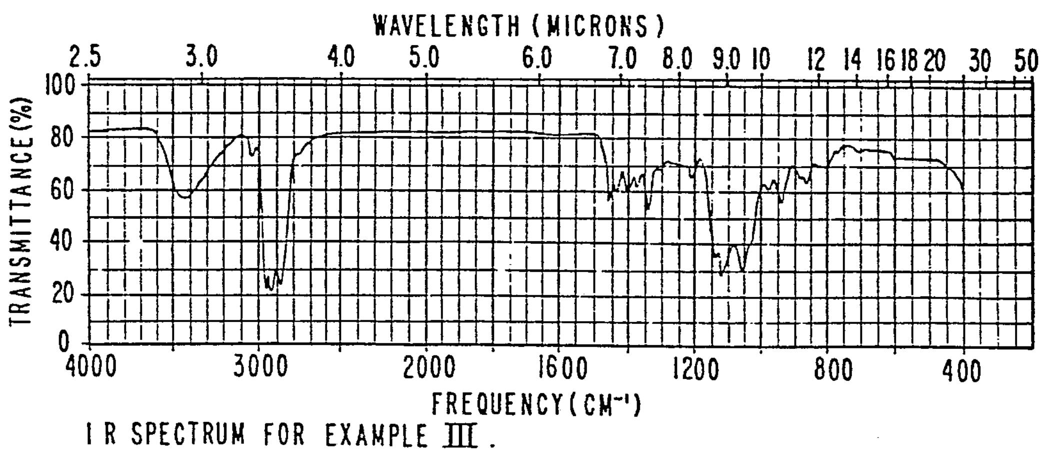


I R SPECTRUM FOR EXAMPLE III.

FIG.10

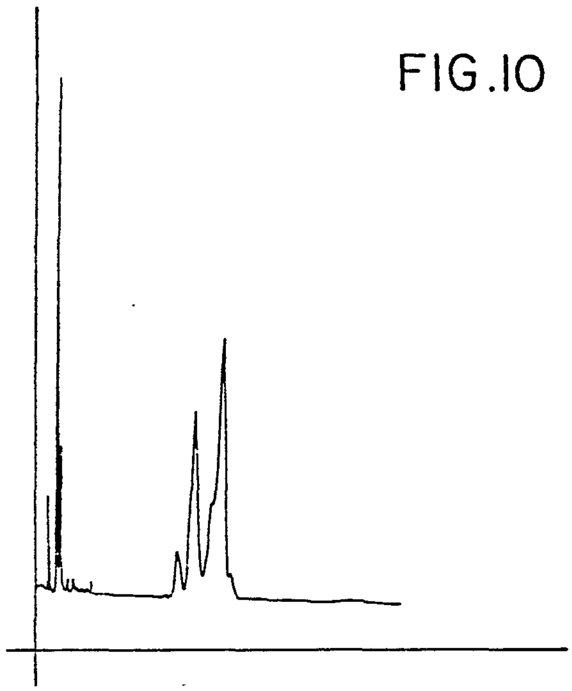

GLC PROFILE FOR EXAMPLE IV.

FIG. 11

NMR SPECTRUM FOR EXAMPLE IV.

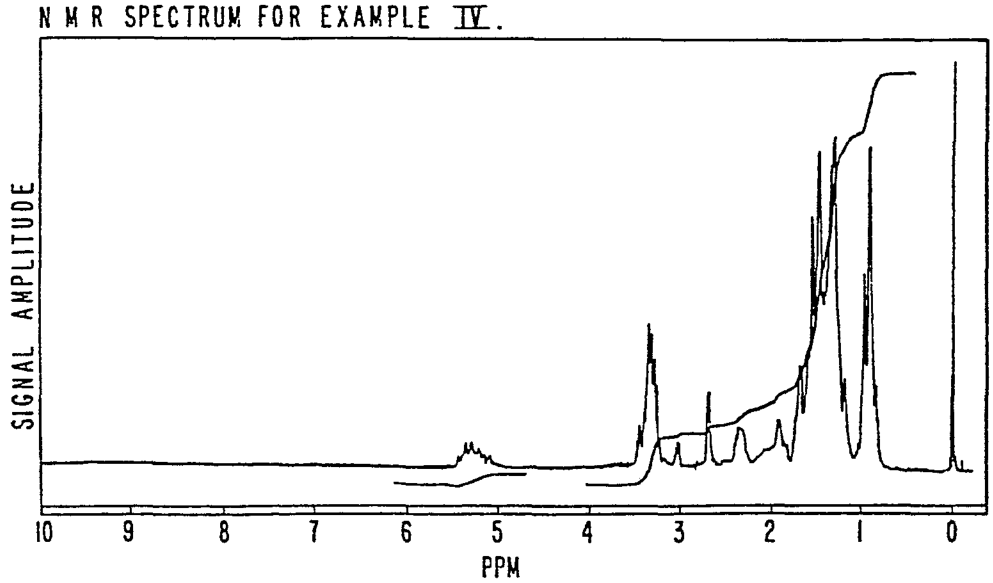

FIG.12

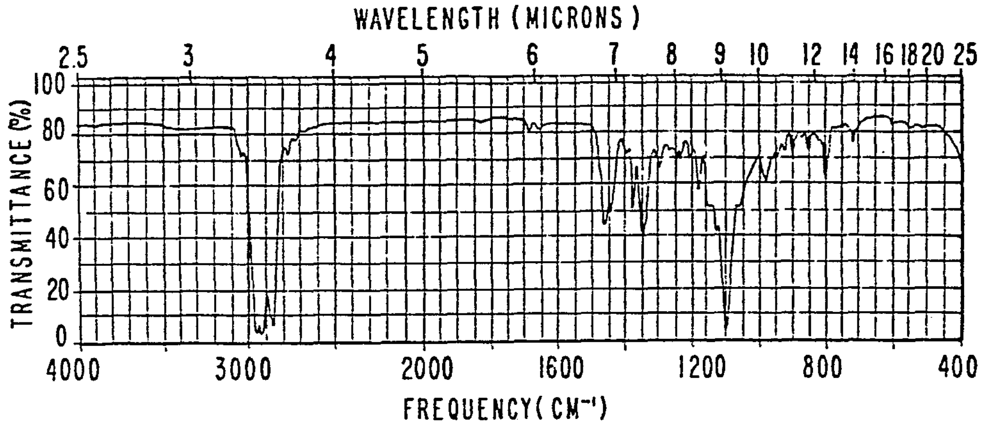


IR SPECTRUM FOR EXAMPLE IV.

FIG.13

NMR SPECTRUM FOR EXAMPLE V.

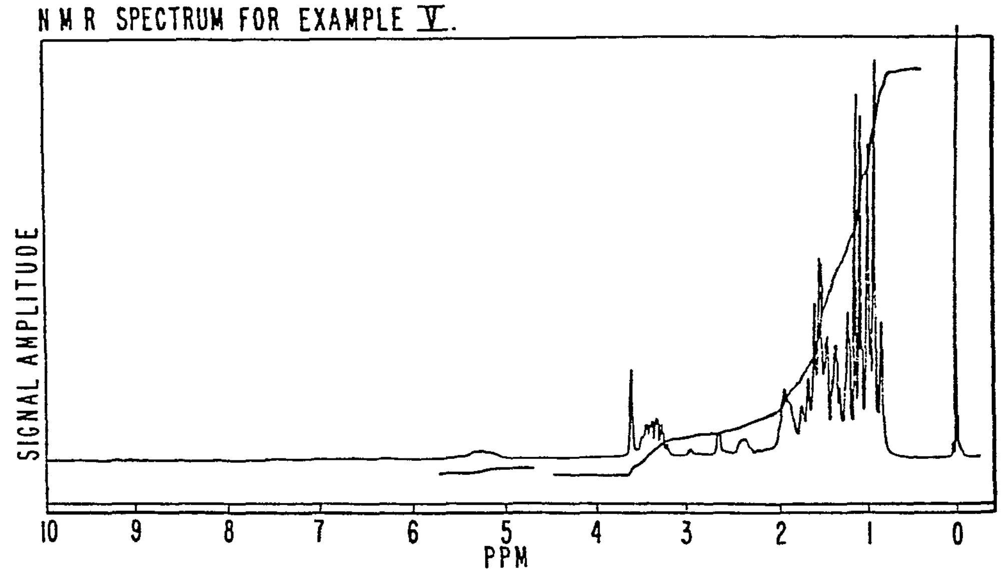

FIG.14

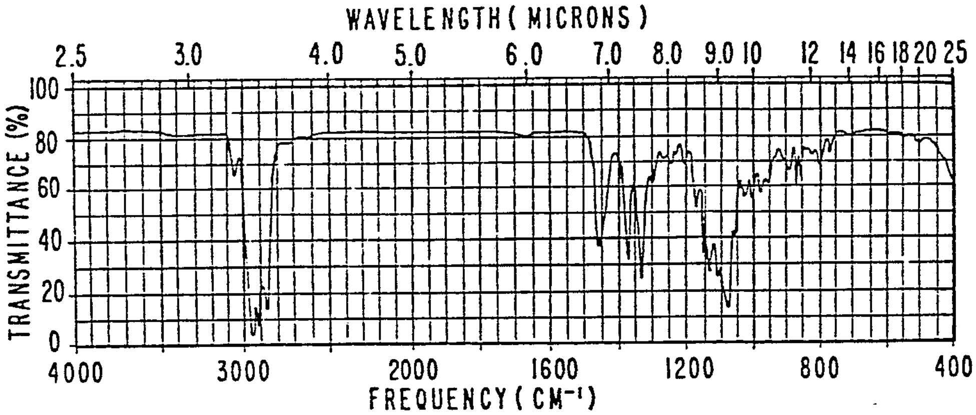


I R SPECTRUM FOR EXAMPLE V .

FIG.15

GLC PROFILE FOR EXAMPLE VI.

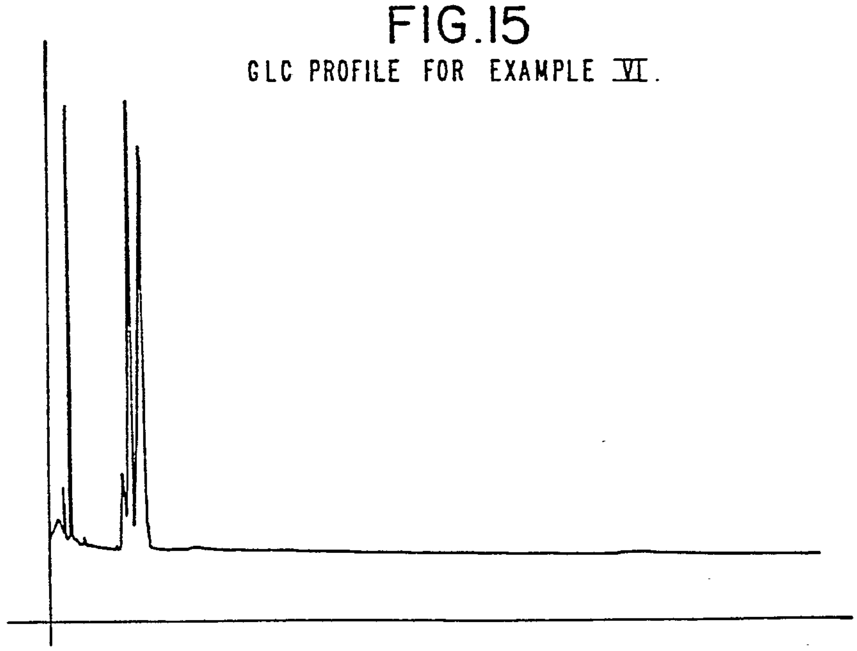

FIG.16

NMR SPECTRUM FOR EXAMPLE VI.

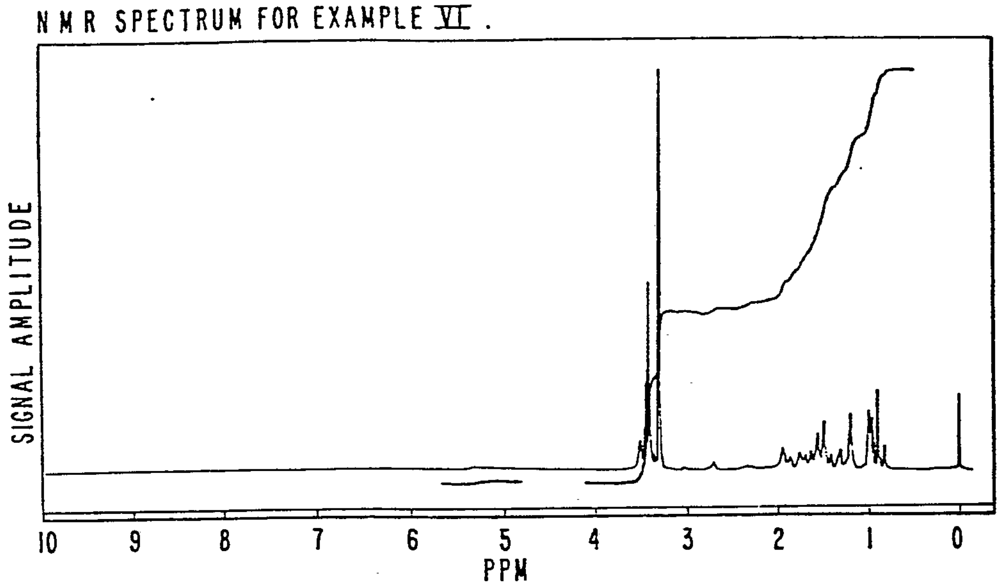


FIG.17

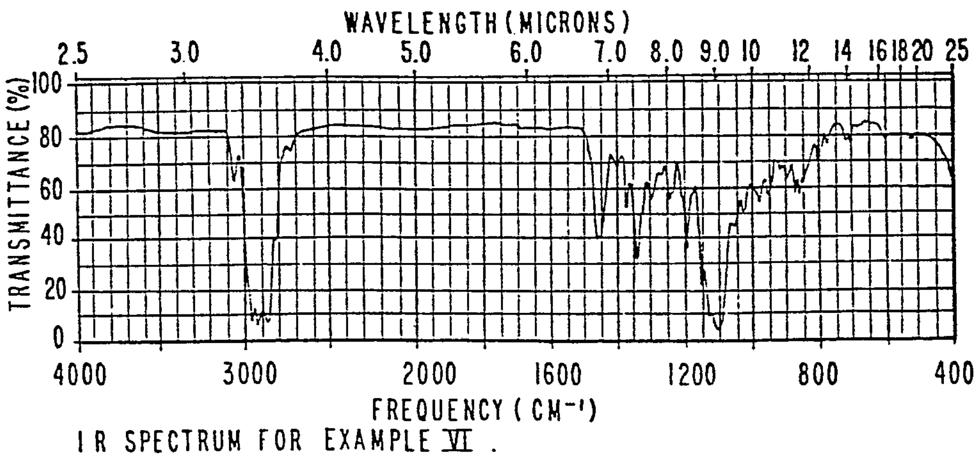


IR SPECTRUM FOR EXAMPLE VI.

FIG.18

GLC PROFILE FOR EXAMPLE VII.

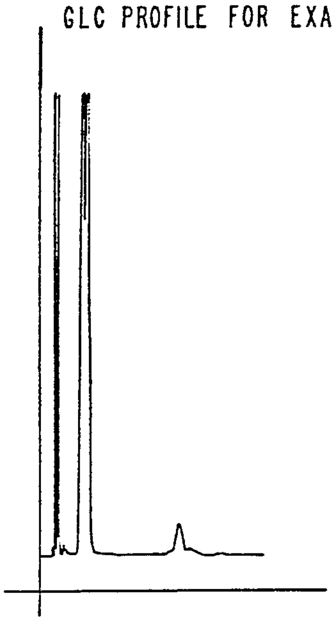

FIG.21

GLC PROFILE FOR EXAMPLE VIII.

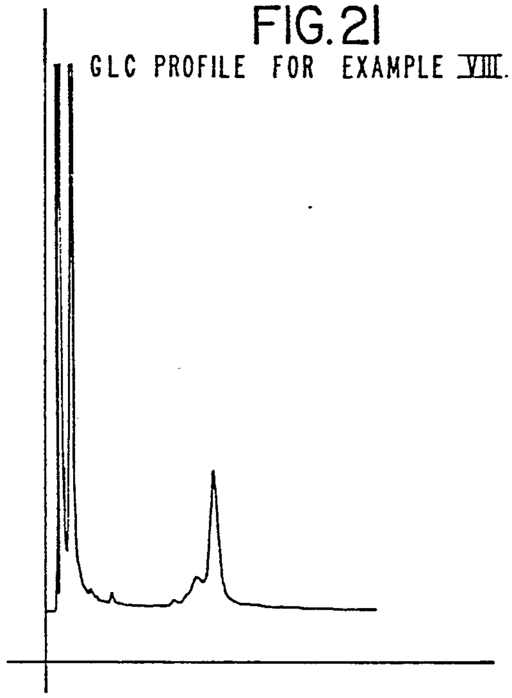

FIG.19

NMR SPECTRUM FOR EXAMPLE VIII.

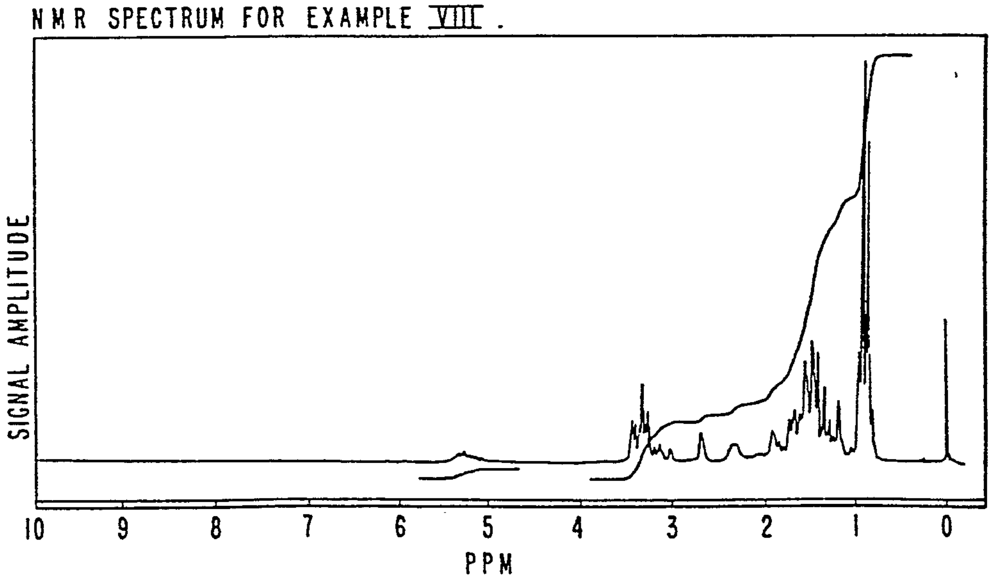

FIG.20

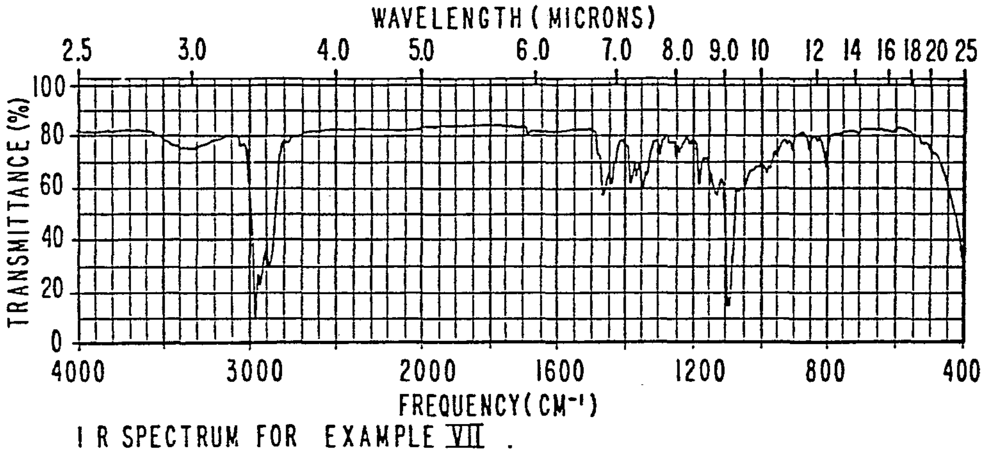


I R SPECTRUM FOR EXAMPLE VII .

FIG. 22

N M R SPECTRUM FOR EXAMPLE VIII .

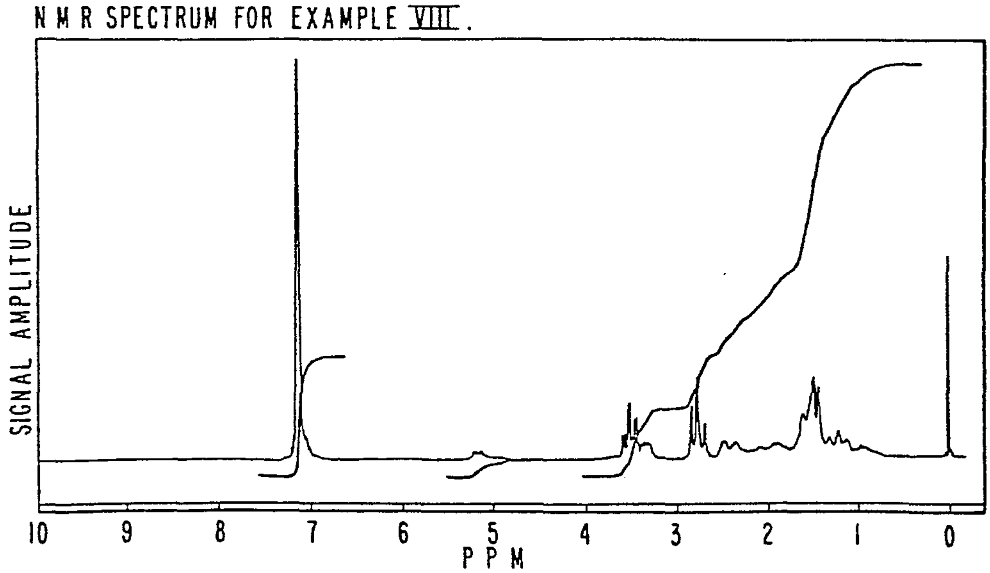

FIG. 25

N M R SPECTRUM FOR EXAMPLE IX.

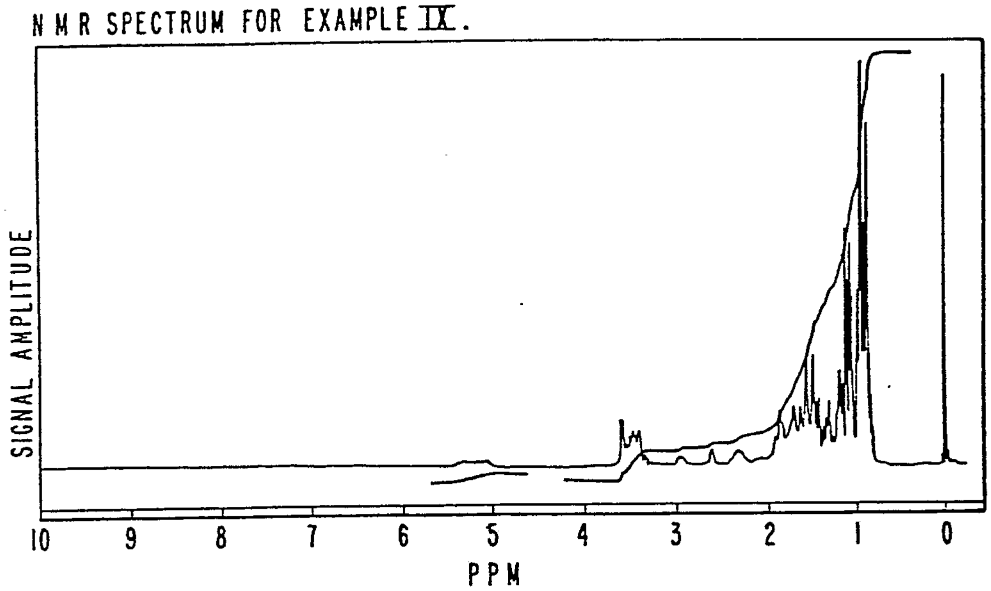


FIG. 26

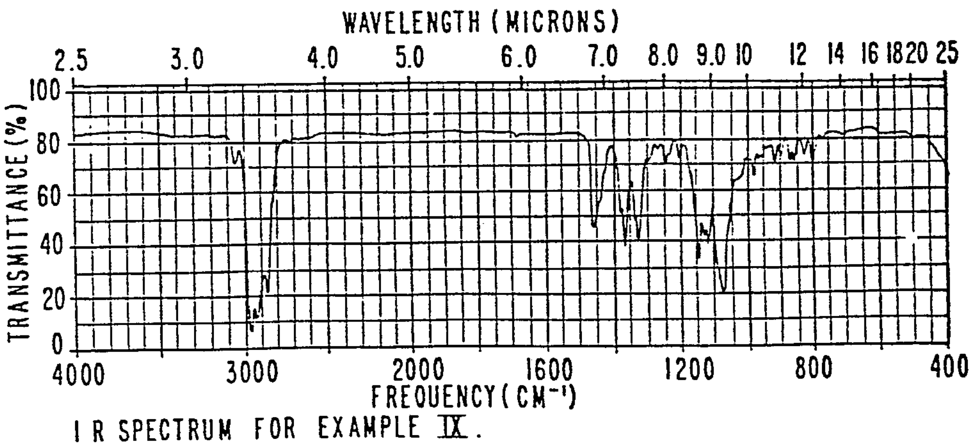


I R SPECTRUM FOR EXAMPLE IX.

FIG. 23

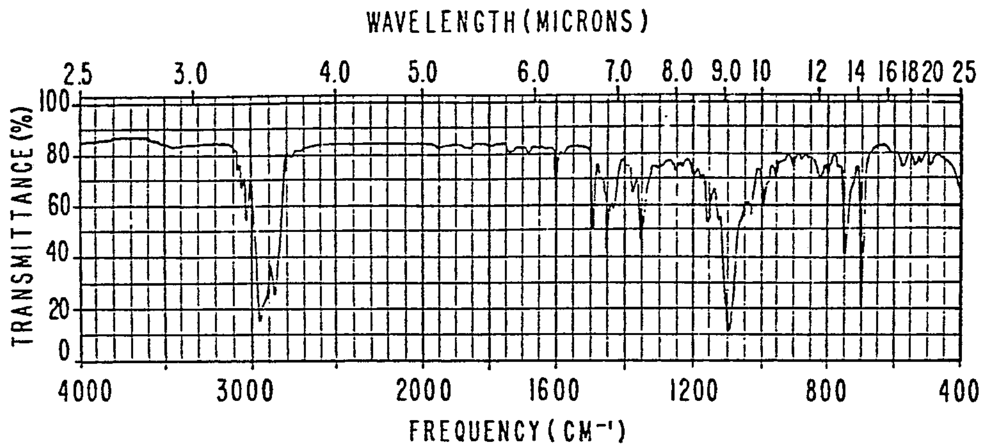


I R SPECTRUM FOR EXAMPLE VIII.

FIG. 24

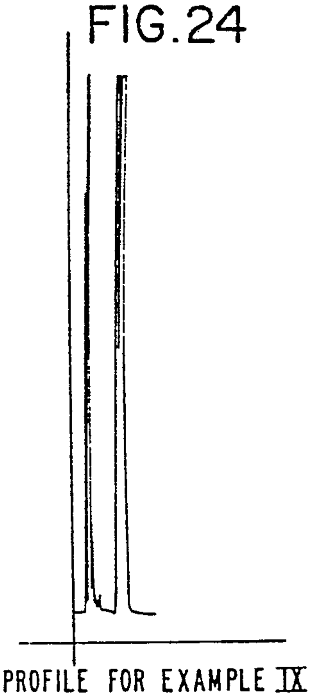

GLC PROFILE FOR EXAMPLE IX.

FIG. 27

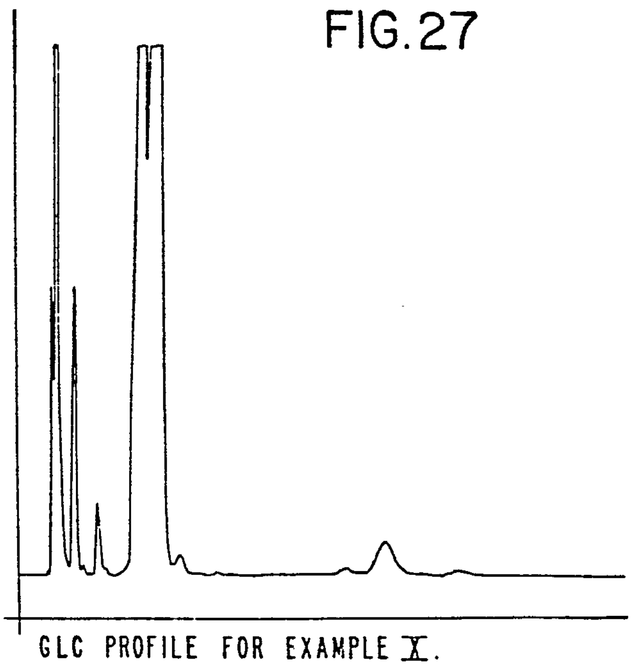

GLC PROFILE FOR EXAMPLE X.

FIG.28

NMR SPECTRUM FOR EXAMPLE X.

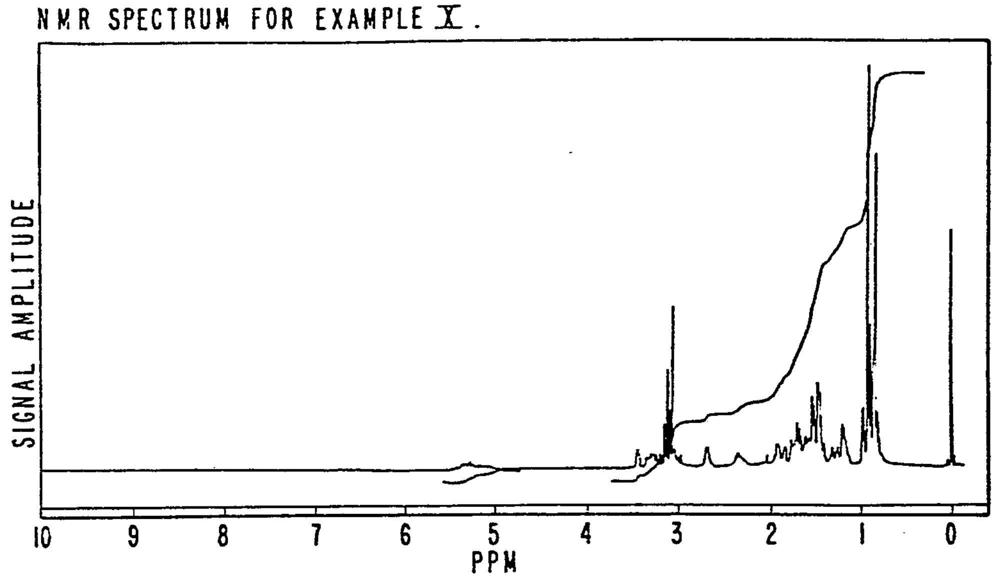


FIG.29

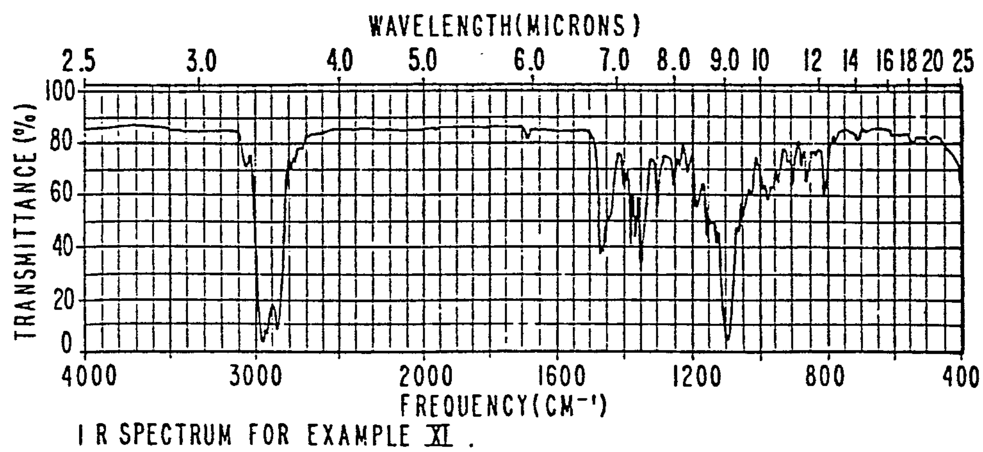


I R SPECTRUM FOR EXAMPLE XI.

FIG. 30

GLC PROFILE FOR EXAMPLE XI.

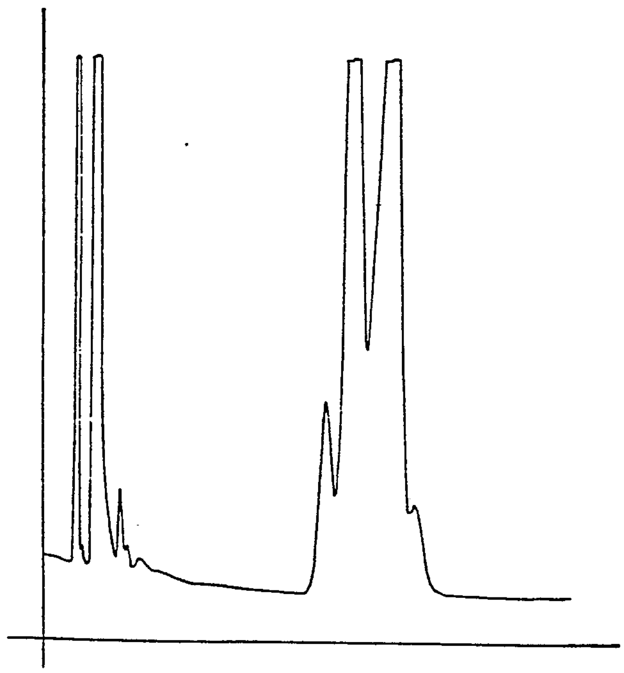

FIG.31

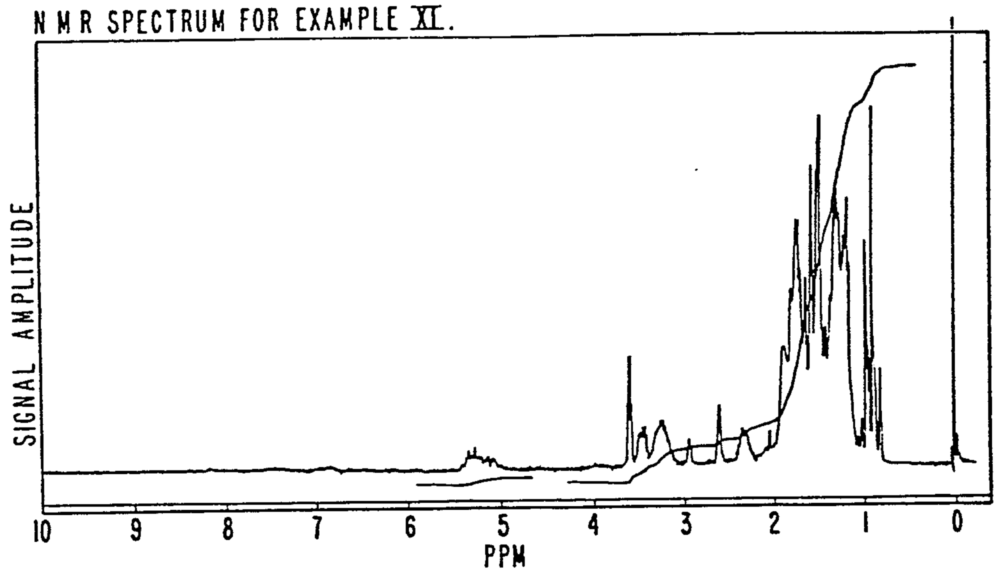
N M R SPECTRUM FOR EXAMPLE XI.
SIGNAL AMPLITUDE
10 9 8 7 6 5 4 3 2 1 0
PPM

FIG.32

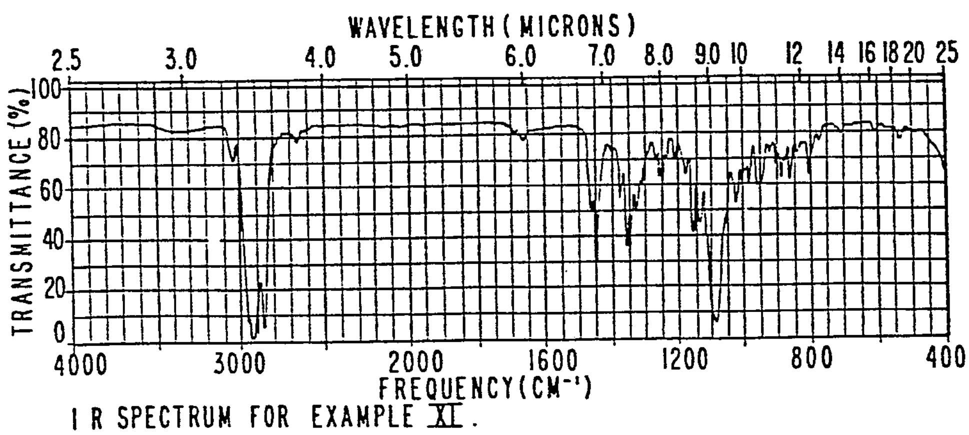
WAVELENGTH (MICRONS)
2.5 3.0 4.0 5.0 6.0 7.0 8.0 9.0 10 12 14 16 18 20 25
TRANSMITTANCE (%)
100 80 60 40 20 0
4000 3000 2000 1600 1200 800 400
FREQUENCY (CM⁻¹)
I R SPECTRUM FOR EXAMPLE XI.

FIG.33

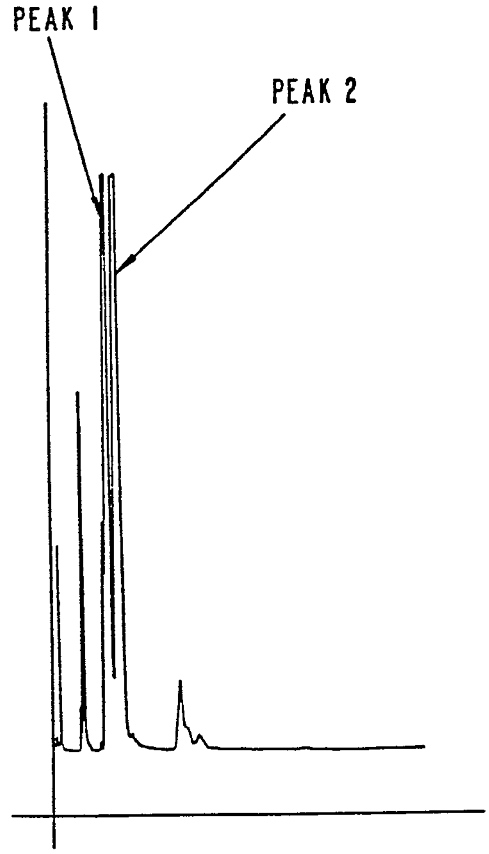


GLC PROFILE FOR EXAMPLE XII.

FIG.34

NMR SPECTRUM FOR PEAK 1 OF EXAMPLE XII.

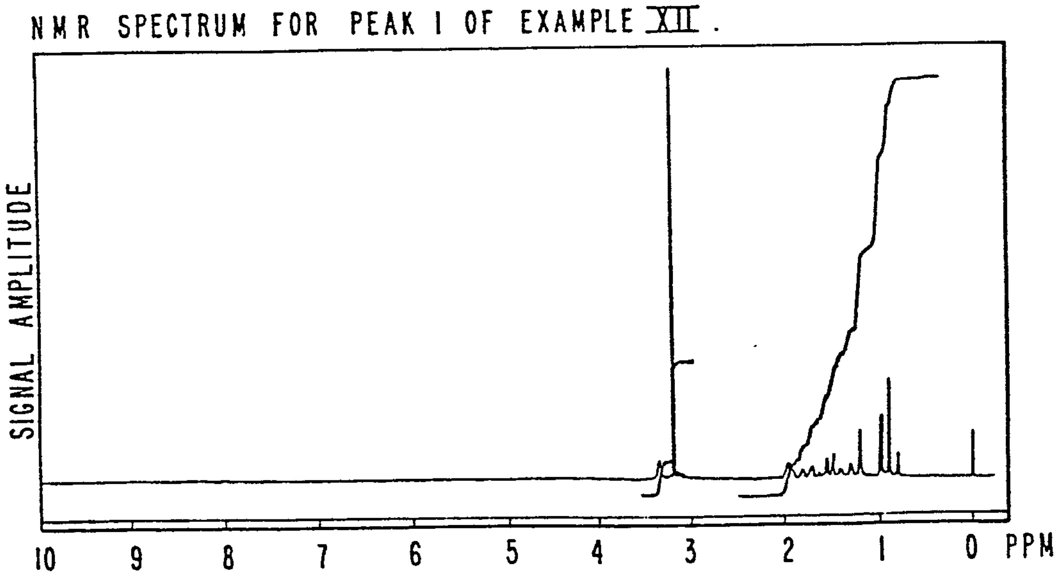

FIG.35

NMR SPECTRUM FOR PEAK 2 OF EXAMPLE XII.

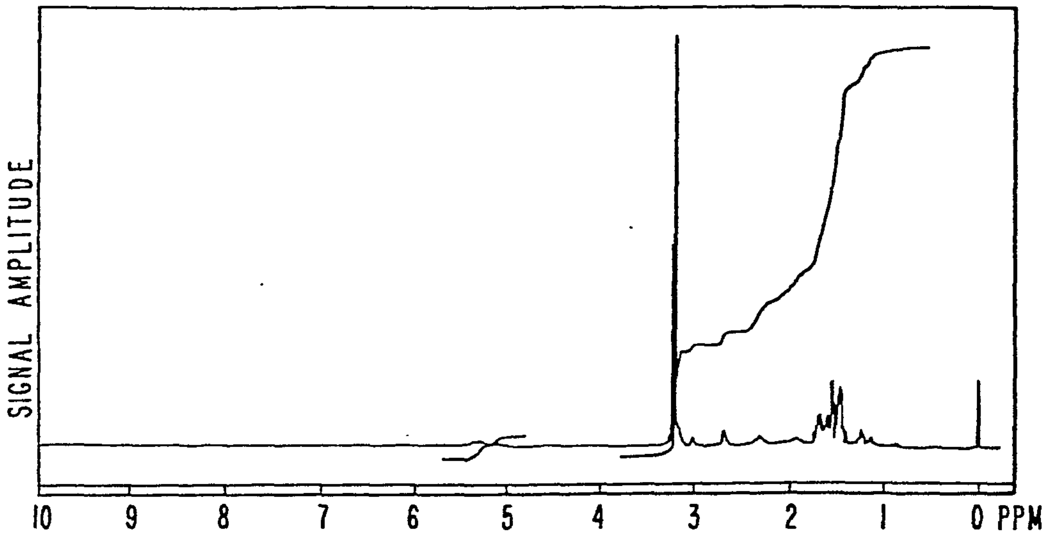


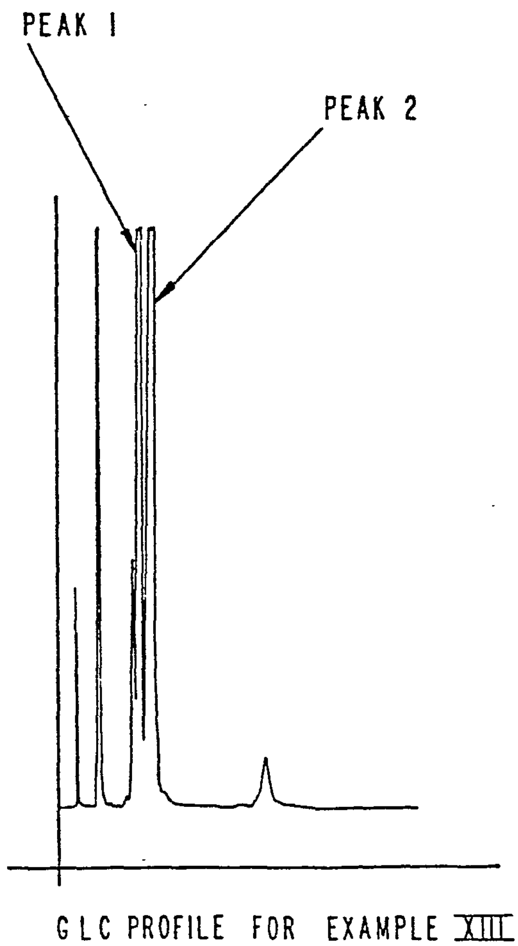


FIG.36

GLC PROFILE FOR EXAMPLE XIII.

FIG. 37

NMR SPECTRUM FOR PEAK 1 OF EXAMPLE XIII .

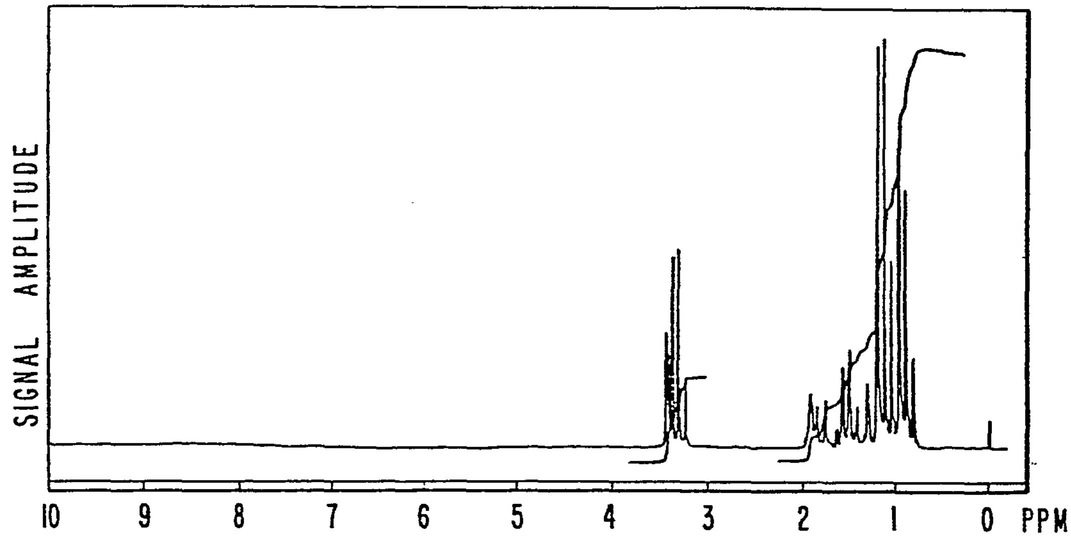


FIG. 38

NMR SPECTRUM FOR PEAK 2 FOR EXAMPLE XIII .

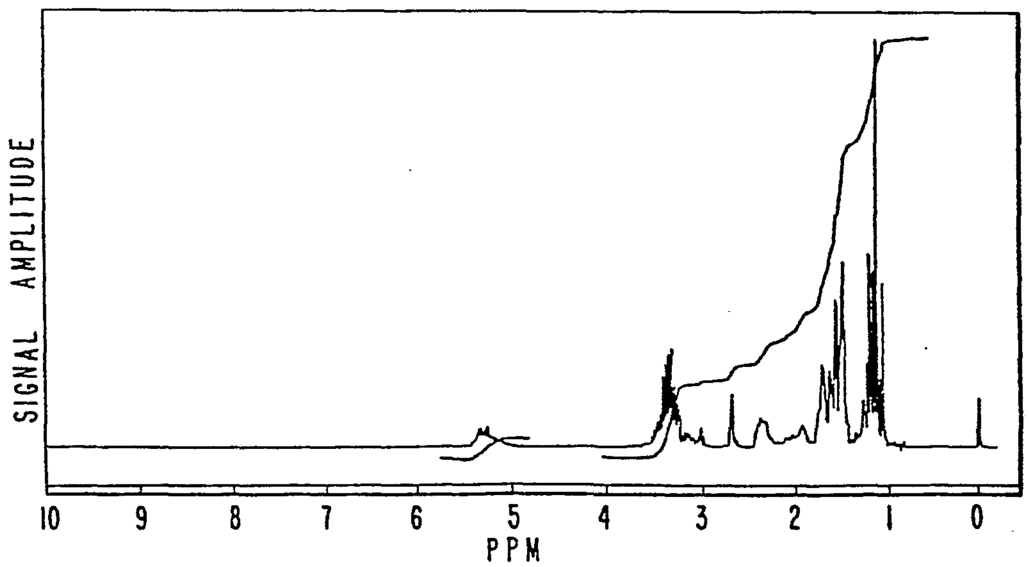

FIG.39

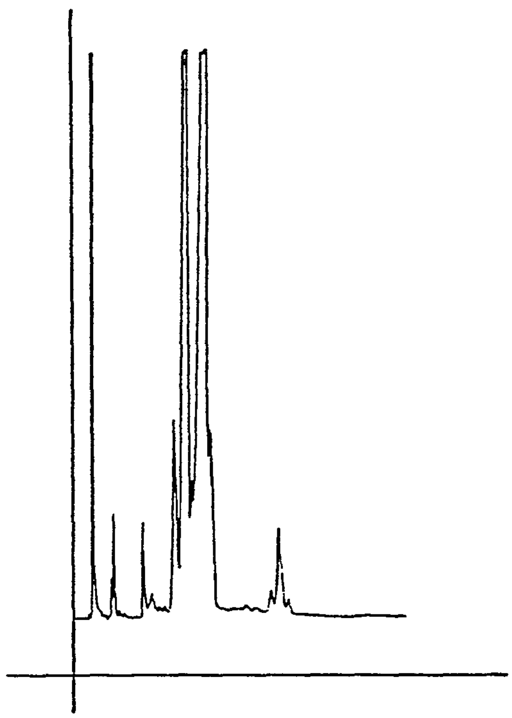

GLC PROFILE FOR EXAMPLE XIV.

FIG.40

N M R SPECTRUM FOR PEAK 1 FOR EXAMPLE XIV .

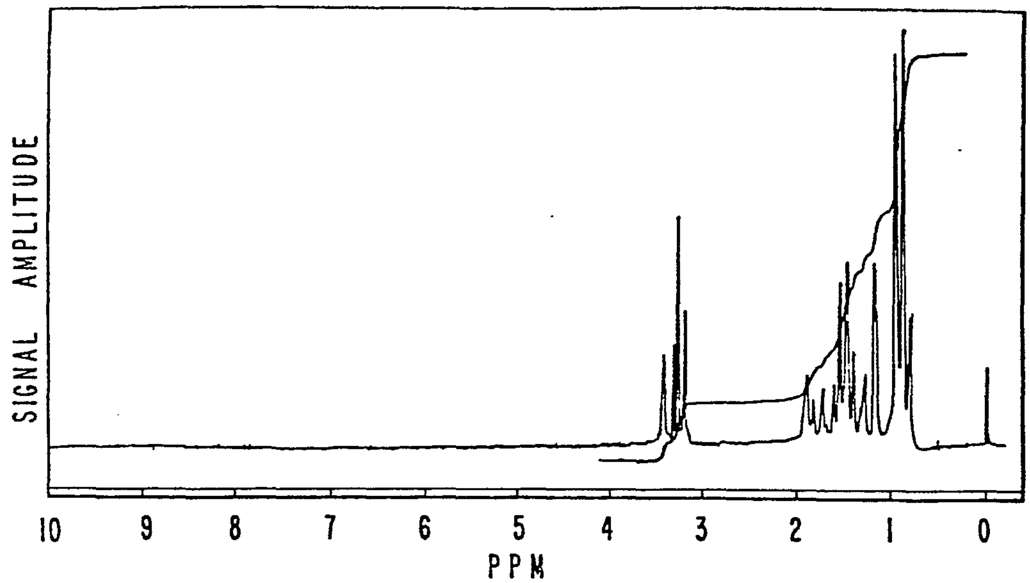


FIG.41

N M R SPECTRUM FOR PEAK 2 OF EXAMPLE XIV .